# EUROPEAN PATENT APPLICATION

(11) **EP 0 949 257 A1**
(43) Date of publication of application: **13.10.1999**
(21) Application number: 97950372.9
(22) Date of filing: 24.12.1997
(51) Int. Cl.: C07D 335/06, C07D 409/12, A01N 43/18

(54) **CYCLOHEXANEDIONE DERIVATIVES AND HERBICIDES PREPARED THEREFROM**

(30) Priority: 27.12.1996 JP 34986696
(71) Applicant: IDEMITSU KOSAN COMPANY LIMITED, Tokyo 100-0005 (JP)
(72) Inventor: KAMANO, Hideki, Sodegaura-shi, Chiba-ken 299-02 (JP); NASUNO, Ichiro, Sodegaura-shi, Chiba-ken 299-02 (JP); YAMAMOTO, Hiroshi, Sodegaura-shi, Chiba-ken 299-02 (JP); KOIKE, Kazuyoshi, Sodegaura-shi, Chiba-ken 299-02 (JP)
(74) Representative: Türk, Gille, Hrabal
(86) International application number: PCT/JP97/04785
(87) International publication number: WO 98/29406

(57) **Abstract**

Cyclohexanedione derivatives of the general formula (I) 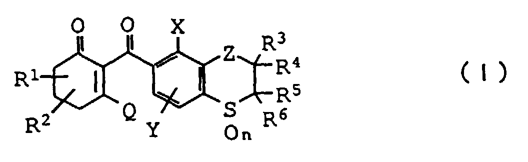 or salts thereof, and herbicides containing them,
the above cyclohexanedione derivatives and salts thereof, provided by the present invention, being capable of selectively controlling a broad range of upland soil weeds at a low dosage without causing phytotoxicity on crops cultivated in upland fields such as corn.

## Description

### Field of the Invention

The present invention relates to cyclohexanedione derivatives and herbicides containing them, more specifically, to cyclohexanedione derivatives which can control a broad range of upland weeds at a low dosage without causing phytotoxicity on crops such as corn, and herbicides containing them.

### Prior Art

Herbicides are very important chemicals for labor-saving of weed control working and production improvement in horticultural crops. Herbicides have been therefore aggressively studied and developed for a long time, and a variety of chemicals are now practically used. However, it is still desired to develop novel herbicides having further superior herbicidal properties, particularly herbicides which can selectively control object weeds alone at a low dosage without causing phytotoxicity on cultivated crops.

During the planting time of corn, triazine-containing herbicides such as atrazine and acid anilide-containing herbicides such as alachlor and metolachlor have been conventionally used. However, atrazine shows low efficacy to grass weeds, and on the other hand, alachlor and metolachlor show low efficacy to broad-leaved weeds. It is therefore difficult at present to control grass weeds and broad-leaved weeds together simultaneously with a single herbicide. Further, these herbicides are undesirable in view of an environmental problem due to their high dosage requirement.

In view of the above circumstances, the present inventors have developed novel cyclohexanedione derivatives having a thiochroman ring and have filed patent applications therefor (WO94/04524 and WO94/08988). Typical examples of these compounds are as follows.

Compounds described in WO94/04524

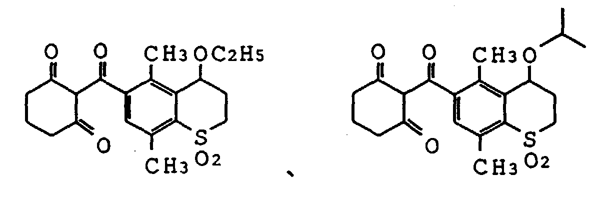

Compounds described in WO94/08988

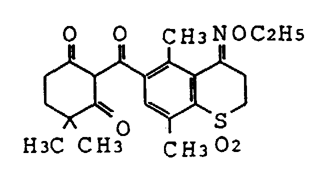

As a cyclohexanedione derivative having bicyclic properties, the following compounds have been disclosed (European Patent 94/283261).

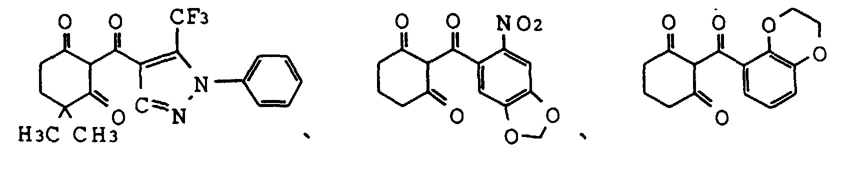

Since, however, the above compounds show phytotoxicity on sorgo and beet, it cannot be said that they have-sufficient activity both in post-emergence treatment and pre-emergence treatment.

### Disclosure of the Invention

It is therefore an object of the present invention to provide a novel cyclohexanedione derivative which can control a broad range of upland weeds at a low dosage without causing phytotoxicity on crops such as corn, and a herbicide containing the same.

The present inventors have made diligent studies to achieve the above object and have found that a cyclohexanedione derivative having a specific structure can control a broad range of upland weeds at a low dosage without causing phytotoxicity on crops such as corn, and the present invention has been completed on the basis of the above finding.

That is, the first object of the present invention is achieved by any one of
(1) cyclohexanedione derivatives of the general formula (I), 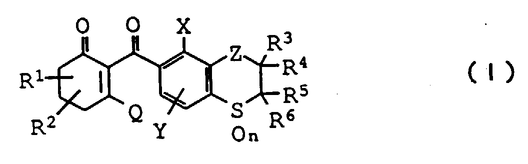
   wherein each of R¹ and R² is independently a hydrogen atom or a C₁-C₆ alkyl group,
   each of R³ to R⁶ is independently a hydrogen atom, a C₁-C₆ alkyl group, a C₁-C₆ haloalkyl group or a halogen atom,
   n is 0, 1 or 2,
   X is a C₁-C₆ alkyl group, a C₁-C₆ haloakyl group, a halogen atom, a C₁-C₆ alkoxy group, a C₁-C₆ haloalkoxy group, a C₂-C₆ alkoxyalkyl group, a C₁-C₆ alkylthio group, a C₁-C₆ haloalkylthio group, a C₁-C₆ alkylsulfinyl group or a C₁-C₆ alkylsulfonyl group,
   Y is a hydrogen atom, a C₁-C₆ alkyl group, C₁-C₆ haloalkyl group, a halogen atom, a C₁-C₆ alkoxy group, a C₁-C₆ haloalkoxy group or a C₂-C₆ alkoxyalkyl group,
   Z is a group of 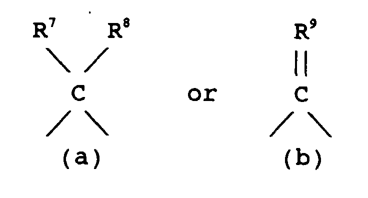
   in which each of R⁷ and R⁸ is independently a hydrogen atom, a halogen atom, a C₁-C₆ alkyl group, a C₁-C₆ alkoxy group, a C₁-C₆ alkylthio group or a group of -NR¹⁰R¹¹, provided that when R⁷, R⁸ or both is/are C₁-C₆ alkyl, C₁-C₆ alkoxy or C₁-C₆ alkylthio group(s), 1 to 13 hydrogen atoms thereof may be substituted with 1 to 13 halogen atoms or C₁-C₆ alkoxy groups and that when the carbon number thereof is C₂-C₆, the group(s) may contain an unsaturated bond and that when it is C₃-C₆, the group(s) may have a cyclized structure, each of R¹⁰ and R¹¹ is a hydrogen atom, a C₁-C₆ alkyl group or a C₁-C₆ alkylcarbonyl group, further provided that when both R⁷ and R⁸ are C₁-C₆ alkyl groups, C₁-C₆ alkoxy groups or C₁-C₆ alkylthio groups, carbon atoms of R⁷ and R⁸ may bond to each other to form a 3- to 7-membered ring, provided that when both R⁷ and R⁸ are alkyl groups, compounds of the general formula (I) in which X is a C₁-C₆ alkyl group, a halogen atom or a haloalkyl group and all of R³, R⁴, R⁵ and R⁶ are hydrogen atoms are excluded,
   provided that when one of R⁷ and R⁸ is an alkoxy group and when the other is a hydrogen atom, compounds of the general formula (I) in which X is a C₁-C₆ alkyl group and all of R³, R⁴, R⁵ and R⁶ are hydrogen atoms are excluded when hydrogen atom of the alkoxy group is not replaced with halogen or alkoxy group or when the alkoxy group does not contain an unsaturated bond or a cyclized structure, and
   R⁹ is an oxygen atom, a sulfur atom or a C₁-C₆ alkoxyimino group, provided that when R⁹ is a C₁-C₆ alkoxyimino group, 1 to 13 hydrogen atoms thereof may be replaced with 1 to 13 halogen atoms and that when the carbon number of R⁹ is C₂-C₆, the C₂-C₆ alkoxyimino group may contain an unsaturated bond, and provided that compounds of the general formula (I) in which X is a C₁-C₆ alkyl group and all of R³, R⁴, R⁵ and R⁶ are hydrogen atoms are excluded when R⁹ is an alkoxyimino group and when hydrogen atom thereof is not replaced with a halogen or the alkoxyimino group does not contain an unsaturated bond, and
   Q is a hydroxyl group or a group of 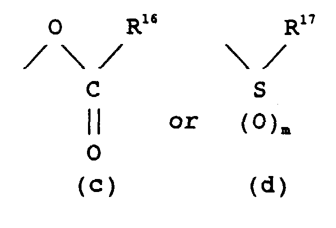
   in which each of R¹⁶ and R¹⁷ is a C₁-C₆ alkyl group, a C₁-C₆ haloalkyl group or a phenyl group which may be substituted with a C₁-C₆ alkyl group, a C₁-C₆ haloalkyl group, a halogen atom, a cyano group or a nitro group, and m is 0, 1 or 2, or salts thereof,
(2) cyclohexanedione derivatives of the general formula (I-a1), 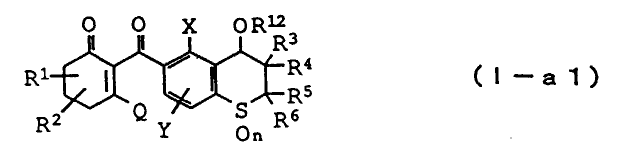
   wherein R¹ to R⁶, n, X, Y and Q are as defined above, and R¹² is a C₁-C₆ alkyl group or a C₁-C₆ haloalkyl group, provided that R¹² may be substituted with a C₁-C₆ alkoxy group, that when the number of carbon atoms of R¹² is C₂-C₆, R¹² may contain an unsaturated bond, that wnen the number of carbon atoms of R¹² is C₃-C₆, R¹² may contain a cyclic structure, and further that when R¹² is a C₁-C₆ alkyl group, compounds of the general formula (I-a1) in which X is a C₁-C₆ alkyl group and all of R³, R⁴, R⁵ and R⁶ are hydrogen atoms are excluded, or salts thereof,
(3) cyclohexanedione derivatives of the general formula (I-a2), 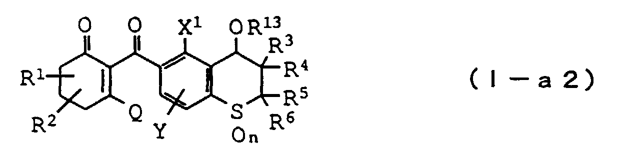
   wherein R¹ to R⁶, n, Y and Q are as defined above, R¹³ is a C₁-C₆ alkyl group, and X¹ is a C₁-C₆ haloalkyl group, a halogen atom, a C₁-C₆ alkoxy group, a C₁-C₆ haloalkoxy group, a C₂-C₆ alkoxyalkyl group, a C₁-C₆ alkylthio group, a C₁-C₆ haloalkylthio group, a C₁-C₆ alkylsulfinyl group or a C₁-C₆ alkylsulfonyl group, or salts thereof,
(4) cyclohexanedione derivatives of the general formula (I-a3), 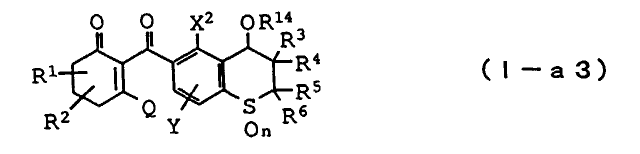
   wherein R¹ to R⁶, n, Y and Q are as defined above, R¹⁴ is a C₁-C₆ haloalkyl group, an alkoxyalkyl group, an alkenyl group, an haloalkenylalkyl group or an alkynylalkyl group, and X² is a C₁-C₆ alkyl group, a C₁-C₆ haloalkyl group, a halogen atom, a C₁-C₆ alkoxy group, a C₁-C₆ haloalkoxy group, a C₂-C₆ alkoxyalkyl group, a C₁-C₆ alkylthio group, a C₁-C₆ haloalkylthio group, a C₁-C₆ alkylsulfinyl group or a C₁-C₆ alkylsulfonyl group, or salts thereof,
(5) cyclohexanedione derivatives of the general formula (I-b1), 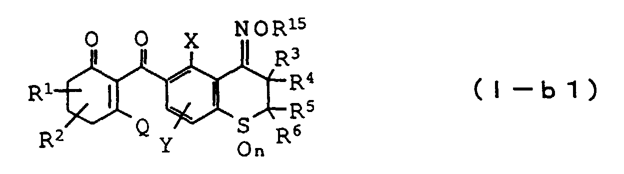
   wherein R¹ to R⁶, n, X, Y and Q are as defined above, R¹⁵ is a C₁-C₆ alkyl group or a C₁-C₆ alkenyl group, provided that when R¹⁵ is a C₁-C₆ alkyl group, compounds of the general formula (I-b1) in which X is a C₁-C₆ alkyl group and all of R³, R⁴, R⁵ and R⁶ are hydrogen atoms are excluded, or salts thereof,
(6) cyclohexanedione derivatives of the general formula (I-b2), 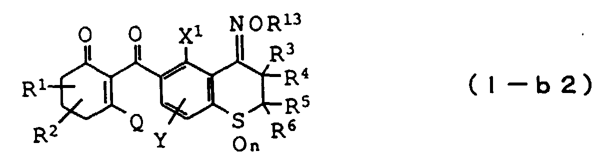
   wherein R¹ to R⁶, R¹³, n, X¹, Y and Q are as defined above, or salts thereof, and
(7) cyclohexanedione derivatives of the general formula (I-c), 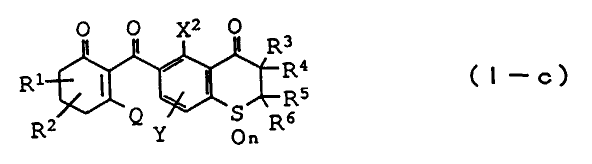
   wherein R¹ to R⁶, n, X², Y and Q are as defined above, or salts thereof.

Further, the above second object of the present invention is achieved by a herbicide (to be sometimes referred to as "herbicide of the present invention" hereinafter) containing at least one of the cyclohexanedione derivatives of the above general formulae (I), (I-a1), (I-a2), (I-a3), (I-b1), (I-b2) and (I-c) and salts thereof.

### Best Modes for Practicing the Invention

The cyclohexanedione derivative of the present invention will be explained first.

The cyclohexanedione derivative of the present invention has the general formula (I).

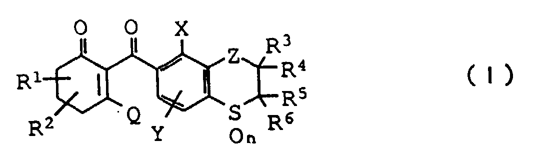

In the general formula (I), X is a C₁-C₆ alkyl group, a C₁-C₆ haloalkyl group, a halogen atom, a C₁-C₆ alkoxy group, a C₁-C₆ haloalkoxy group, a C₂-C₆ alkoxyalkyl group, a C₁-C₆ alkylthio group, a C₁-C₆ haloalkylthio group, a C₁-C₆ alkylsulfinyl group or a C₁-C₆ alkylsulfonyl group.

Examples of the above C₁-C₆ alkyl group include methyl, ethyl, propyl, butyl, pentyl and hexyl, and the propyl, butyl, pentyl and hexyl may be linear, cyclic or branched. The C₁-C₆ haloalkyl group is a group formed by replacing 1 to 13 hydrogen atoms of the above C₁-C₆ alkyl group with 1 to 13 halogen atoms (e.g., chlorine, flourine, bromine and iodine). Specific examples thereof include -CF₃, -C₂F₅, -C₂H₄F, -CH₂Cl, -CHF₂, -CCl₃, -C₂H₃Cl₂, - C₂H₃F₂, -C₂H₂F₃, -C₂H₂Cl₃, -C₃H₆F, -C₄H₈F, -CH₂Br, -CH₂I, -C₃H₄F₃ and -C₄H₆F₃. The halogen atom includes chlorine, fluorine, bromine and iodine.

Specific examples of the C₁-C₆ alkoxy group include methoxy, ethoxy, propoxy, butoxy, pentoxy and hexoxy and the propoxy, butoxy, pentoxy and hexoxy may be linear, cyclic or branched. The C₁-C₆ haloalkoxy group is a group formed by replacing 1 to 13 hydrogen atoms of the above C₁-C₆ alkoxy group with 1 to 13 halogen atoms (e.g., chlorine, fluorine, bromine and iodine). Examples thereof include -OCF₃, -OC₂F₅, -OC₂H₄F, -OC₂H₄Cl, -OCHF₂, -OCH₂F, -OCCl₃, -OC₂H₃Cl₂, -OC₂H₃F₂, -OCH₂Br and -OCH₂I.

The C₂-C₆ alkoxyalkyl group is a group formed by replacing one hydrogen atom of the above alkyl group with a C₁-C₆ alkoxy group (any one of methoxy, ethoxy, n-propoxy, i-propoxy, n-butoxy, i-butoxy, s-butoxy, t-butoxy, n-hexoxy, i-hexoxy, s-hexoxy and t-heoxoxy). Specific examples thereof include -CH₂OCH₃, -CH₂OC₂H₅, -CH₂OC₃H₇, -C CH₃)₂OCH₃, -C(CH₃)₂OC₂H₅, -CH₂CH₂OCH₃, -CH₂CH₂OC₂H₅, -CH₂(CH₂)₂OCH₃, -CH₂C(CH₃)₂OCH₃, -CH₂CH₂CH₂OCH₃ and -CH(CH₃)CH₂OCH₃.

Specific examples of the C₁-C₆ alkylthio group include methylthio, ethylthio, propylthio, butylthio, pentylthio and hexylthio, and the propylthio, butylthio, pentylthio and hexylthio may be linear, cyclic or branched. The C₁-C₆ haloalkylthio group is a group formed by replacing 1 to 13 hydrogen atoms of the above C₁-C₆ alkylthio group with 1 to 13 halogen atoms (e.g., chlorine, fluorine, bromine and iodine). Examples thereof include -SCF₃, -SC₂F₅, -SC₂H₄F, -SC₂H₄Cl, -SCHF₂, -SCH₂F, - SCCl₃, -SC₂H₃Cl₂, -SC₂H₃F₂, -SCH₂Br and -SCH₂I.

Specific examples of the C₁-C₆ alkylsulfinyl group include methylsulfinyl, ethylsulfinyl, propylsulfinyl, butylsulfinyl, pentylsulfinyl and hexylsulfinyl, and the propylsulfinyl, butylsulfinyl, pentylsulfinyl and hexylsulfinyl may be linear, cyclic or branched. Examples of the C₁-C₆ alkylsulfonyl group include methylsulfonyl, ethylsulfonyl, propylsulfonyl, butylsulfonyl, pentylsulfonyl and hexylsulfonyl, and the propylsulfonyl, butylsulfonyl, pentylsulfonyl and hexylsulfonyl may be linear, cyclic or branched.

X is preferably a C₁-C₆ alkyl group, a C₁-C₆ haloakyl group or a halogen atom, more preferably methyl, chlorine or -CF₃.

Y is a hydrogen atom, a C₁-C₆ alkyl group, a C₁-C₆ haloalkyl group, a halogen atom, a C₁-C₆ alkoxy group, a C₁-C₆ haloalkoxy group or a C₂-C₆ alkoxyalkyl group.

The above C₁-C₆ alkyl group, C₁-C₆ haloalkyl group, halogen atom, C₁-C₆ alkoxy group, C₁-C₆ haloalkoxy group or C₂-C₆ alkoxyalkyl group includes those specified with regard to the above X. The position of the substituent Y is the 7- or 8-position of the thiochroman ring, particularly preferably the 8-position. Y is preferably a hydrogen atom, a C₁-C₆ alkyl group or a halogen atom, particularly preferably hydrogen, methyl or chlorine.

Each of R¹ and R² is independently a hydrogen atom or a C₁-C₆ alkyl group, and each of R³, R⁴, R⁵ and R⁶ is independently a hydrogen atom, a C₁-C₆ alkyl group, a C₁-C₆ haloalkyl group or a halogen atom. Specific examples of the above C₁-C₆ alkyl groups, C₁-C₆ haloalkyl group and halogen atom are those specified with regard to X.

Preferably, each of R³, R⁴, R⁵ and R⁶ is independently a hydrogen atom or a C₁-C₆ alkyl group such as methyl, particularly preferably a hydrogen atom or methyl.

n refers to the number of oxygen atom(s) bonding to the sulfur atom of the thiochroman ring, and it is 0 (sulfide), 1 (sulfoxide) or 2 (sulfone), preferably 0 (sulfide) or 2 (sulfone).

Z is a group of

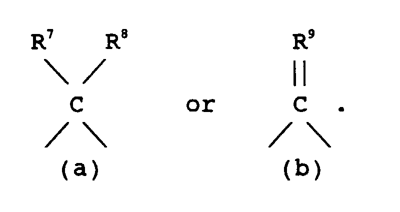

In the group (a) in the definition of Z, each of R⁷ and R⁸ is independently a hydrogen atom, a halogen atom, a C₁-C₆ alkyl group, a C₁-C₆ alkoxy group, a C₁-C₆ alkylthio group or a group of -NR¹⁰R¹¹. When R⁷, R⁸ or both are C₁-C₆ alkyl, C₁-C₆ alkoxy or C₁-C₆ alkylthio group(s), 1 to 13 hydrogen atoms of the group(s) may be replaced with 1 to 13 halogen atoms or C₁-C₆ alkoxy groups, and when the number of carbon atoms of the group(s) is C₂-C₆, the group(s) may contain an unsaturated bond. Further, when the above number is C₃-C₆, the group(s) may have a cyclic structure. Each of R¹⁰ and R¹¹ is a hydrogen atom, a C₁-C₆ alkyl group or a C₁-C₆ alkylcarbonyl group. Further, when both R⁷ and R⁸ are C₁-C₆ alkyl groups, C₁-C₆ alkoxy groups or C₁-C₆ alkylthio groups, carbon atoms of R⁷ and R⁸ may form a 3- to 7-membered ring by bonding to each other.

However, when R⁷ and R⁸ are both alkyl groups, there is excluded a case where X is a C₁-C₆ alkyl group, a halogen atom or a haloalkyl group and all of R³, R⁴, R⁵ and R⁶ are hydrogen atoms.

Further, when one of R⁷ and R⁸ is an alkoxy group and when the other is a hydrogen atom, there is excluded a case where X is a C₁-C₆ alkyl group and all of R³, R⁴, R⁵ and R⁶ are hydrogen atoms when hydrogen atom of the alkoxy group is not replaced with halogen or when the alkoxy group does not contain an unsaturated bond.

Specific examples of the halogen atom, C₁-C₆ alkyl group, C₁-C₆ alkoxy group and C₁-C₆ alkylthio group in the above R⁷, R⁸, R¹⁰ and R¹¹ are those specified with regard to X. Examples of the C₁-C₆ alkylcarbonyl group in the definition of R¹⁰ and R¹¹ include acetyl, propionyl, butylyl and valeryl, and the butylyl and valeryl may be linear, cyclic or branched.

In the group (b) in the definition of Z, R⁹ is an oxygen atom, a sulfur atom or a C₁-C₆ alkoxyimino group. When R⁹ is a C₁-C₆ alkoxyimino group, 1 to 13 hydrogen atoms of the group may be replaced with 1 to 13 halogen atoms, and when the number of carbon atoms thereof is C₂-C₆, the group may contain an unsaturated bond. However, when R⁹ is a C₁-C₆ alkoxyimino group, and when hydrogen atom thereof is not replaced with halogen or when the group does not contain an unsaturated bond, there is excluded a case where X is a C₁-C₆ alkyl group and all of R³, R⁴, R⁵ and R⁶ are hydrogen atoms. The alkoxyimino group is preferably methoxyimino or ethoxyimino.

Q is a hydroxyl group or a group of

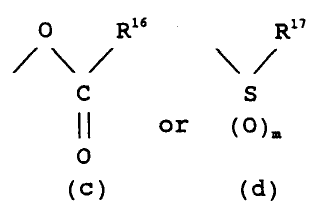

In the groups (c) and (d) in the definition of Q, each of R¹⁶ and R¹⁷ is a C₁-C₆ alkyl group, a C₁-C₆ haloalkyl group or a phenyl group which may be substituted with a C₁-C₆ alkyl group, a C₁-C₆ haloalkyl group, a halogen atom, a cyano group or a nitro group. Specific examples of the C₁-C₆ alkyl group and the C₁-C₆ haloalkyl group include those described with regard to the above X. Further, 0 to 5 C₁-C₆ alkyl groups, C₁-C₆ haloalkyl groups, halogen atoms, cyano groups and nitro groups may be introduced into the phenyl groups, and the positions of the substituents can be the 2-position to the 6-position. m is 0, 1 or 2, and it is 0 (sulfide), 1 (sulfoxide) or 2 (sulfone).

Of the cyclohexanedione derivatives of the general formula (I), preferred are cyclohexanedione derivatives of the general formula (I-a1), 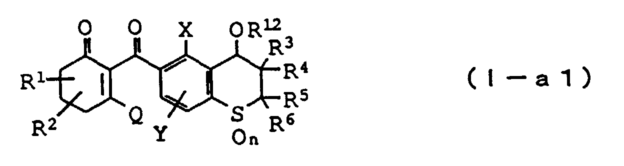 wherein R¹ to R⁶, n, X, Y and Q are as defined above, and R¹² is a C₁-C₆ alkyl group or a C₁-C₆ haloalkyl group, provided that R¹² may be substituted with a C₁-C₆ alkoxy group, that when the number of carbon atoms thereof is C₂-C₆, the group may contain an unsaturated bond, that when the number of carbon atoms thereof is C₃-C₆, the group may have a cyclic structure and further that when R¹² is a C₁-C₆ alkyl group, compounds of the general formula (I-a1) in which X is a C₁-C₆ alkyl group and all of R³, R⁴, R⁵ and R⁶ are hydrogen atoms are excluded.

Of the above cyclohexanedione derivatives, particularly preferred are cyclohexanedione derivatives of the general formula (I-a2), 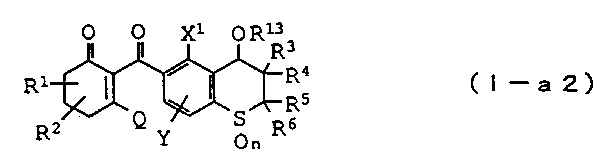
wherein R¹ to R⁶, n, Y and Q are as defined above, R¹³ is a C₁-C₆ alkyl group, and X¹ is a C₁-C₆ haloalkyl group, a halogen atom, a C₁-C₆ alkoxy group, a C₁-C₆ haloalkoxy group, a C₂-C₆ alkoxyalkyl group, a C₁-C₆ alkylthio group, a C₁-C₆ haloalkylthio group, a C₁-C₆ alkylsulfinyl group or a C₁-C₆ alkylsulfonyl group, and
cyclohexanedione derivatives of the general formula (I-a3), 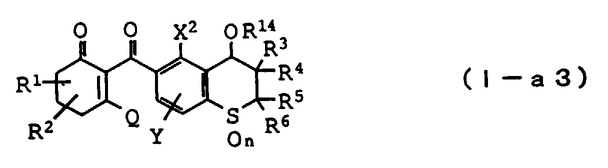
wherein R¹ to R⁶, n, Y and Q are as defined above, R¹⁴ is a C₁-C₆ haloalkyl group, an alkoxyalkyl group, an alkenyl group, a haloalkenylalkyl group or an alkynylalkyl group, and X² is a C₁-C₆ alkyl group, a C₁-C₆ haloalkyl group, a halogen atom, a C₁-C₆ alkoxy group, a C₁-C₆ haloalkoxy group, a C₂-C₆ alkoxyalkyl group, a C₁-C₆ alkylthio group, a C₁-C₆ haloalkylthio group, a C₁-C₆ alkylsulfinyl group or a C₁-C₆ alkylsulfonyl group.

Further, preferred are cyclohexanedione derivatives of the general formula (I-b1), 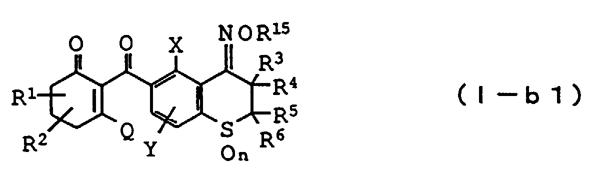 wherein R¹ to R⁶, n, X, Y and Q are as defined above, R¹⁵ is a C₁-C₆ alkyl group or a C₂-C₆ alkenyl group, provided that when R¹⁵ is a C₁-C₆ alkyl group, there are excluded compounds of the general formula (I-b1) in which X is a C₁-C₆ alkyl group and all of R³, R⁴, R⁵ and R⁶ are hydrogen atoms.

Of the above cyclohexanedione derivatives, particularly preferred are cyclohexanedione derivatives of the general formula (I-b2), 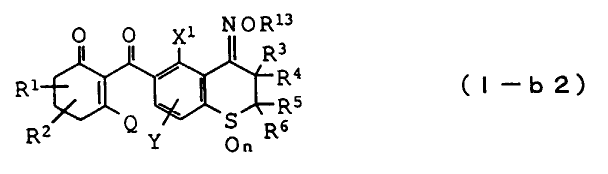 wherein R¹ to R⁶, R¹³, n, X¹, Y and Q are as defined above.

Further, preferred are also cyclohexanedione derivatives of the general formula (I-c), 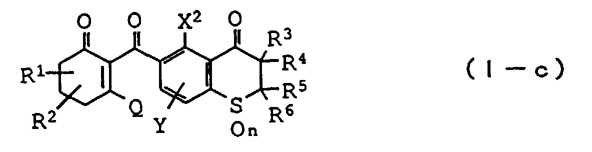 wherein R¹ to R⁶, n, X², Y and Q are as defined above.

The cyclohexanedione derivatives of the general formula (I) can have the following structures of tautomerism when Q is a hydroxyl group, and the cyclohexanedione derivative of the present invention includes all the compounds having these structures and mixtures of these. 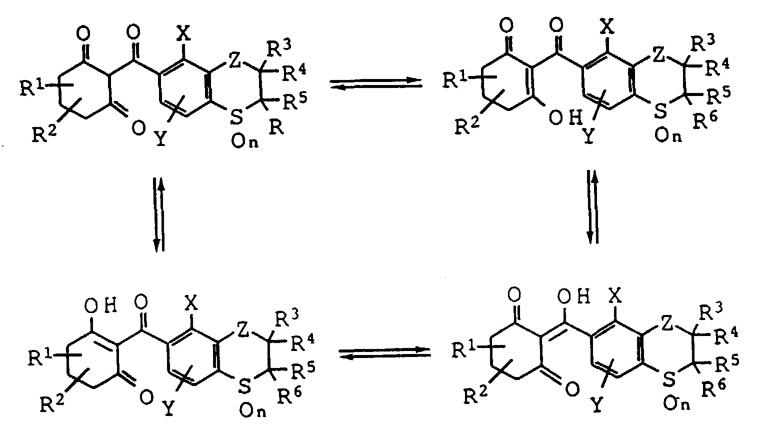 wherein X, Y, Z, R¹, R², R³, R⁴, R⁵, R⁶ and n are as defined above.

Further, the cyclohexanedione derivatives of the general formula (I) are acidic materials, and can be easily converted to salts by treating them with a base. The cyclohexanedione derivative of the present invention also includes these salts.

The above base can be selected from known bases without any limitation, and examples of the base include organic bases such as amines and anilines and inorganic bases such as sodium compounds and potassium compounds. Examples of the amines include alkylamines such as monoalkylamine, dialkylamine and trialkylamine. Alkyl groups of the alkylamines are generally C₁-C₄ alkyl groups . Examples of the anilines include aniline and alkylanilines such as monoalkylaniline and dialkylaniline. Alkyl groups of the alkylanilines are generally C₁-C₄ alkyl groups. Examples of the sodium compounds include sodium hydroxide and sodium carbonate. Examples of the potassium compounds include potassium hydroxide and potassium carbonate.

The cyclohexanedione derivatives of the general formula (I) can be produced, for example, by the following method when Q is OH [general formula (I-OH)]. 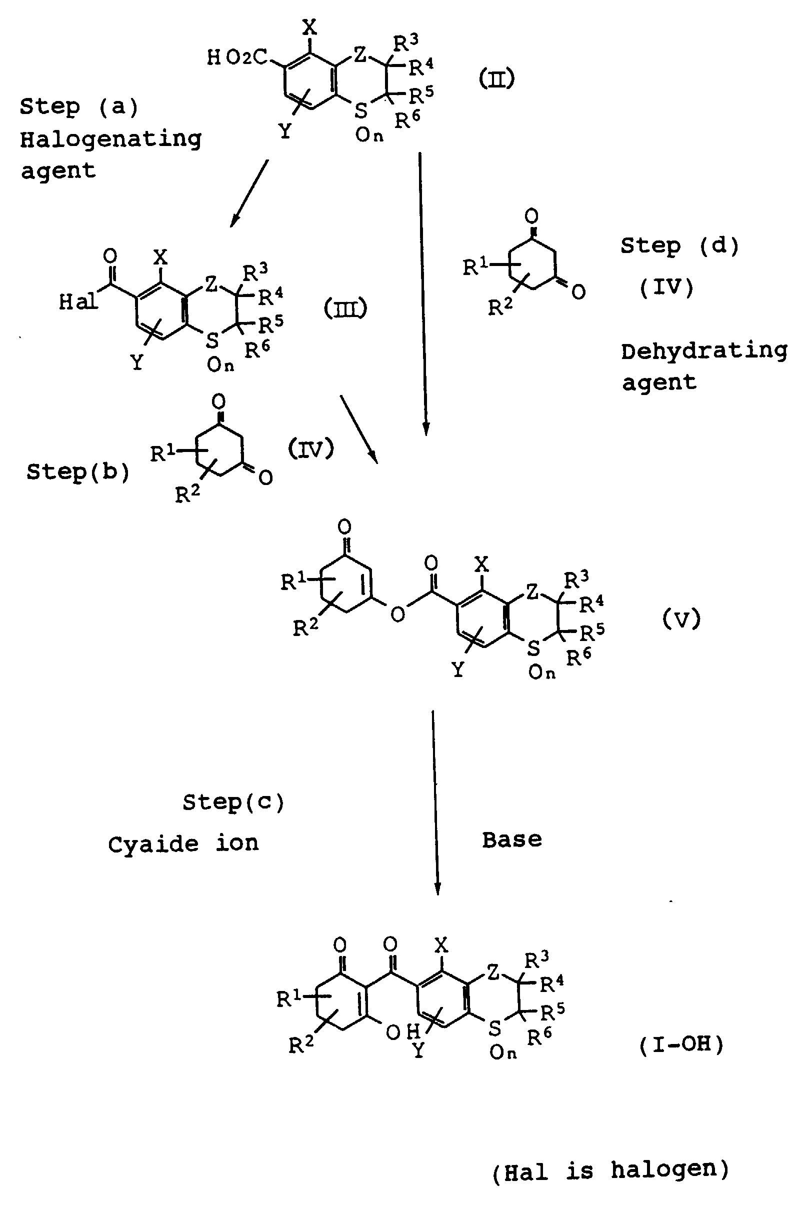 wherein X, Y, Z, R¹, R², R³, R⁴, R⁵, R⁶ and n are as defined above.

That is, Compound of the general formula (II) is reacted with a halogenating agent to obtain Compound of the general formula (III), and then the Compound of the general formula (III) is reacted with Compound of the general formula (IV) to obtain Compound of the general formula (V). Then, the Compound of the general formula (V) is subjected to a rearrangement reaction to obtain Cyclohexanedione derivative of the general formula (I-OH).

Further, Compound of the general formula (V) can be obtained by reacting Compound of the general formula (II) with Compound of the general formula (IV) in the presence of a dehydrating agent such as dicyclohexylcarbodiimide (to be referred to as "DCC" hereinafter).

Each step will be explained hereinafter.

### Step (a)

In step (a), Compound of the general formula (II) is reacted with a halogenating agent (thionyl chloride or phosphorus oxychloride) to obtain Compound of the general formula (III). In step (a), preferably, a halogenating agent is used in an amount of 1 mol or more per mole of Compound of the general formula (II). The reaction may be carried out in a diluted state in an inert solvent (methylene chloride or chloroform) or without any solvent. Further, an excess of thionyl chloride as a halogenating agent may be used to work it as a solvent. Although not specially limited, the reaction temperature is preferably 0°C to the boiling point of the solvent, particularly preferably a temperature of 60°C or around it.

### Step (b)

In step (b), Compound of the general formula (III) obtained in step (a) is reacted with Compound of the general formula (IV) to obtain Compound of the general formula (V). In step (b), preferably, the molar ratio of Compound of the general formula (III)/Compound of the general formula (IV) is preferably approximately 1/1 to 1/3, and the reaction is carried out in an inert solvent such as dioxane, acetonitrile, benzene, toluene, chloroform, methylene chloride or 1,2-dichloroethane. Further, the reaction may be carried out in a solvent of two-phase system such as water-benzene, water-toluene or water-chloroform. When a base such as sodium hydroxide, potassium hydroxide, sodium carbonate, potassium carbonate, triethylamine or pyridine is present in an equimolar amount or more, the reaction proceeds smoothly. The reaction temperature is preferably 0°C to 60°C, particularly preferably in the range of from 0°C to room temperature.

### Step (c)

In step (c), Compound of the general formula (V) obtained in step (b) is subjected to a rearrangement reaction to obtain Cyclohexanedione derivative of the general formula (I-OH). In step (c), preferably, the reaction is carried out in an inert solvent such as methylene chloride, 1,2-dichloroethane, toluene, acetonitrile, N,N-dimethylformamide or ethyl acetate. Acetonitrile is particularly preferred. In step (c), a proper base (sodium carbonate, potassium carbonate, triethylamine or pyridine) is used in an amount of, generally, 1 to 4 equivalent weights, preferably 1 to 2 equivalent weights, per equivalent weight of Compound of the general formula (V). In this case, the reaction smoothly proceeds in the catalytic presence of hydrogen cyanide or a compound which can generate cyanide anion in the reaction system, a so-called "cyanide source". The cyanide source is selected, for example, from metal cyanides such as sodium cyanide and potassium cyanide and cyanhydrin compounds of lower alkyl (C₃-C₅) ketones such as acetonecyanhyrdin and methylisopropylketonecyanhydrin. When the metal cyanide is used, the reaction can be smoothly proceeded with by adding a phase transfer catalyst such as a crown ether during the reaction. The amount of the cyanide source per mole of Compound of the general formula (V) is generally 0.01 to 0.5 mol, preferably 0.05 to 0.2 mol. The reaction temperature is preferably 0 to 80°C, particularly preferably in the range of from 20 to 40°C.

### Step (d)

Step (d) shows a method for obtaining Compound of the general formula (V), which method is different from the above method. That is, in step (d), Compound (V) is obtained from Compound (II) and Compound (IV) by condensation in the presence of a dehydrating agent such as DCC. Although not specially limited, the reaction solvent used for the above condensation is preferably selected from acetonitrile, a tertiary amine or an alcohol. The reaction temperature is not specially limited so long as it is in the range of from 0°C to the boiling point of the solvent, while the reaction temperature is preferably room temperature. The dehydrating agent can be selected from the above DCC or other agent such as 1,1-carbonyldiimidazole (CDI) or 1-(3-dimethylaminopropyl)-3-ethylcarbodiimide (EDC). The amount of the dehydrating agent based on the Compound is generally 1.0 to 3.0 equivalent weights, preferably 1.0 to 1.5 equivalent weights. The molar ratio of Compound (II)/Compound (IV) is generally 1/1 to 1/3, preferably 1/1 to 1/1.5. It is sufficient to carry out the condensation of Compound (II) and Compound (IV) for 1 to 48 hours, and the condensation is generally completed for about 8 hours.

Tables 1 to 23 show preferred embodiments of the cyclohexanedione derivatives of the general formula (I-OH) of the present invention, obtained as described above.

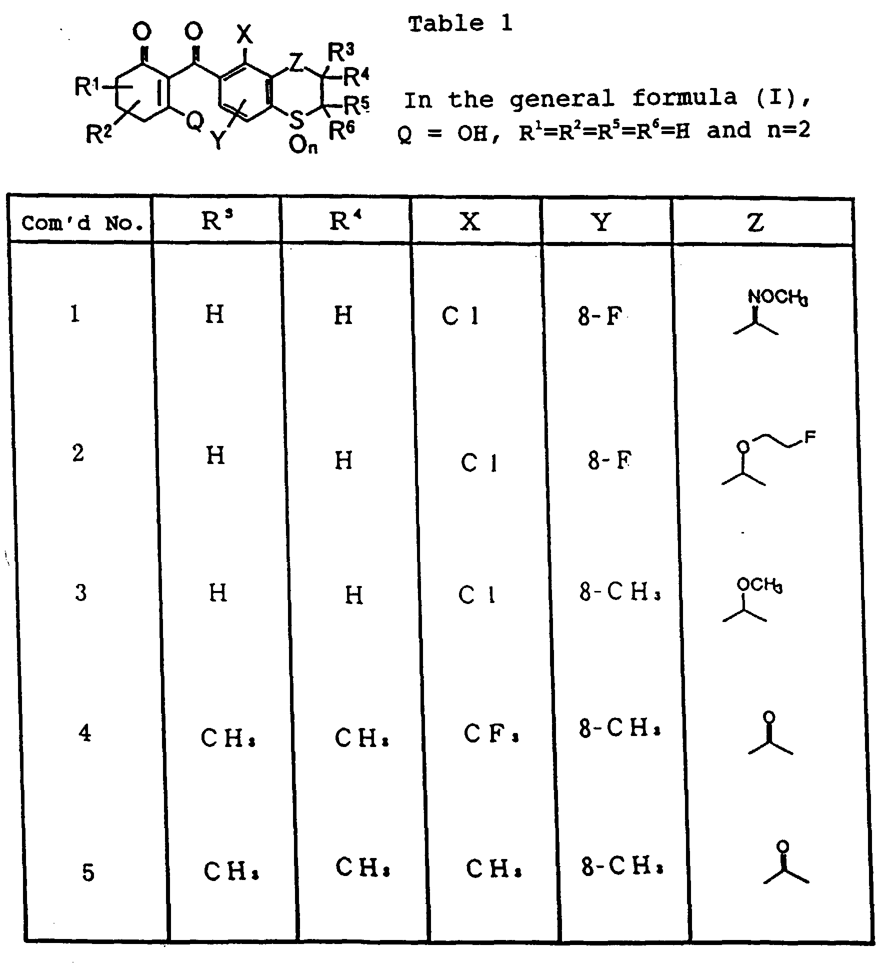

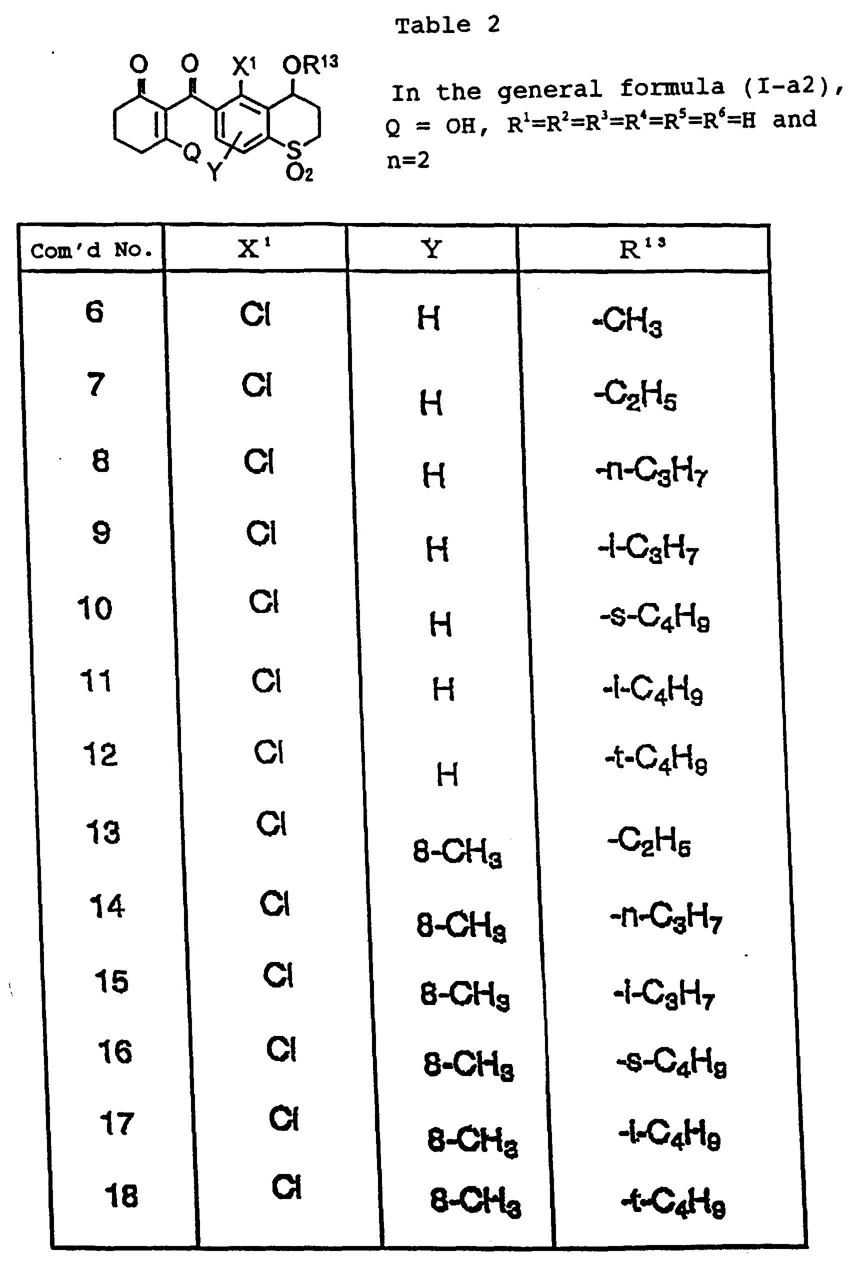

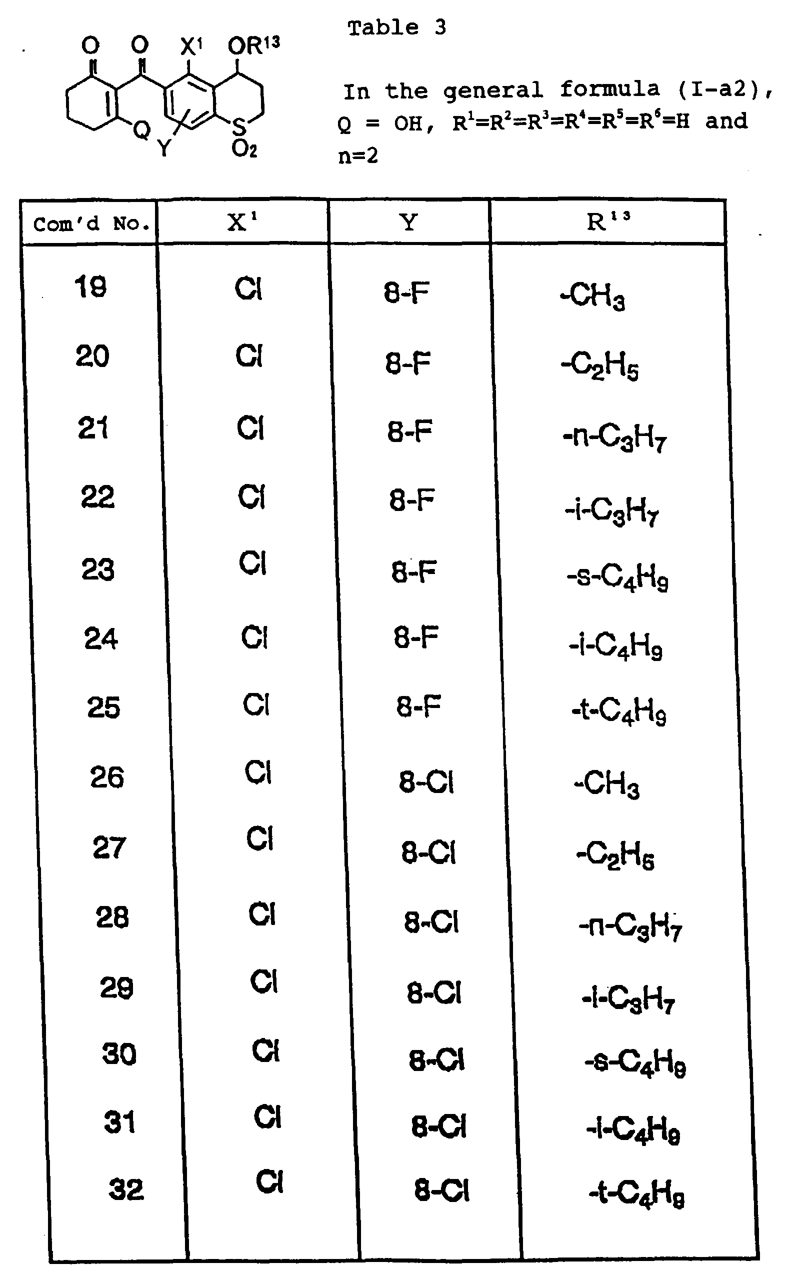

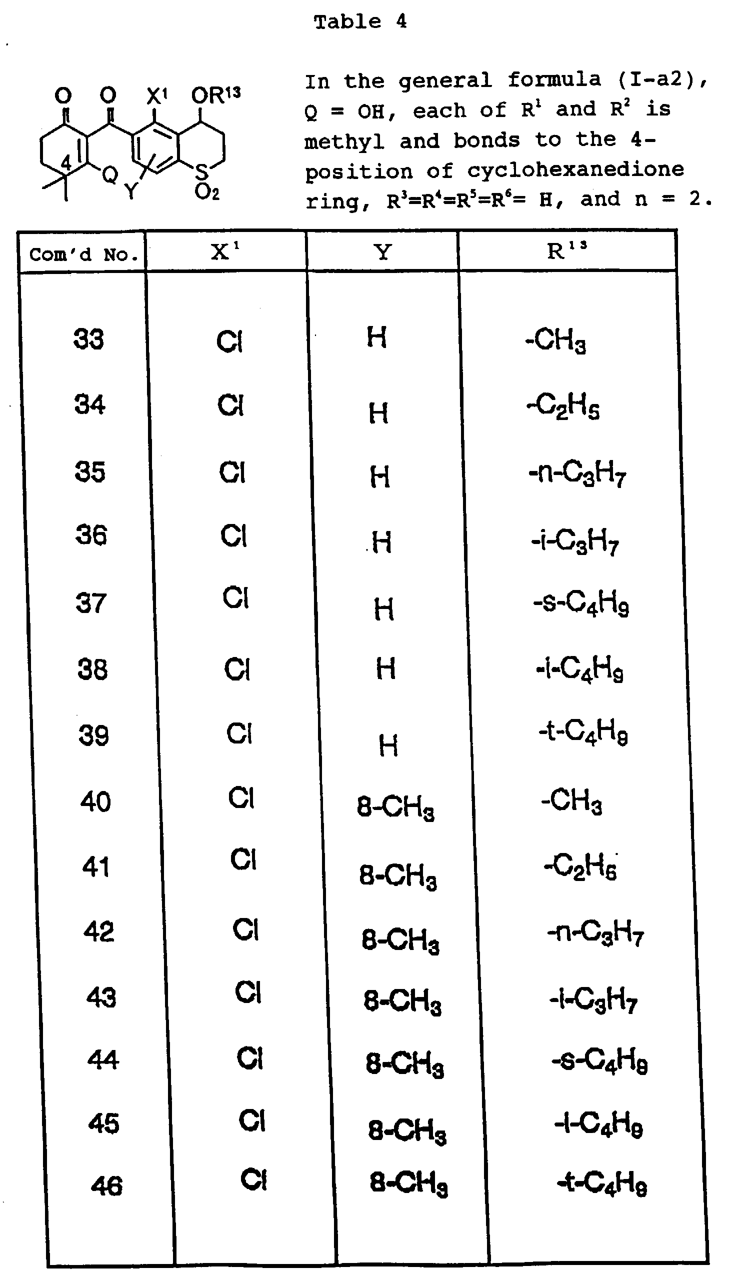

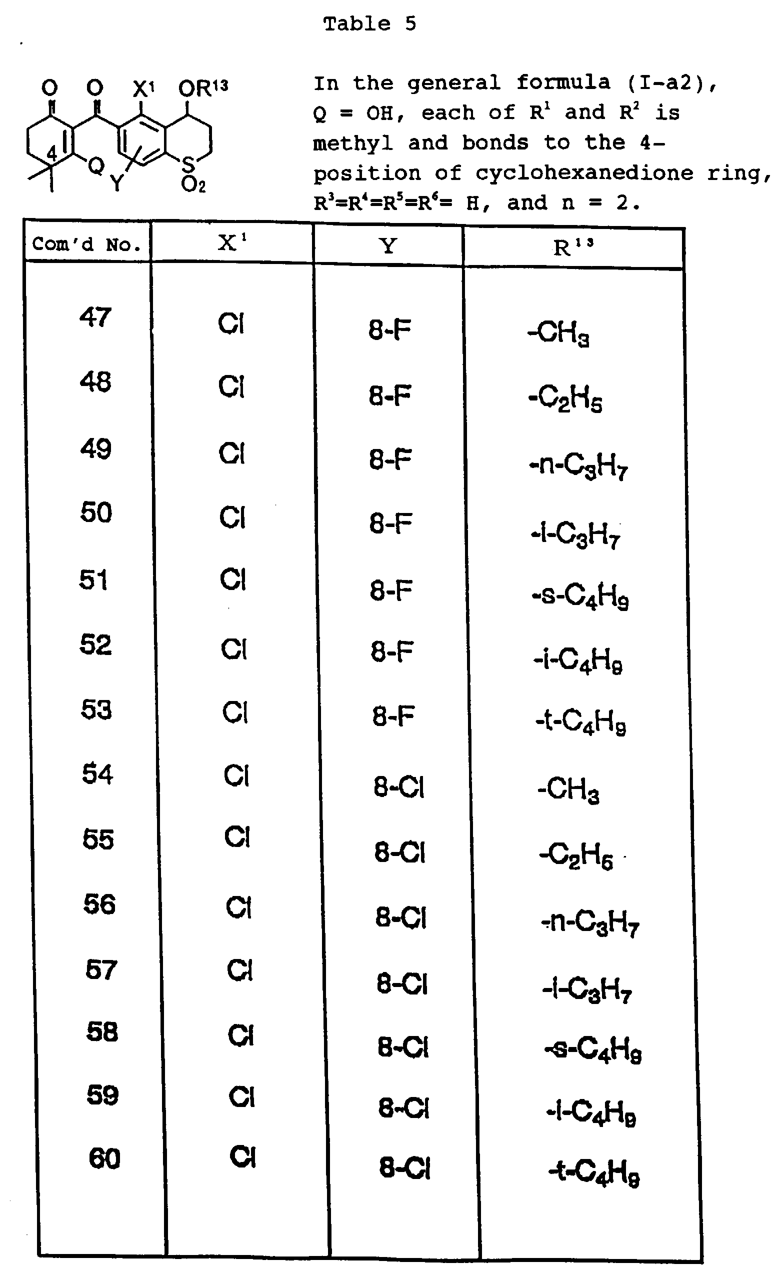

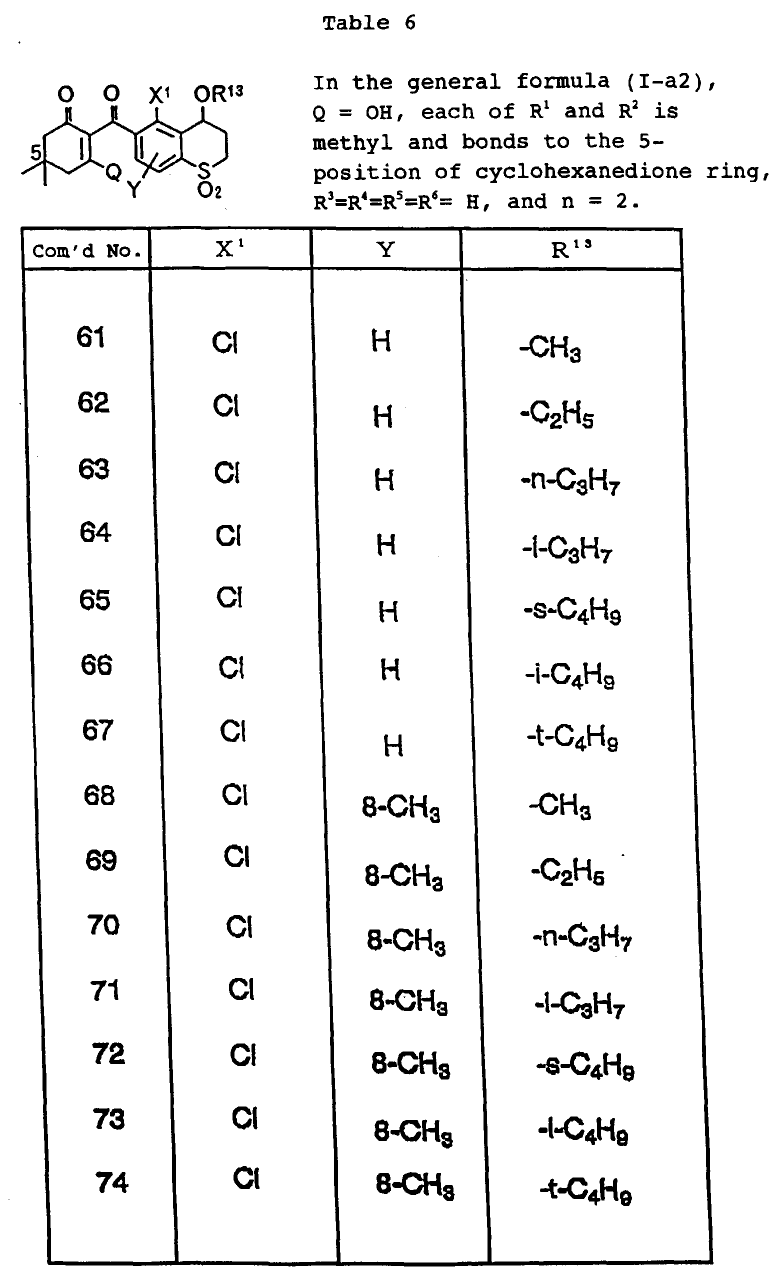

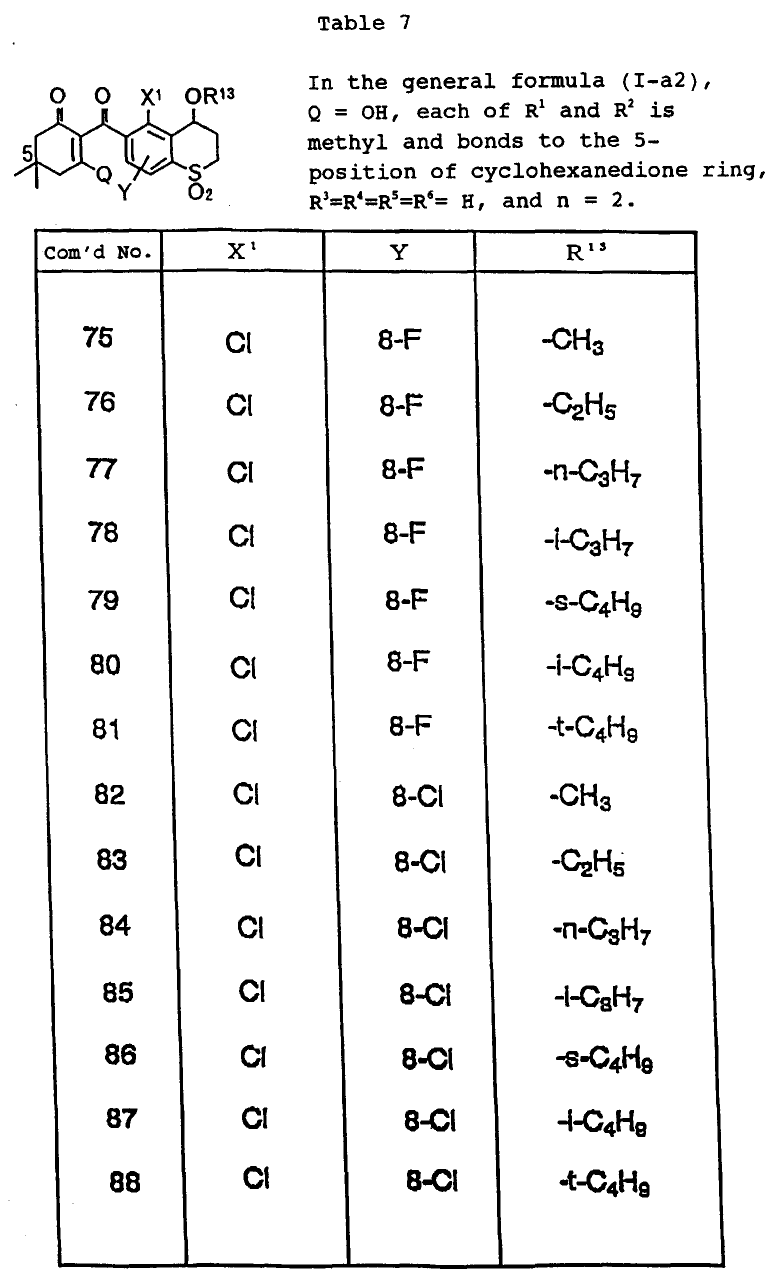

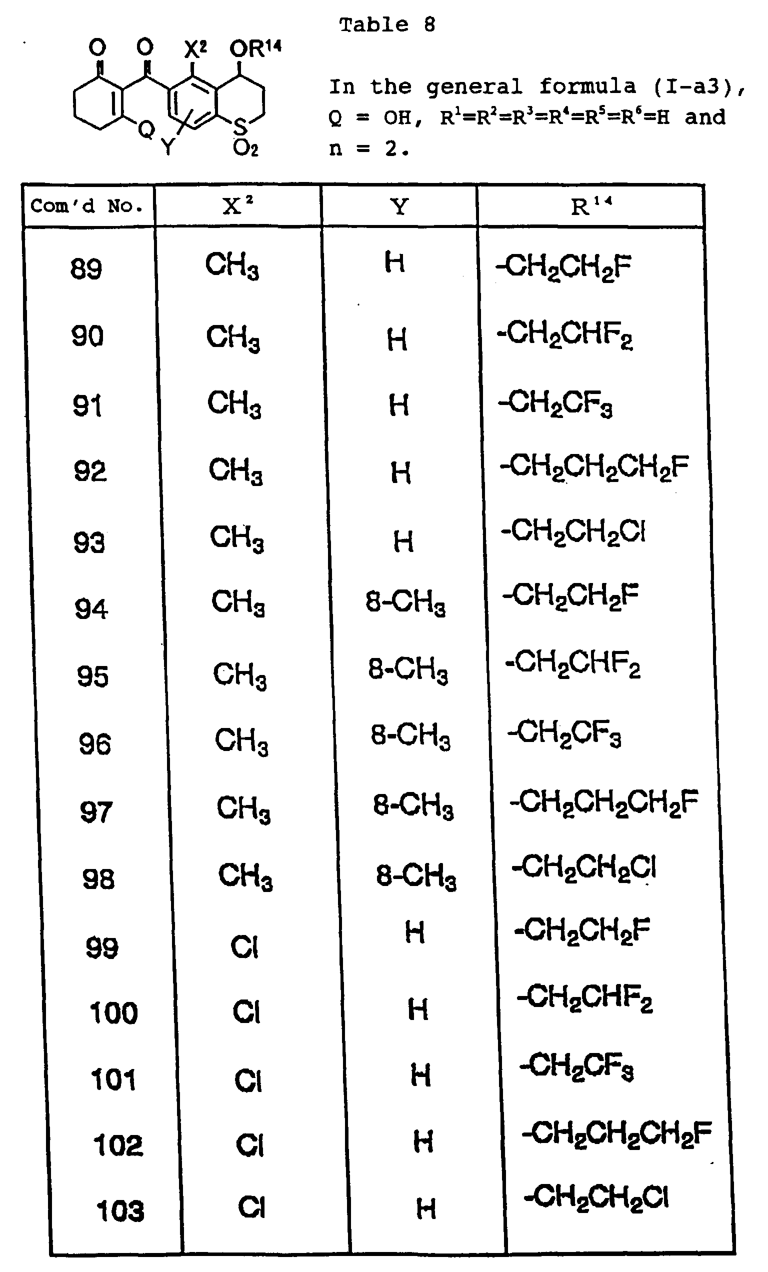

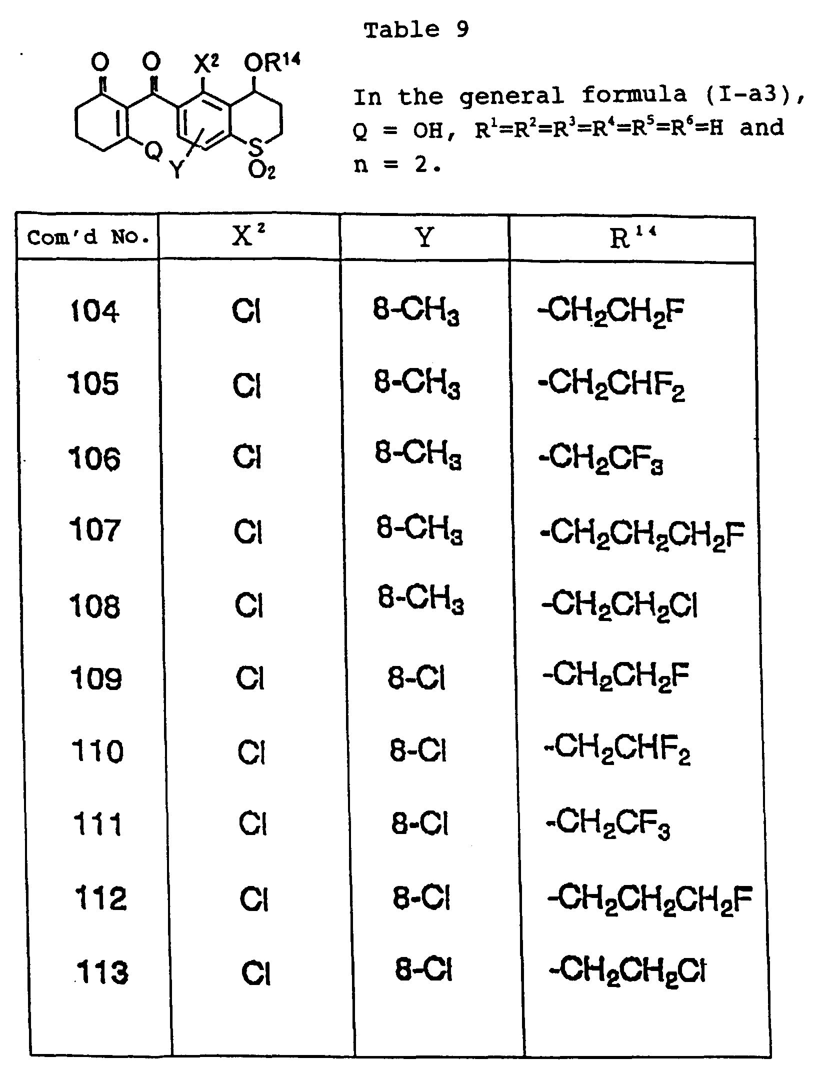

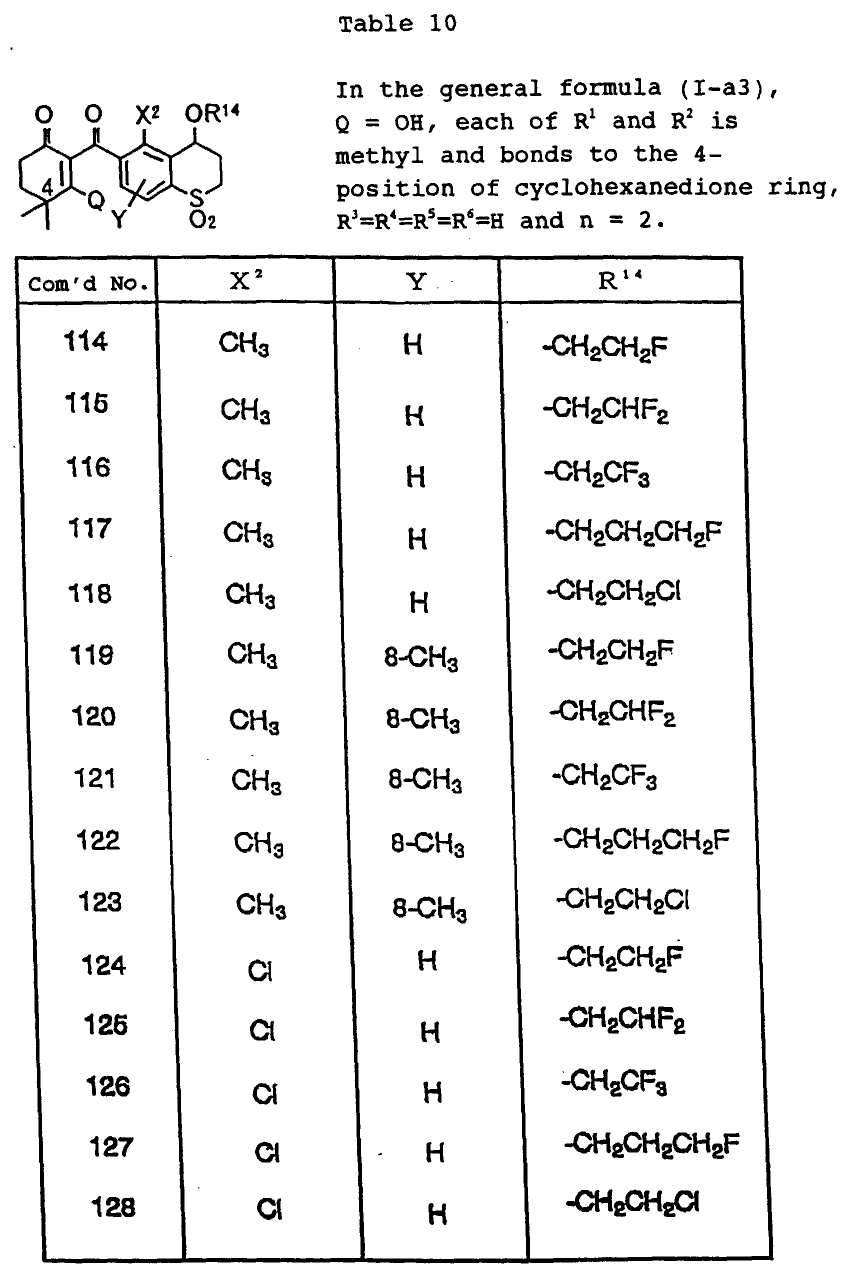

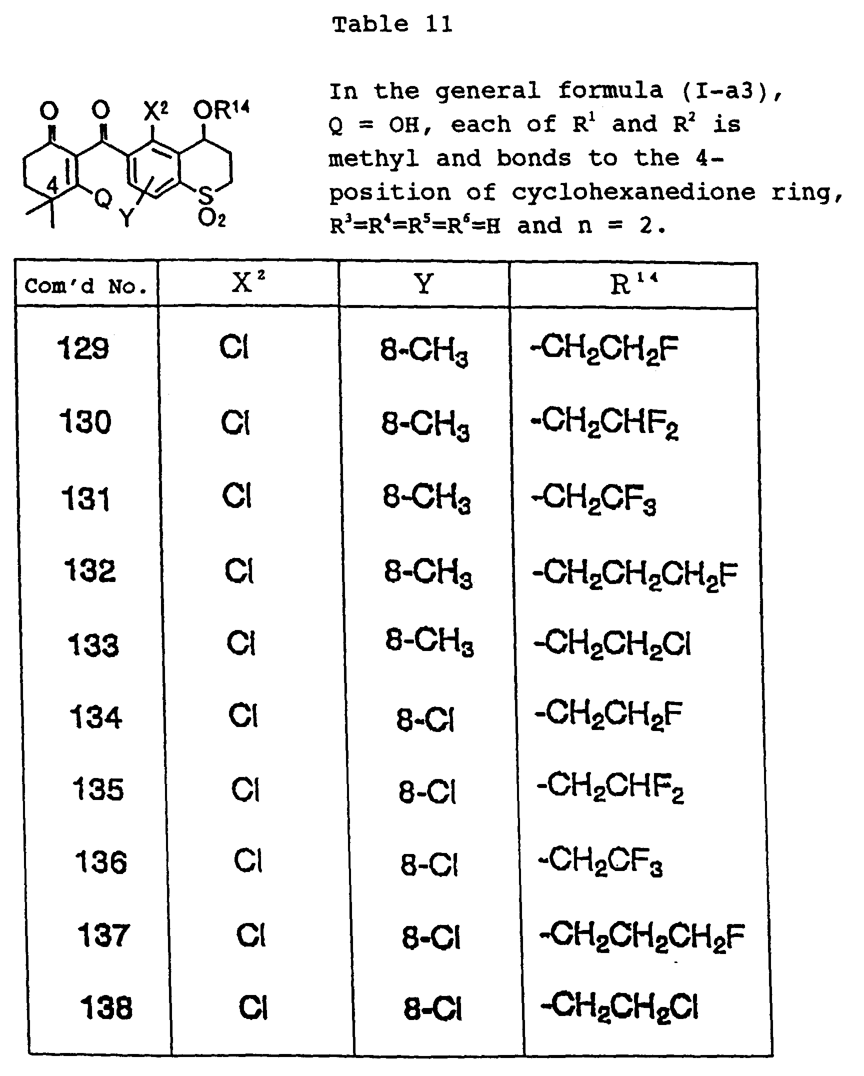

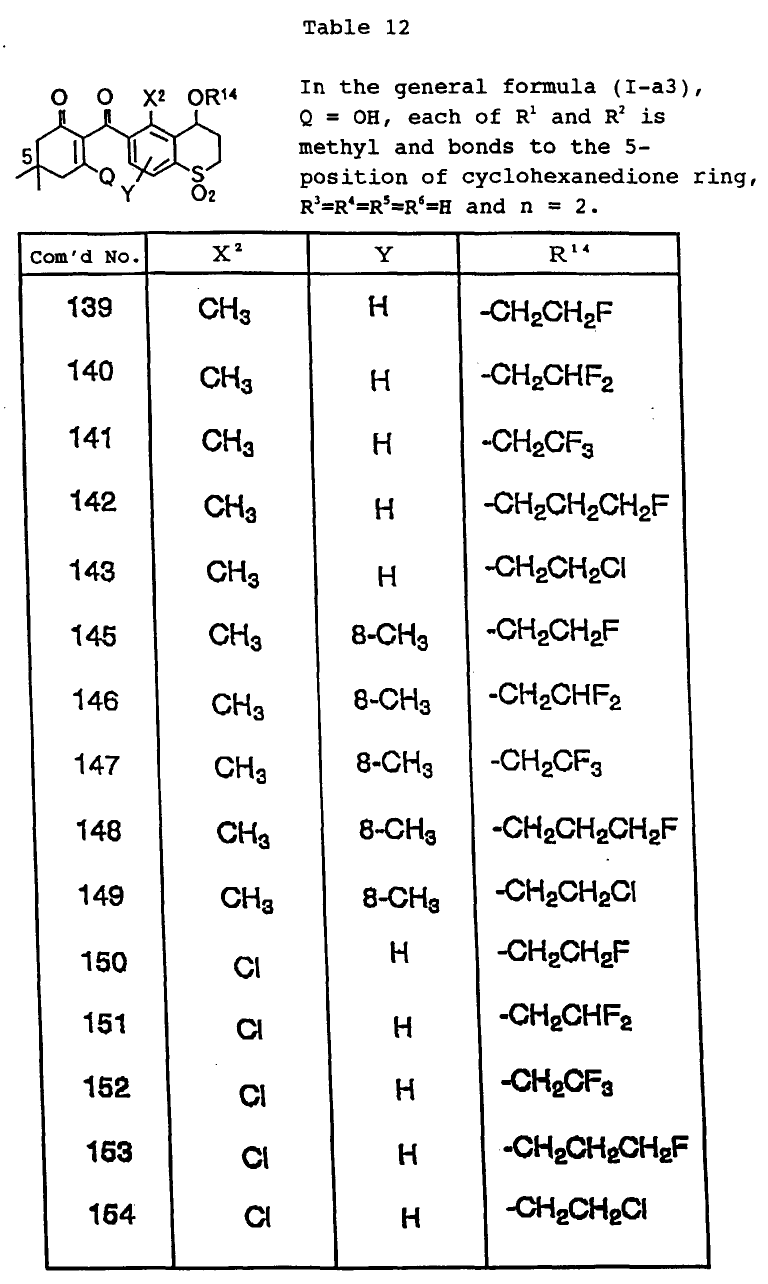

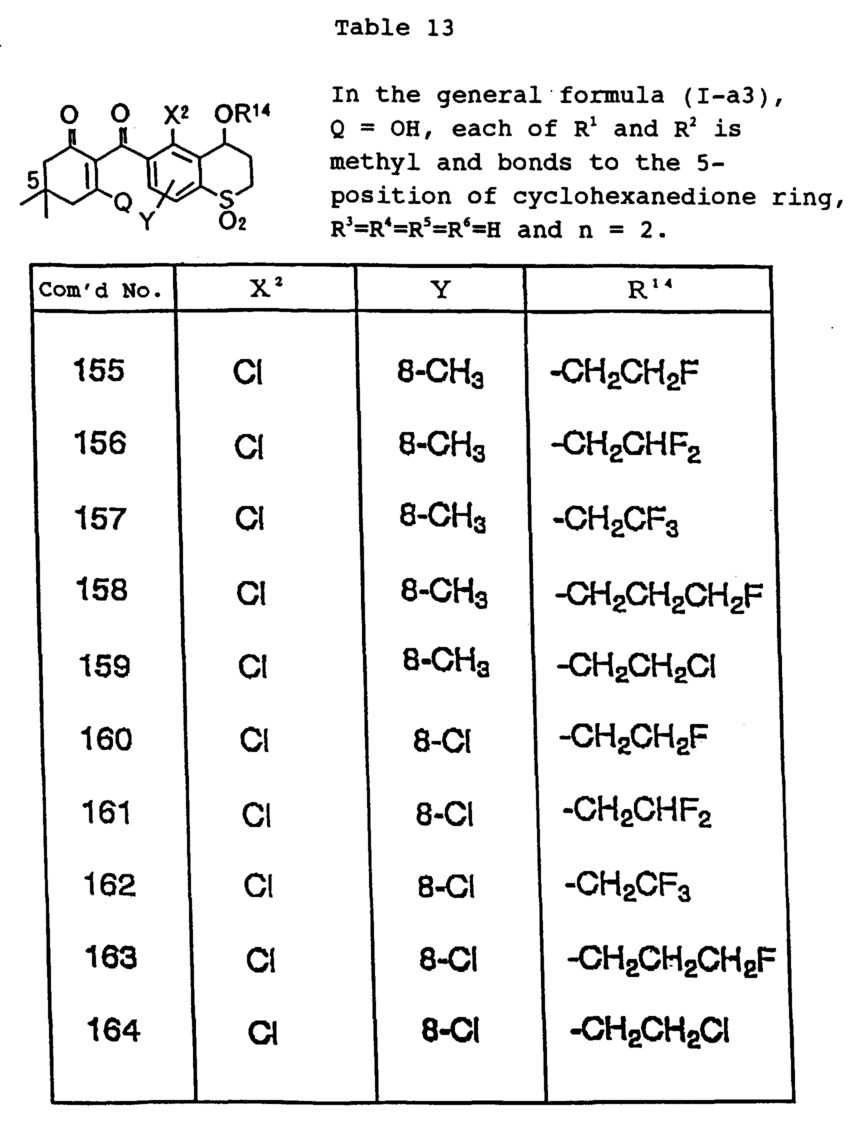

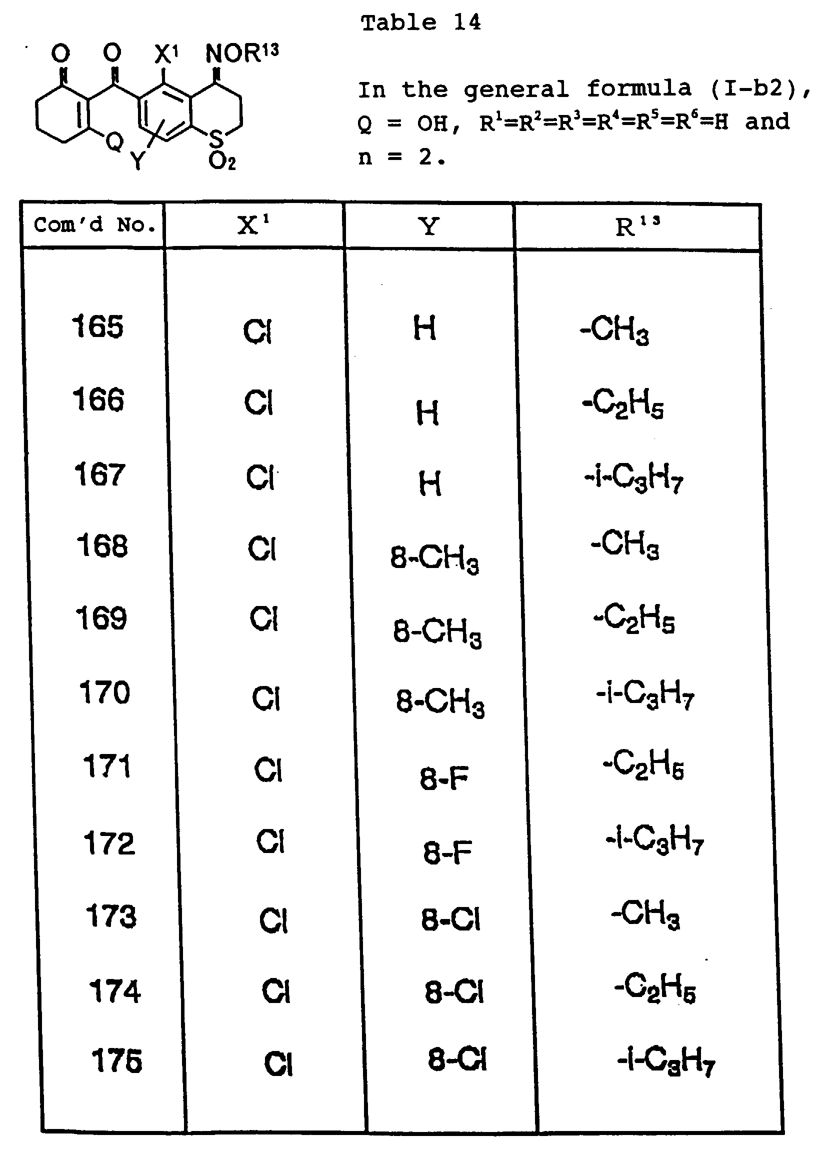

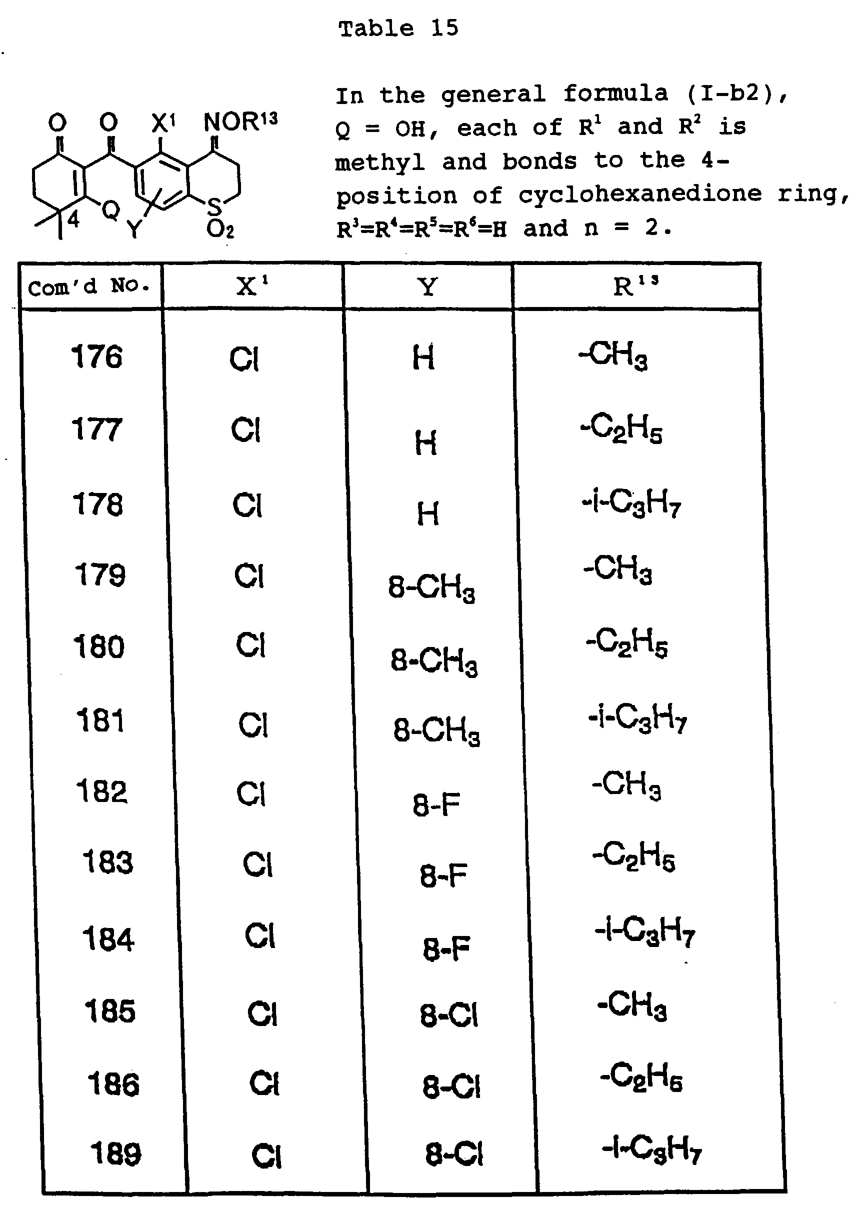

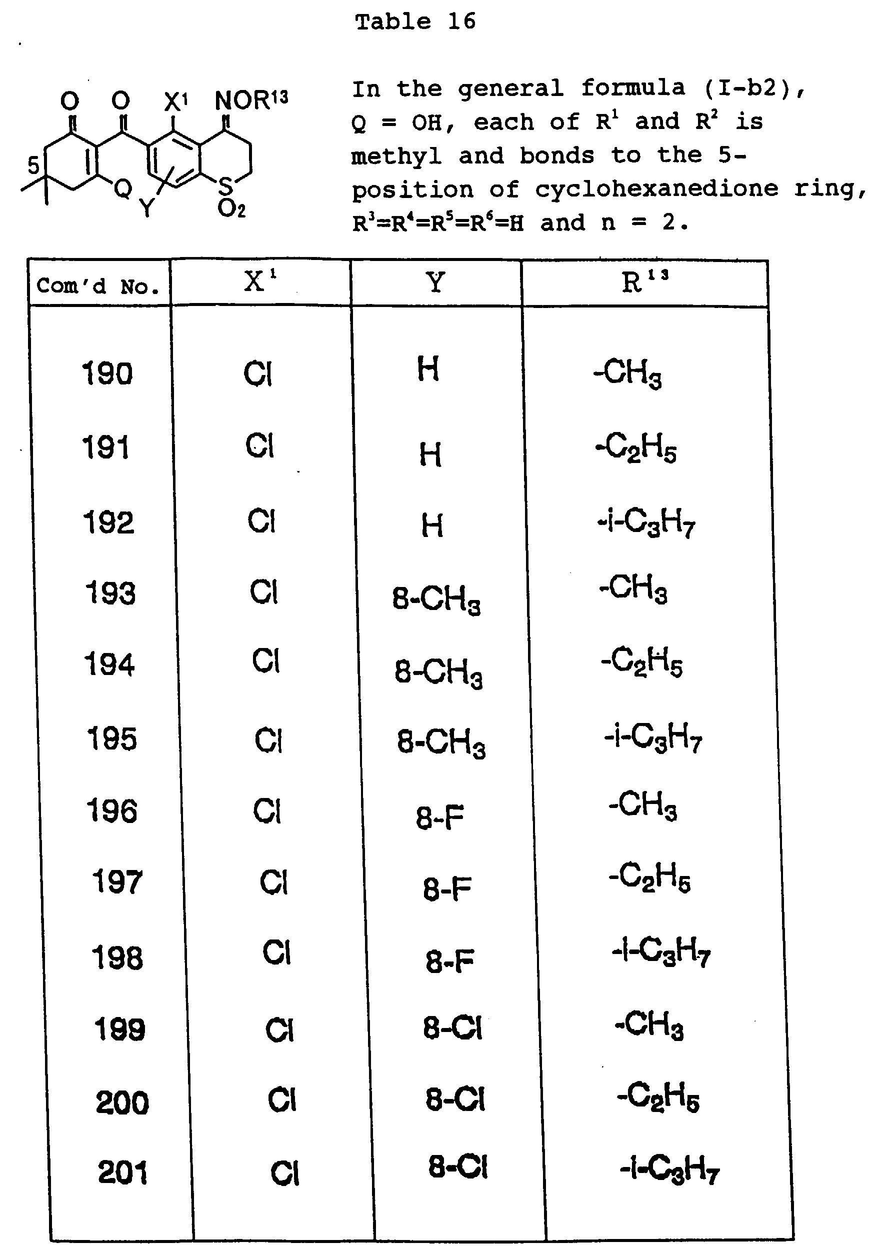

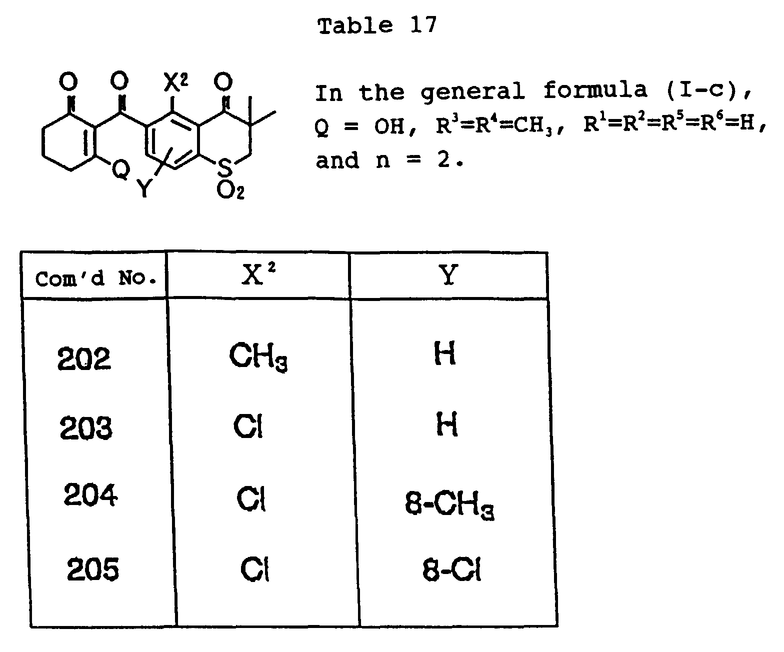

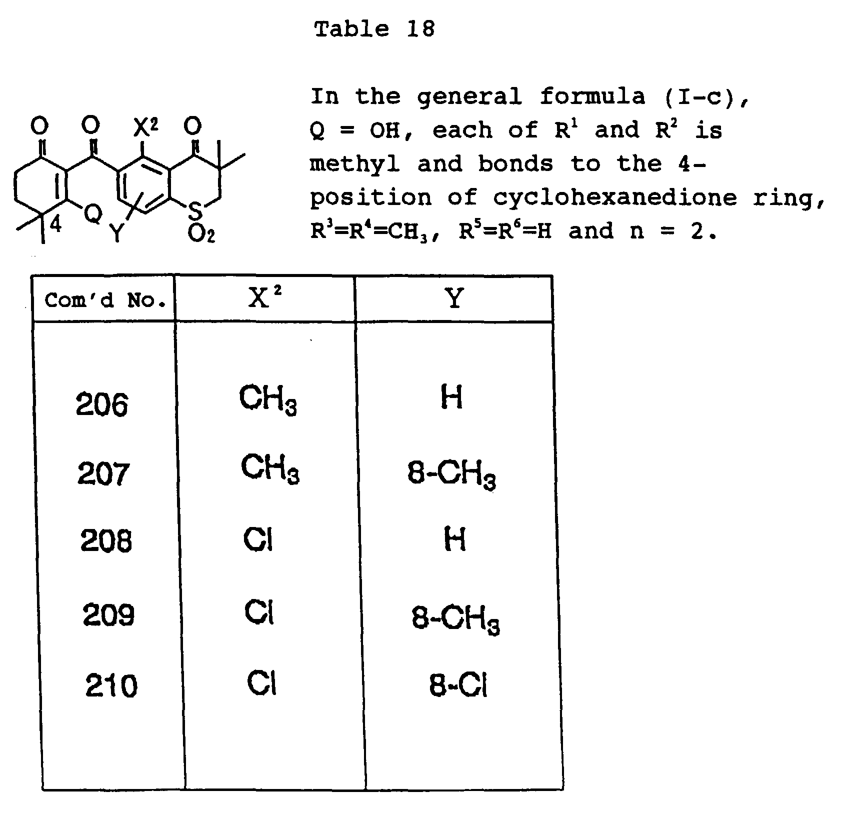

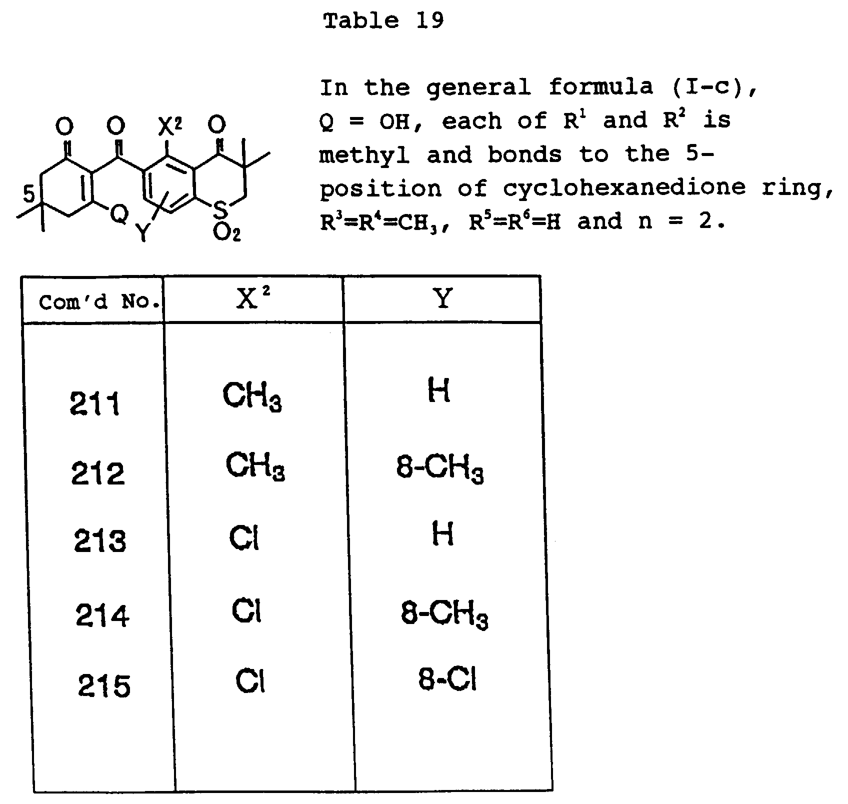

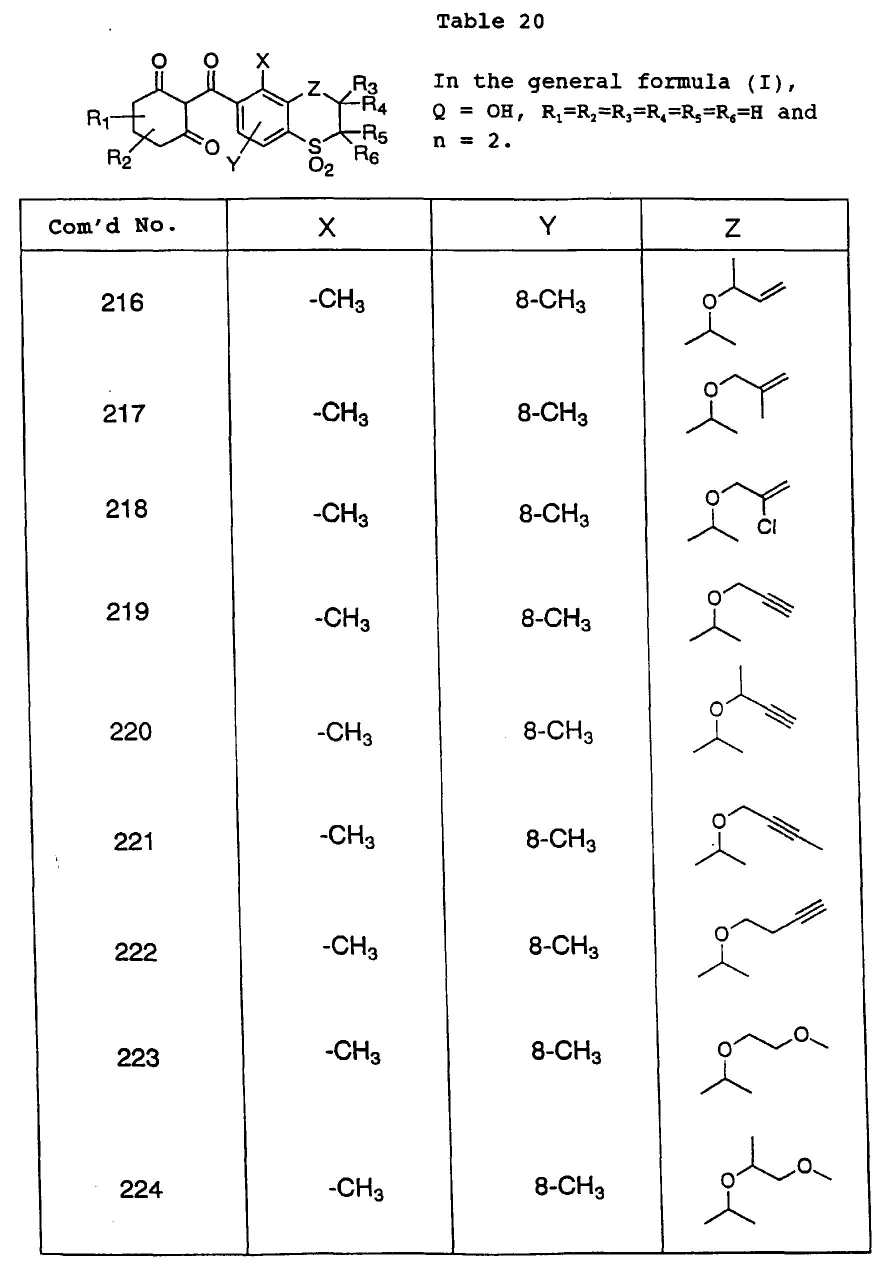

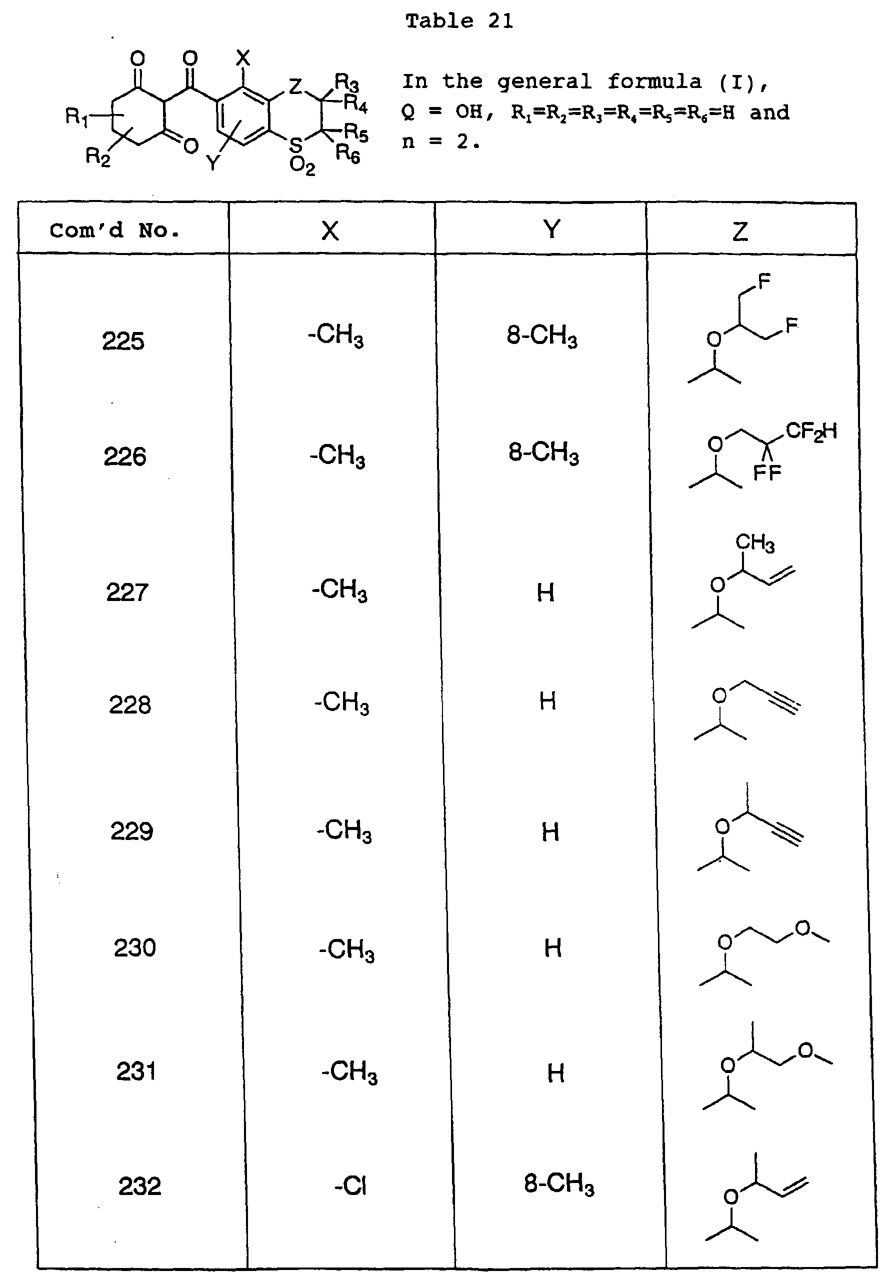

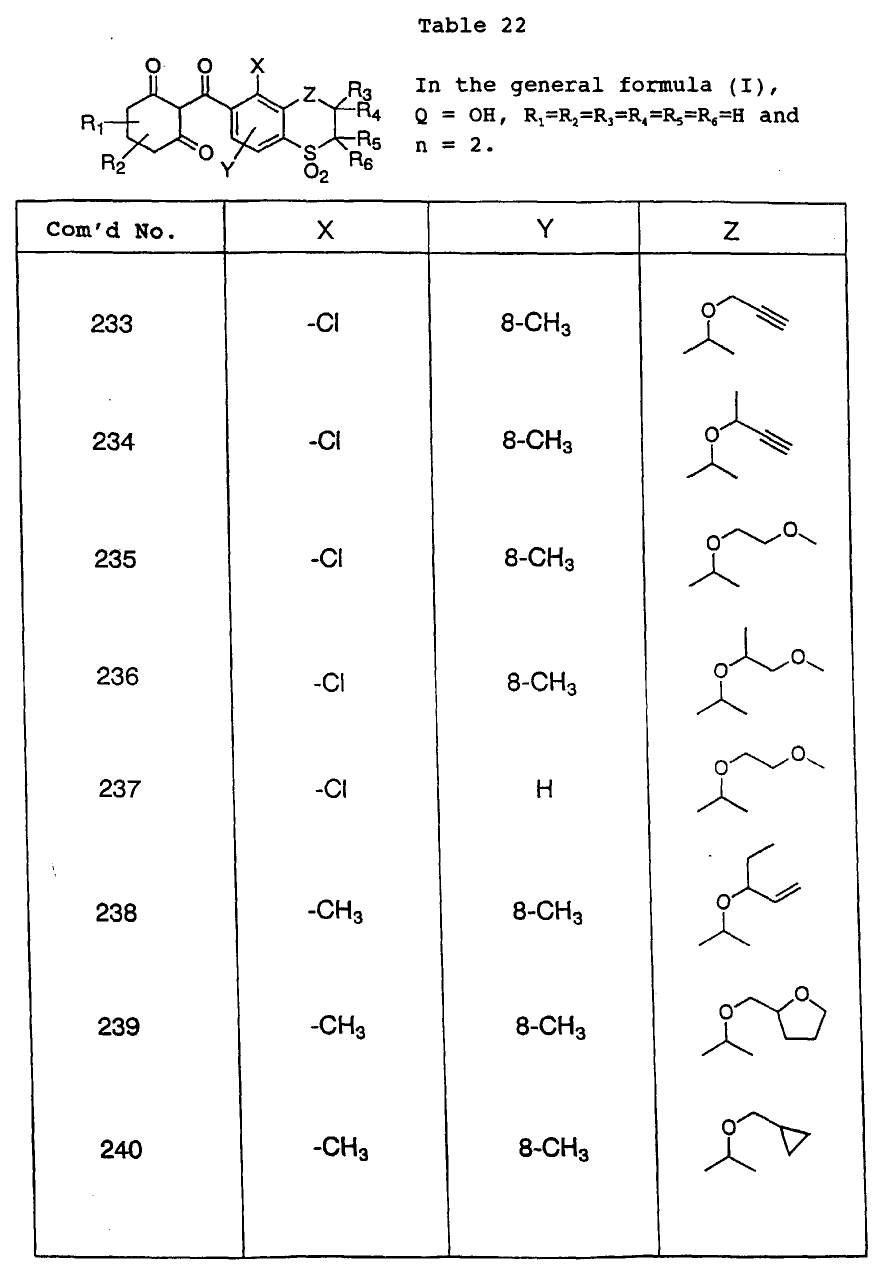

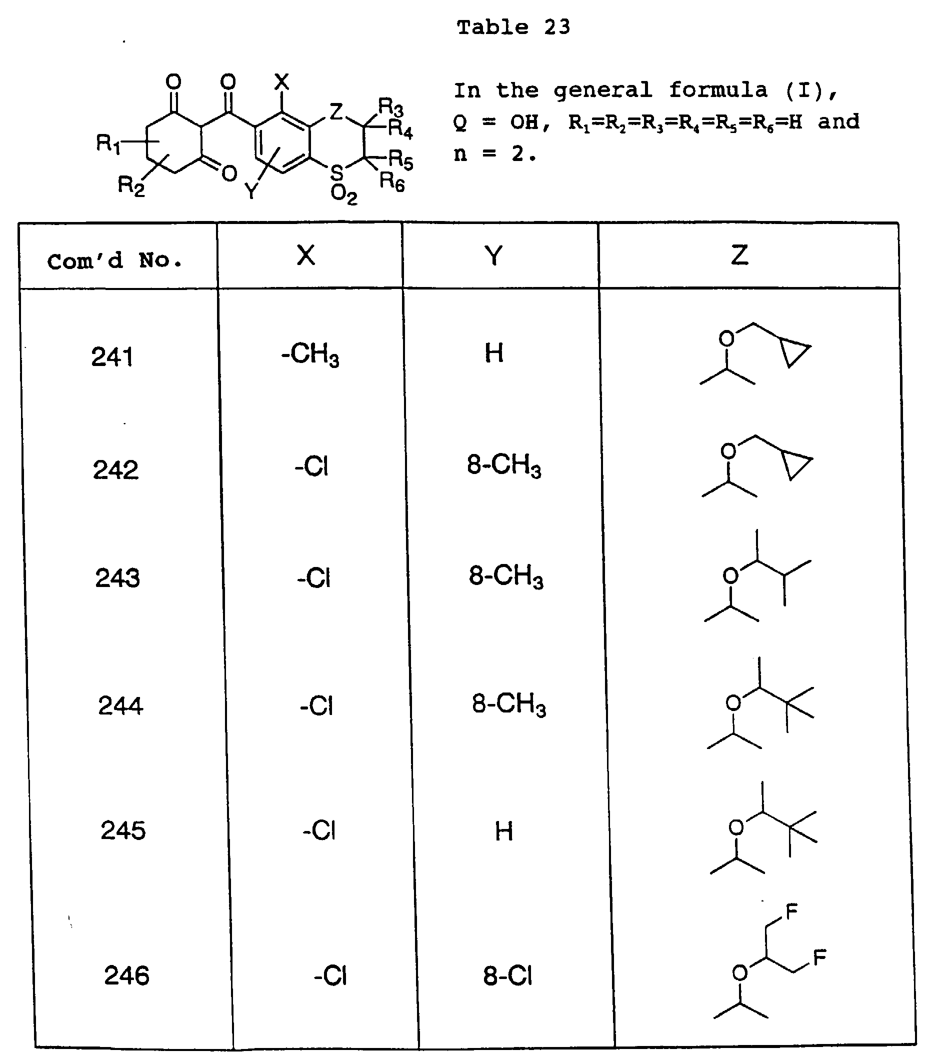

The cyclohexanedione derivatives of the general formula (I) in which Q is other than OH [general formula (I-Q)], provided by the present invention, are produced, for example, by the following method. 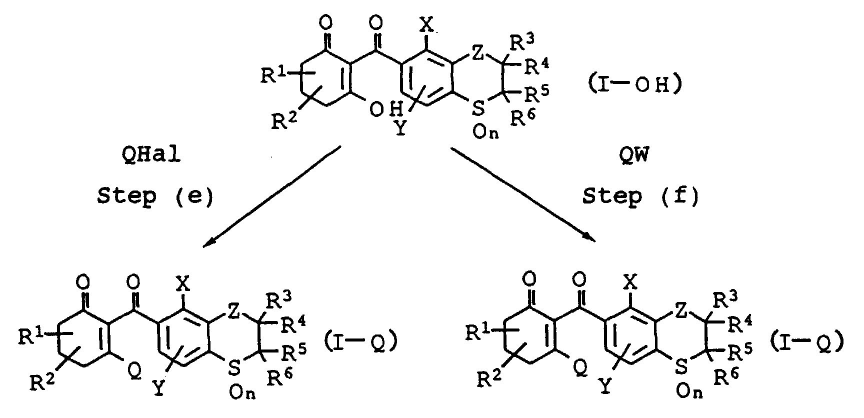 (Hal is a halogen.)
(W is a halogen, a hydroxyl group or a hydrogen atom.) wherein X, Y, Z, R¹, R², R³, R⁴, R⁵, R⁶ and n are as defined above.

That is, Compound of the general formula (I-OH) is reacted with Compound of QHal to obtain Compound of the general formula (I-Q).

Salts of the derivatives are obtained by a reaction thereof with a base Q.

Each step will be explained below.

### Step (e)

In step (e), Compound of the general formula (I-OH) is reacted with Compound represented by QHal to obtain Compound of (I-Q). In this step (e), preferably, Compound represented by QHal is used in an amount equimolar or greater than the amount of Compound of the general formula (I-OH), and the reaction is carried out in the presence of an organic base or an inorganic base. The reaction is preferably carried out in a solvent inert to the reaction, such as dioxane, benzene, toluene, chloroform, methylene chloride, 1,2-dichloroethane or tetrahydrofuran. Further, the reaction may be carried out in a two-phase system such as water-benzene or water-chloroform. The reaction smoothly proceeds in the presence of a base such as sodium hydroxide, potassium hydroxide, sodium carbonate, potassium carbonate, triethylamine or pyridine. The reaction temperature is preferably 0 to 60°C, particularly preferably in the range of from 0°C to room temperature.

### Step (f)

In step (f), Compound of the general formula (I-OH) is reacted with Compound represented by QW to obtain Compound of (I-Q). In this step (f), preferably, Compound represented by QX is used in an amount equimolar or greater than the amount of Compound of the general formula (I-OH). The reaction is preferably carried out in a solvent inert to the reaction, such as dioxane, benzene, toluene, chloroform, methylene chloride, 1,2-dichloroethane or tetrahydrofuran. The reaction smoothly proceeds. The reaction temperature is preferably 0 to 60°C, particularly preferably in the range of from 0°C to room temperature.

Table 24 shows preferred embodiments of the cyclohexanedione derivatives of the general formula (I-Q) obtained as described above.

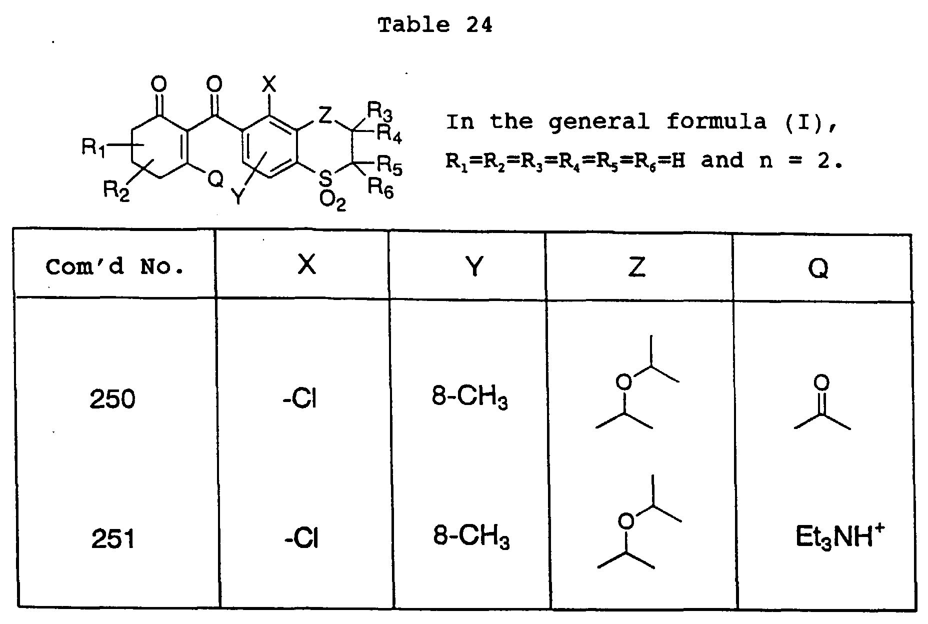

In Tables 1 to 24, for example, 8-F in the column of Y means that a fluorine atom is substituted on the 8-position of the thiochroman ring.

The herbicide of the present invention contains, as an essential component, an cyclohexanedione derivative of the general formula (I) provided by the present invention. The cyclohexanedione derivative of the present invention is mixed with a liquid carrier such as a solvent or a solid carrier such as a mineral fine powder, and the mixture can be prepared into the form of a wettable powder, an emulsifiable concentrate, a dust, granules, or the like. For imparting the preparation with emulsifiability, dispersibility and spreadability, a surfactant can be added.

When the herbicide of the present invention is used in the form of a wettable powder, generally, a composition is prepared by mixing 10 to 55 % by weight of the cyclohexanedione derivative of the present invention, 40 to 88 % by weight of a solid carrier and 2 to 5 % by weight of a surfactant, and the composition can be used. Further, when it is used in the form of an emulsifiable concentrate, generally, it can be prepared by mixing 20 to 50 % by weight of the cyclohexanedione derivative of the present invention, 35 to 75 % by weight of a solvent and 5 to 15 % by weight of a surfactant.

When the herbicide of the present invention is used in the form of a dust, generally, the dust can be prepared by mixing 1 to 15 % by weight of the cyclohexanedione derivative of the present invention, 80 to 97 % by weight of a solid carrier and 2 to 5 % by weight of a surfactant. Further, when it is used in the form of granules, the granules can be prepared by mixing 1 to 15 % by weight of the cyclohexanedione derivative of the present invention, 80 to 97 % by weight of a solid carrier and 2 to 5 % by weight of a surfactant. The above solid carrier can be selected from mineral fine powders, and the mineral fine powders include oxides such as diatomaceous earth and slaked lime, phosphates such as apatite, sulfates such as gypsum, and silicates such as talc, pyroferrite, clay, kaolin, bentonite, acid clay, white carbon, powdered quartz and powdered silica.

The solvent is selected from organic solvents. Specific examples of the solvent include aromatic hydrocarbons such as benzene, toluene and xylene, chlorinated hydrocarbons such as o-chlorotoluene, trichloroethane and trichloroethylene, alcohols such as cyclohexanol, amyl alcohol and ethylene glycol, ketones such as isophorone, cyclohexanone and cyclohexenyl-cyclohexanone, ethers such as butyl cellosolve, diethyl ether and methyl ethyl ether, esters such as isopropyl acetate, benzyl acetate and methyl phthalate, amides such as dimethylformamide, and mixtures of these.

Further, the surfactant can be selected from anionic, nonionic, cationic and amphoteric ones (amino acid and betaine).

The herbicide of the present invention may contain, as an active ingredient, other herbicidally active component in combination with the cyclohexanedione derivative of the general formula (I) as required. The "other" herbicidally active component includes known herbicides such as phenoxy-, diphenyl ether-, triazine-, urea-, carbamate-, thiocarbamate-, acid anilide-, pyrazole-, phosphoric acid-, sulfonylurea- and oxadiazone-based herbicides, and it can be properly selected from these herbicides as required.

Further, the herbicide of the present invention may be used as a mixture with any one of insecticides, bactericides, plant growth regulators and fertilizers.

The herbicide of the present invention can be used as a herbicide for upland soil by any method of pre-emergence treatment, treatment by mixing it with soil, and post-emergence treatment. The cropland weeds to which the compound of the present invention is applied include broad-leaved weeds such as solanaceous weeds typified by black nightshade (Solanum nigrum) and Jimsonweed (Datura stramonium); malvaceous weeds typified by velvetleaf (Abutilon theophrasti) and pricky sida (Sida spinosa); convolvulaceous weeds typified by morning-glories (Impmoea spps.) such as tall morning-glory (Ipomoea purpurea) and hedge bindweeds (Calystegia spps.); amaranthaceous weeds typified by livid amaranth (Amaranthus lividus); composite weeds typified by cocklebur (Xanthium strumarium), common ragweed (Ambrosia artemisiaefolia), sunflower (Helianthus annus), hairy galinsoga (Galinsoga ciliata), Canada thistle (Cirsium arvense), groundsel (Senecio vulgaris) and annual fleabane (Erigeron annus); brassicaceous weeds typified by yellow cress (Rorippa indica), wild mustard (Sinapis arvensis) and shepherdspurse (Capsella bursa-pastris); polygonaceous weeds typified by wild buckwheat (Polygonum convolvulus) and wild buckwheat (Polygonum convolvulus); portulacaceous weeds typified by common purslane (Portulaca oleracea); chenopodiaceous weeds typified by common lambsquaters (Chenopodium album), fig-leaved goosefoot (Chenopodium ficifolium) and kochia (Kochia scoparia); caryophyllaceous weeds typified by common chickweed (Stellaria media); scrophularaceous weeds typified by persian speedwell (Veronica persica); commelinaceous weeds typified by Asiatic dayfower (Commelina communis); labiatae weeds typified by henbit (Laminum amplexicaule) and purple deadnettle (Lamium purpureum); euphorbiaceous weeds typified by milk purslane (Euphorbia supina) and spotted spurge (Euphorbia maculata); rubiaceous weeds typified by bedstraw (Galium spurium), cleavers (Galium aparine) and madder (Rubia akane); violaceous weeds typified by violet (Viola arvensis); and leguminous weeds typified by hemp sesbania (Sesbania exaltata) and sicklepod (Cassia obtusifolia); graminaceous weeds typified by sorghum (Sorghum bicolor), fall panicum (Panicum dichotomiflorum), Johnsongrass (Sorghum halepense), barnyardgrass (Ehinocholoa crusgalli), henry crabgrass (Digitaria adscendens), wildoat (Avena fatua), goosegrass (Eleusine indica), green foxtail (Setaria viidis) and water foxtail (Alopecurus aequalis); and cyperaceous weeds typified by purple nutsedge (Cyperus rotundus, Cyperus esculentus).

Further, the herbicide of the present invention can be also used for any one of pre-emergence treatment and post-emergence treatment under submergence as a herbicide for paddy land. Examples of paddy weeds include alismataceous weeds typified by oriental waterplantain (Alisma canaliculatum), arrowhead (Sagittaria trifolia) and Sagittaria pygmaea, cyperaceous weeds typified by umbrella plant (Cyperus difformis), Cyperus serotinus, bulrush (Scirpus juncoides) and water chestnut (Eleochadaris kuroguwai); scrothulariaceous weeds typified by common falsepimpernel (Lindernia pyxidaria); potenderiaceous weeds typified by monochoria (Monochoria Vaginalis); potamogetonaceous weeds typified by largeleaf pondweed (Potamogeton distinctus); lythraceous weeds typified by toothcup (Rotala indica); and graminaceous weeds typified by barnyardgrass (Echinochloa crus-galli).

### Examples

The present invention will be explained more in detail with reference to Preparation Examples and Herbicide Examples hereinafter, while the present invention shall not be limited by these Examples.

### Preparation Example 1

### 5-Chloro-8-fluoro-6-(1',3'-dioxocyclohexan-2-yl-carbonyl)-4-methoxyiminothiochroman-1,1-dioxide (Compound No. 1)

### 1-1) Synthesis of 5-chloro-8-fluoro-6-carboxyl-4-methoxyiminothiochroman-1,1-dioxide

5-Chloro-8-fluoro-6-carboxyl-4-methoxyiminothiochroman-1,1-dioxide was synthesized according to the method described in WO96/30368.
¹H-NMR (CDCl₃): δ 3.35-3.45 (m,2H), 3.63-3.69 (m,2H), 4.08 (s,3H), 7.69 (d,1H)

### 1-2) Synthesis of 5-chloro-8-fluoro-6-(3'-oxocyclohexenyl)oxycarbonyl-4-methoxyiminothiochroman-1,1-dioxide

0.50 Gram (1.6 mmol) of 5-chloro-8-fluoro-6-carboxyl-4-methoxyiminothiochroman-1,1-dioxide was dissolved in 3 ml of dichloroethane, 0.34 ml (3.0 eq., 4.7 mmol) of thionyl chloride was added, and the mixture was refluxed under heat for 3 hours. Then, the solvent was distilled off, to give an acid chloride. Then, a solution of the obtained acid chloride in tetrahydrofuran was added to a solution of 0.18 g (1.0 eq., 1.6 mmol) of 1,3-cyclohexanedione in tetrahydrofuran, and further, 0.2 ml (1.0 eq., 1.6 mmol) of triethylamine was dropwise added. The mixture was stirred at room temperature for 2 hours, and the solvent was distilled off. The resultant residue was dissolved in ethyl acetate and consecutively washed with a 0.2 N hydrochloric acid aqueous solution, with a saturated sodium hydrogencarbonate aqueous solution and with a saturated sodium chloride aqueous solution. Then, the washed product was dried over anhydrous sodium sulfate, and the solvent was distilled off. The residue was subjected to column chromatography (ethyl acetate:n-hexane = 1:1) to give 0.21 g (yield 58 %) of 5-chloro-8-fluoro-6-(3'-oxocyclohexenyl)oxycarbonyl-4-methoxyiminothiochroman-1,1-dioxide.
¹H-NMR (CDCl₃): δ 2.0-2.7 (m,6H), 3.4-3.5 (m,4H), 4.11 (s,3H), 6.06 (bs,1H), 7.54 (d,1H)

### 1-3) Synthesis of 5-chloro-8-fluoro-6-(1',3'-dioxocyclohexan-2-yl-carbonyl)-4-methoxyiminothiochroman-1,1-dioxide

0.22 Gram (0.53 mmol) of 5-chloro-8-fluoro-6-(3'-oxocyclohexenyl)oxycarbonyl-4-methoxyiminothiochroman-1,1-dioxide was dissolved in 4 ml of acetonitrile, 0.1 ml (1.0 eq., 0.53 mmol) of triethylamine and 3 drops of acetonecyanhydrin were added, and the mixture was stirred at room temperature for 1 day. After the completion of the reaction, the reaction mixture was extracted with a saturated sodium hydrogencarbonate aqueous solution, and an aqueous layer was washed with methylene chloride. The aqueous layer was neutralized with 2 % hydrochloric acid and extracted with ethyl acetate. An organic layer was washed with a saturated sodium chloride aqueous solution and dried over anhydrous sodium sulfate, and the solvent was distilled off to give 0.22 g (yield 100 %) of 5-chloro-8-fluoro-6-(1',3'-dioxocyclohexan-2-yl-carbonyl)-4-methoxyiminothiochroman-1,1-dioxide.
¹H-NMR (CDCl₃): δ 2.0-2.8 (m,7H), 3.3-3.4 (m,2H), 3.6-3.7 (m,2H), 4.05 (s,3H), 7.32 (d,1H)
IR (KBr): 2950, 1710, 1680, 1250, 1150cm⁻¹

### Preparation Example 2

### 5-Chloro-8-fluoro-6-(1',3'-dioxocyclohexan-2-yl-carbonyl)-4-(2'-fluoroethoxy)thiochroman-1,1-dioxide (Compound No. 2)

### 2-1) Synthesis of 5-chloro-8-fluoro-6-carboxyl-4-(2'-fluoroethoxy)thiochroman-1,1-dioxide

5-Chloro-8-fluoro-6-carboxyl-4-(2'-fluoroethoxy)thiochroman-1,1-dioxide was synthesized according to the method described in WO96/31507.
¹H-NMR (acetone-d₆): δ 2.3-3.2 (m,2H), 3.3-4.5 (m,5H), 4.88 (t,1H), 5.07 (m,1H), 7.79 (d,1H)
m.p. 163 - 165°C

### 2-2) Synthesis of 5-chloro-8-fluoro-6-(3'-oxocyclohexenyl)oxycarbonyl-4-(2'-fluoroethoxy)thiochroman-1,1-dioxide

0.47 Gram (1.4 mmol) of 5-chloro-8-fluoro-6-carbonyl-4-(2'-fluoroethoxy)thiochroman-1,1-dioxide was dissolved in 3 ml of dichloroethane, 0.20 ml (2.0 eq., 2.8 mmol) of thionyl chloride was added, and the mixture was stirred at 40 - 50°C for 3 hours. Then, the solvent was distilled off to give an acid chloride. Then, a solution of the obtained acid chloride in tetrahydrofuran was added to a solution of 0.17 g (1.0 eq., 1.4 mmol) of 1,3-cyclohexanedione in tetrahydrofuran, and further, 0.2 ml (1.0 eq., 1.6 mmol) of triethylamine was dropwise added. The mixture was stirred at room temperature for 2 hours, and the solvent was distilled off. The resultant residue was dissolved in ethyl acetate and consecutively washed with a 0.2 N hydrochloric acid aqueous solution, with a saturated sodium hydrogencarbonate aqueous solution and with a saturated sodium chloride aqueous solution. Then, the washed product was dried over anhydrous sodium sulfate, and the solvent was distilled off. Thereafter, the residue was subjected to column chromatography (ethyl acetate:n-hexane = 1:1) to give 0.50 g (yield 81 %) of 5-chloro-8-fluoro-6-(3'-oxocyclohexenyl)oxycarbonyl-4-(2'-fluoroethoxy)thiochroman-1,1-dioxide.
¹H-NMR (CDCl₃): δ 2.0-2.8 (m,8H), 3.0-4.3 (m,4H), 4.85 (t,1H), 4.96 (bs,1H), 6.06 (bs,1H), 7.54 (d,1H)

### 2-3) Synthesis of 5-chloro-8-fluoro-6-(1',3'-dioxocyclohexan-2-yl-carbonyl)-4-(2'-fluoroethoxy)-1,1-dioxide

0.50 Gram (1.1 mmol) of 5-chloro-8-fluoro-6-(3'-oxocyclohexenyl)oxycarbonyl-4-(2'-fluoroethoxy)thiochroman-1,1-dioxide was dissolved in 3 ml of acetonitrile, 0.15 ml (1.0 eg., 1.1 mmol) of triethylamine and 3 drops of acetonecyanhydrin were added, and the mixture was stirred at room temperature for 1 day. After the completion of the reaction, the reaction mixture was extracted with a saturated sodium hydrogencarbonate aqueous solution, and an aqueous layer was washed with methylene chloride. The aqueous layer was neutralized with 2 % hydrochloric acid and extracted with ethyl acetate. An organic layer was washed with a saturated sodium chloride aqueous solution and dried over anhydrous sodium sulfate, and the solvent was distilled off to give 0.50 g (yield 100 %) of 5-chloro-8-fluoro-6-(1',3'-dioxocyclohexan-2-yl-carbonyl)-4-(2'-fluoroethoxy) thiochroman-1,1-dioxide.
¹H-NMR (CDCl₃): δ 1.8-3.4 (m,8H), 3.6-4.4 (m,5H), 4.85 (t,1H), 4.98 (bs,1H), 7.36 (d,1H)
IR (KBr): 2975, 1690, 1320, 1170cm⁻¹

### Preparation Example 3

### 5-Chloro-8-methyl-6-(1',3'-dioxocyclohexan-2-yl-carbonyl)-4-methoxythiochroman-1,1-dioxide (Compound No. 3)

### 3-1) Synthesis of 5-chloro-8-methyl-6-carboxyl-4-methoxythiochroman-1,1-dioxide

5-Chloro-8-methyl-6-carboxyl-4-methoxythiochroman-1,1-dioxide was synthesized according to the method described in WO93/18031.
¹H-NMR (acetone-d₆): δ 2.4-4.0 (m,4H), 2.73 (s,3H), 3.52 (s,3H), 4.85 (t,1H), 7.72 (d,1H)

### 3-2) Synthesis of 5-chloro-8-methyl-6-(3'-oxocyclohexenyl)oxycarbonyl-4-methoxythiochroman-1,1-dioxide

0.84 Gram (2.8 mmol) of 5-chloro-8-methyl-6-carboxyl-4-methoxythiochroman-1,1-dioxide was dissolved in 6.7 ml of t-amyl alcohol, 0.34 g (1.1 eq., 3.0 mmol) of 1,3-cyclohexanedione and 0.63 g (1.1. eq., 3.0 mmol) of N,N-dicyclohexylcarbodiimide were added, and the mixture was stirred at room temperature for 3 hours. Then, the solvent was distilled off, ethyl acetate and water were added, and an insoluble substance was removed by filtration. Then, an organic layer was washed with a saturated sodium hydrogencarbonate aqueous solution and with a saturated sodium chloride aqueous solution. Then, the washed product was dried over anhydrous sodium sulfate, and the solvent was distilled off. Then, the residue was subjected to column chromatography (ethyl acetate:n-hexane = 1:1) to give 0.40 g (yield 36 %) of 5-chloro-8-methyl-6-(3'-oxocyclohexenyl)oxycarbonyl-4-methoxythiochroman-1,1-dioxide.
¹H-NMR (CDCl₃): δ 2.0-2.8 (m,9H), 2.78 (s,3H), 3.0-3.4 (m,1H), 3.49 (s,3H), 3.6-4.2 (m,1H), 4.81 (t,1H), 6.07 (bs,1H), 7.69 (s,1H)

### 3-3) Synthesis of 5-chloro-8-methyl-6-(1',3'-dioxocyclohexan-2-yl-carbonyl)-4-methoxythiochroman-1,1-dioxide

0.40 Gram (1.0 mmol) of 5-chloro-8-methyl-6-(3'-oxocyclohexenyl)oxycarbonyl-4-methoxythiochroman-1,1- dioxide was dissolved in 2.4 ml of acetonitrile, 0.14 ml (1.0 eq., 1.0 mmol) of triethylamine and 3 drops of acetonecyanhydrin were added, and the mixture was stirred at room temperature for 8 hours. After the completion of the reaction, the reaction mixture was extracted with a sodium carbonate aqueous solution, and an aqueous layer was washed with ethyl acetate. The aqueous layer was adjusted to a pH of 1 with 5 % hydrochloric acid and then extracted with ethyl acetate. An organic layer was washed with a saturated sodium chloride aqueous solution and dried over anhydrous sodium sulfate, and the solvent was distilled off to give 0.26 g (yield 65 %) of 5-chloro-8-methyl-6-(1',3'-dioxocyclohexan-2-yl-carbonyl)-4-methoxythiochroman-1,1-dioxide.
¹H-NMR (CDCl₃): δ 2.0-2.8 (m,9H), 2.80 (s,3H), 3.0-3.4 (m,1H), 3.46 (s,3H), 3.6-4.1 (m,1H), 4.72 (t,1H), 7.06 (s,1H)
IR (KBr): 2950, 1690, 1300, 1145cm⁻¹

### Preparation Example 4

### 5-Trifluoromethyl-3,3,8-trimethyl-6-(1',3'-dioxocyclohexan-2-yl-carbonyl)-thiochroman-4-one-1,1,-dioxide (Compound No. 4)

### 4-1) Synthesis of 5-trifluoromethyl-3,3,8-trimethyl-6-carboxyl-thiochroman-4-one-1,1-dioxide

5-Trifluoromethyl-3,3,8-trimethyl-6-carboxyl-thiochroman-4-one-1,1-dioxide was synthesized according to the method of synthesizing 3,3,5,8-tetramethyl-6-carboxyl-thiochroman-4-one-1,1-dioxide described in WO96/25413.
¹H-NMR (CDCl₃): δ 1.51 (s,6H), 2.82 (s,2H), 3.61 (s,2H), 7.75 (s,1H)

### 4-2) Synthesis of 5-trifluoromethyl-3,3,8-trimethyl-6-(3'-oxocyclohexenyl)oxycarbonylthiochroman-4-one-1,1-dioxide

0.40 Gram (1.1 mmol) of 5-trifluoromethyl-3,3,8-trimethyl-6-carboxyl-thiochroman-4-one-1,1-dioxide was dissolved in 3 ml of dichloroethane, 0.28 g (2.0 eq., 2.4 mmol) of thionyl chloride was added, and the mixture was refluxed under heat for 1.5 hours. Then, the solvent was distilled off to give an acid chloride. Then, a solution of the obtained acid chloride in tetrahydrofuran was added to a solution of 0.14 g (1.1 eq., 1.3 mmol) of 1,3-cyclohexanedione in tetrahydrofuran, and further, 0.13 g (1.1 eq., 1.3 mmol) of triethylamine was dropwise added. The mixture was stirred at room temperature for 2 hours, and then the solvent was distilled off. The resultant residue was dissolved in ethyl acetate and washed with a 0.2 N hydrochloric acid aqueous solution, with a saturated sodium hydrogencarbonate aqueous solution and with a saturated sodium chloride aqueous solution. Then, the washed product was dried over anhydrous sodium sulfate, and the solvent was distilled off to give 0.29 g (yield 58%) of 5-trifluoromethyl-3,3,8-trimethyl-6-(3'-oxocyclohexenyl)oxycarbonylthiocchroman-4-one-1,1-dioxide.
¹H-NMR (CDCl₃): δ 1.52 (s,6H), 2.0-2.8 (m,6H), 2.85 (s,3H), 3.61 (s,2H), 6.09 (bs,1H), 7.73 (s,1H)

### 4-3) Synthesis of 5-trifluoromethyl-3,3,8-trimethyl-6-(1',3'-dioxocyclohexan-2-yl-carbonyl)thiochroman-4-one-1,1-dioxide

0.29 Gram (0.65 mmol) of 5-trifluoromethyl-3,3,8-trimethyl-6-(3'-oxocyclohexenyl)oxycarbonylthiocchroman-4-one-1,1-dioxide was dissolved in 1.5 ml of acetonitrile, 0.07 g (1.1 eq., 0.69 mmol) of triethylamine and 3 drops of acetonecyanhydrin were added, and the mixture was stirred at room temperature for 1 day. After the completion of the reaction, the reaction mixture was extracted with a saturated sodium hydrogencarbonate aqueous solution, and an aqueous layer was washed with methylene chloride. The aqueous layer was neutralized with 2 % hydrochloric acid and extracted with ethyl acetate. An organic layer was washed with a saturated sodium chloride aqueous solution and then dried over anhydrous sodium sulfate, and the solvent was distilled off to give 0.15 g (yield 30 %) of 5-trifluoromethyl-3,3,8-trimethyl-6-(1',3'-dioxocyclohexan-2-yl-carbonyl) thiochroman-4-one-1,1-dioxide.
¹H-NMR (CDCl₃): δ 1.51 (s,6H), 2.0-2.8 (m,7H), 2.82 (s,3H), 2.70 (s,3H), 3.58 (s,2H), 7.16 (s,1H)
IR (KBr): 3000, 1730, 1690, 1300, 1195, 1150 cm⁻¹

### Preparation Example 5

### 3,3,5,8-Tetramethyl-6-(1',3'-dioxocyclohexan-2-yl-carbonyl)-thiochroman-4-one-1,1-dioxide (Compound No. 5)

### 5-1) Synthesis of 3,3,5,8-tetramethyl-6-carboxylthiochroman-4-one-1,1-dioxide

3,3,5,8-Tetramethyl-6-carboxyl-thiochroman-4-one-1,1-dioxide was synthesized according to the method described in WO96/25413.
¹H-NMR (CDCl₃): δ 1.47 (s,6H), 2.58 (s,3H), 2.76 (s,3H), 3.53 (s,2H), 7.93 (s,1H)

### 5-2) Synthesis of 3,3,5,8-tetramethyl-6-(3'-oxocyclohexenyl)oxycarbonylthiochroman-4-one-1,1-dioxide

0.70 Gram (2.4 mmol) of 3,3,5,8-tetramethyl-6-carboxyl-thiochroman-4-one-1,1-dioxide was dissolved in 4 ml of dichloroethane, 0.56 g (2.0 eq., 4.7 mmol) of thionyl chloride was added, and the mixture was stirred at 55°C for 1.5 hours. Then, the solvent was distilled off to give an acid chloride. Then, a solution of the obtained acid chloride in tetrahydrofuran was added to a solution of 0.29 g (2.6 mmol) of 1,3-cyclohexanedione in tetrahydrofuran, and further, 0.27 g (1.1 eq., 2.7 mmol) of triethylamine was dropwise added. The mixture was stirred at room temperature for 2 hours, and then the solvent was distilled off. The resultant residue was dissolved in ethyl acetate and consecutively washed with a 0.2 N hydrochloric acid aqueous solution, with a saturated sodium hydrogencarbonate aqueous solution and with a saturated sodium chloride aqueous solution. Then, the washed product was dried over anhydrous sodium sulfate, and the solvent was distilled off to give 0.67 g (yield 68 %) of 3,3,5,8-tetramethyl-6-(3'-oxocyclohexenyl)oxycarbonylthiochroman-4-one-1,1-dioxide.
¹H-NMR (CDCl₃): δ 1.46 (s,6H), 2.0-2.8 (m,6H), 2.54 (s,3H), 2.76 (s,3H), 3.52 (s,2H), 6.04 (bs,1H), 7.85 (s,1H)

### 5-3) Synthesis of 3,3,5,8-tetramethyl-6-(1',3'-dioxocyclohexan-2-yl-carbonyl)-thiochroman-4-one-1,1-dioxide

0.63 Gram (1.6 mmol) of 3,3,5,8-tetramethyl-6-(3'-oxocyclohexenyl)oxycarbonylthiochroman-4-one-1,1-dioxide was dissolved in 3 ml of acetonitrile, 0.17 g (1.1 eq., 1.7 mmol) of triethylamine and 3 drops of acetonecyanhydrin were added, and the mixture was stirred at room temperature for 1 day. After the completion of the reaction, the reaction mixture was extracted with a saturated sodium hydrogencarbonate aqueous solution, and an aqueous layer was washed with methylene chloride. The washed aqueous layer was neutralized with 2 % hydrochloric acid, and extracted with ethyl acetate. An organic layer was washed with a saturated sodium chloride aqueous solution and dried over anhydrous sodium sulfate, and the solvent was distilled off to give 0.51 g (yield 81 %) of 3,3,5,8-tetramethyl-6-(1',3'-dioxocyclohexan-2-yl-carbonyl)-thiochroman-4-one-1,1-dioxide.
1H-NMR (CDCl₃): δ 1.45 (s,6H), 2.0-3.0 (m,7H), 2.05 (s,3H), 2.70 (s,3H), 3.51 (s,2H), 7.07 (s,1H)
IR (KBr): 2975, 1700, 1680, 1260, 1195, 1125cm⁻¹

Table 25 shows the structures and NMR spectra of starting materials used in the above Preparation Examples 1 to 5, and Tables 37 and 49 show the structures and physical property data of the obtained compounds.

### Preparation Examples 6 - 55

Compounds shown in Table 26 to 36 were used as starting materials, and Compounds shown in Tables 38 to 48 were synthesized in the same manner as in Preparation Example 5. Tables 50 to 60 show physical property data of the obtained compounds.

### Preparation Example 56

### Synthesis of 5-chloro-8-methyl-6-(1-acetoxy-3-oxocyclohexen-2-yl)-4-(2-porpoxy)thiochroman-1,1-dioxide

0.5 Gram of 5-chloro-8-methyl-6(1,3-dioxocyclohexan-2-ylcarbonyl)-4-(2-propoxy)thiochroman-1,1-dioxide (Compound No. 15 in Table 38) obtained in Preparation Example 6 was dissolved in 5 ml of 1,2-dichloroethane, and 0.10 g of triethylamine was added. To the resultant solution was added 0.13 g of acetyl chloride, and the mixture was stirred at room temperature for 8 hours. The reaction mixture was diluted with ethyl acetate, washed with a 5 % hydrochloric acid aqueous solution twice, washed with a saturated sodium bicarbonate solution twice, washed with a saturated sodium chloride aqueous solution and dried over anhydrous sodium sulfate. A drying agent was filtered off, and the remainder was concentrated. Then, the resultant oil was purified by column chromatography to give 0.30 g of Compound No. 250 shown in Table 38 (yield 54 %). Table 60 shows the physical property data of the obtained compound.

### Preparation Example 57

### Synthesis of 5-chloro-8-methyl-6-(3-oxocyclohexen-2-yl)-4-(2-propoxy)thiochroman-1,1-dioxide triethylamine salt

0.5 Gram of 5-chloro-8-methyl-6(1,3-dioxocyclohexan-2-yl)-4-(2-propoxy)thiochroman-1,1-dioxide (Compound No. 15 in Table 38) obtained in Preparation Example 6 was dissolved in 5 ml of 1,2-dichloroethane, and 0.12 g of triethylamine was added. The mixture was stirred at room temperature for 1 hour. A formed crystal was recovered by filtration to give, as an end product, 0.3 g of Compound No. 251 shown in Table 48 (yield 65 %). Table 60 shows the physical property date of the obtained compound.

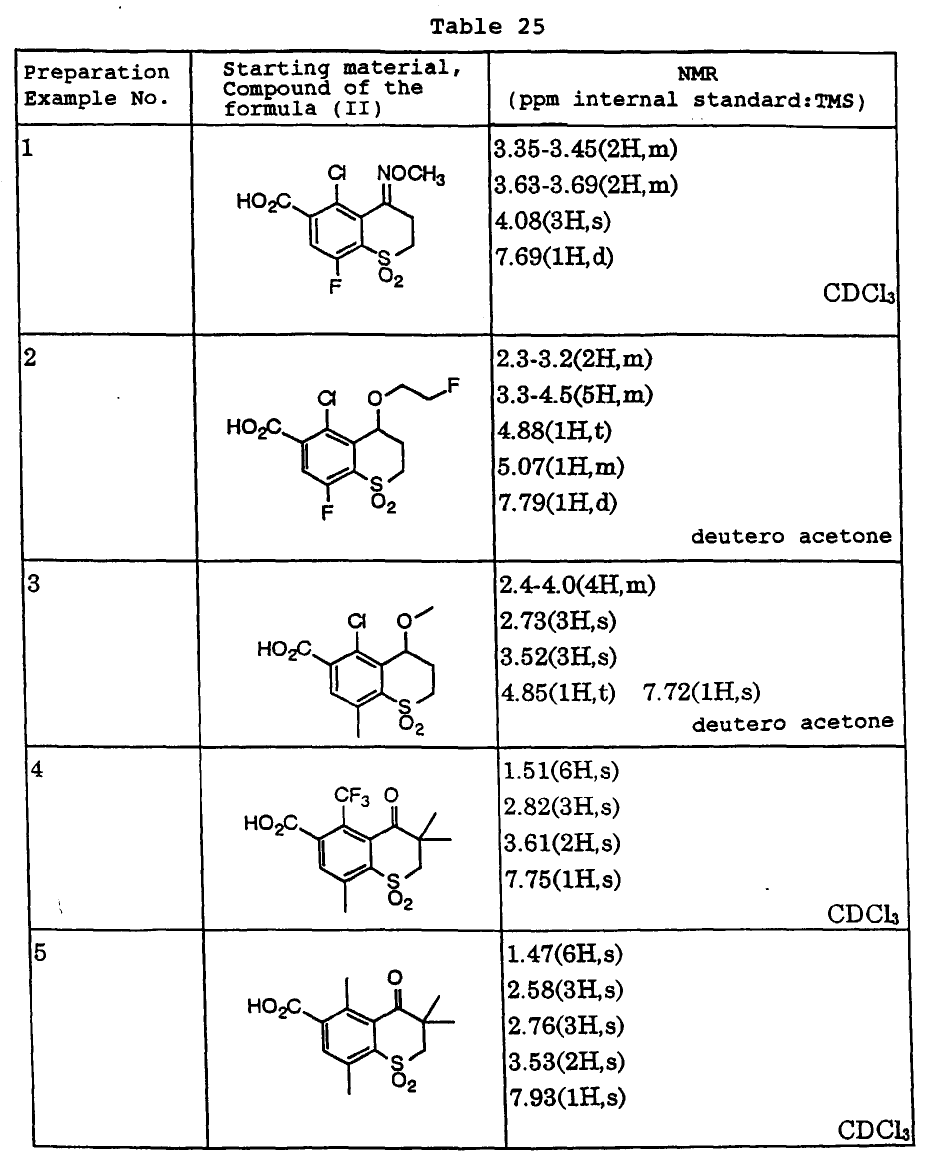

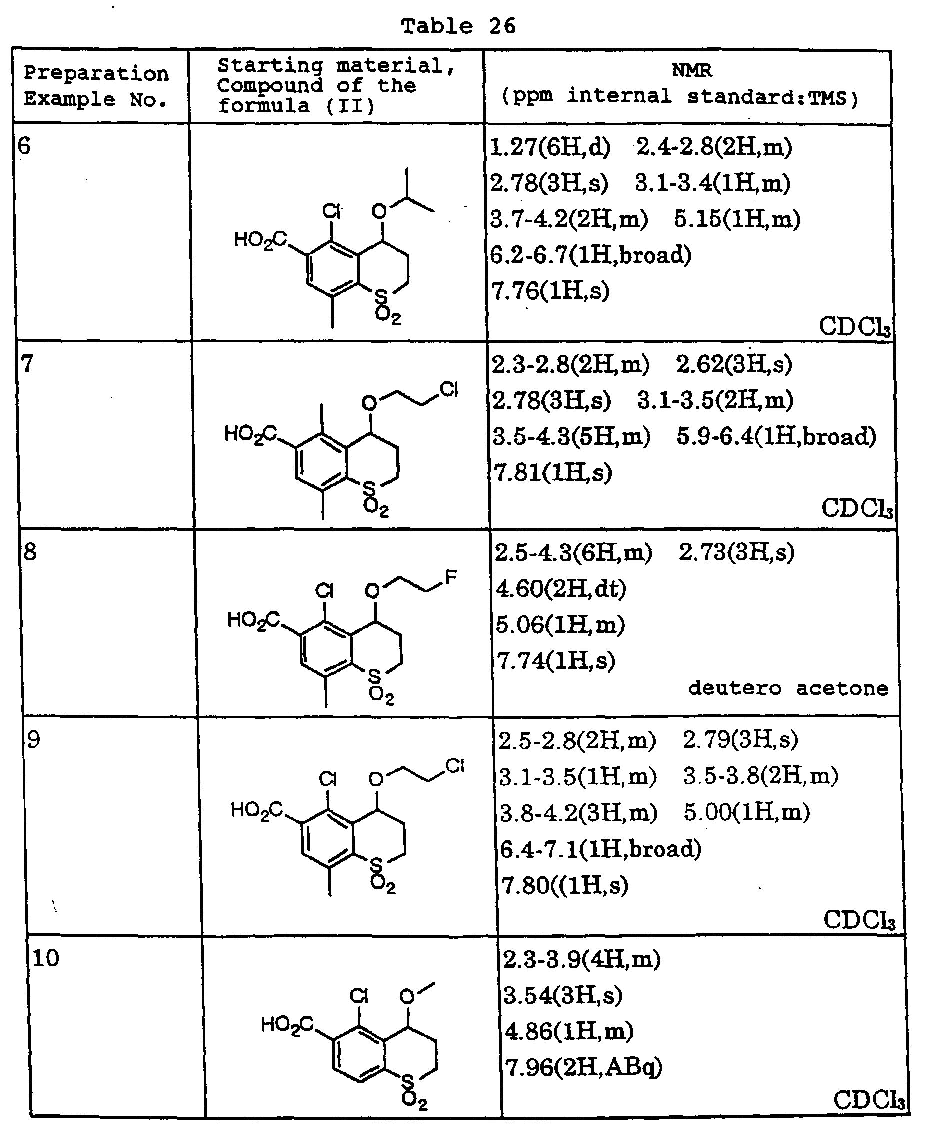

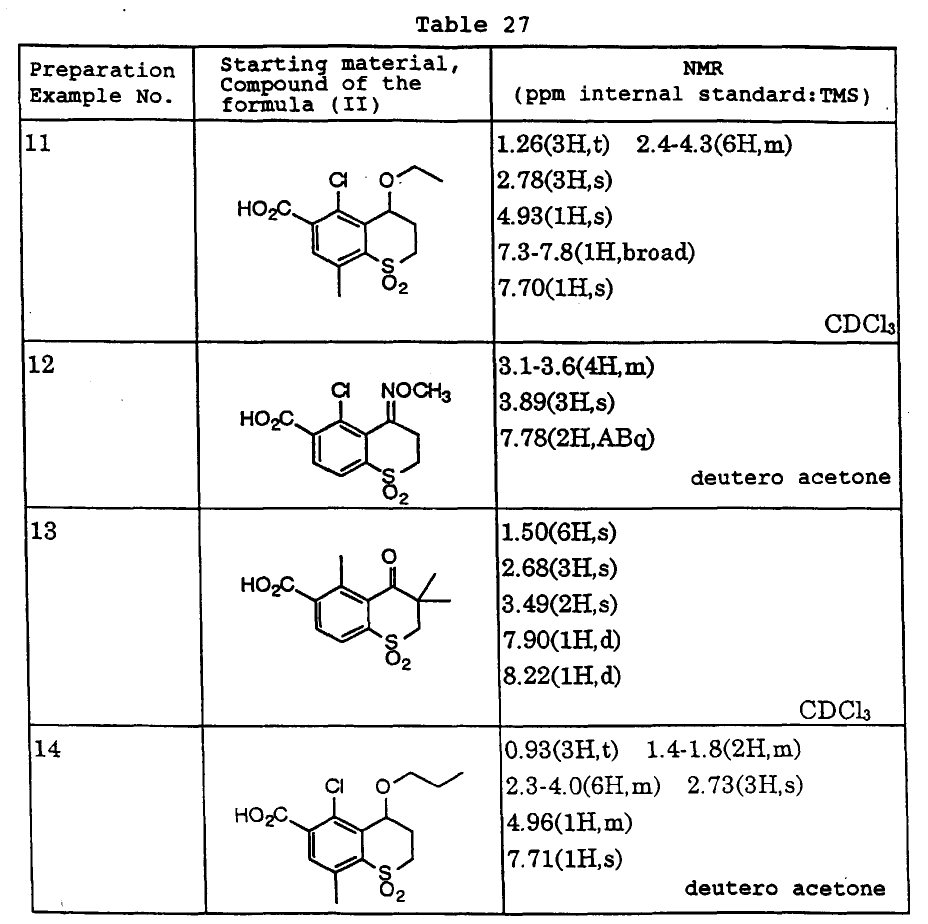

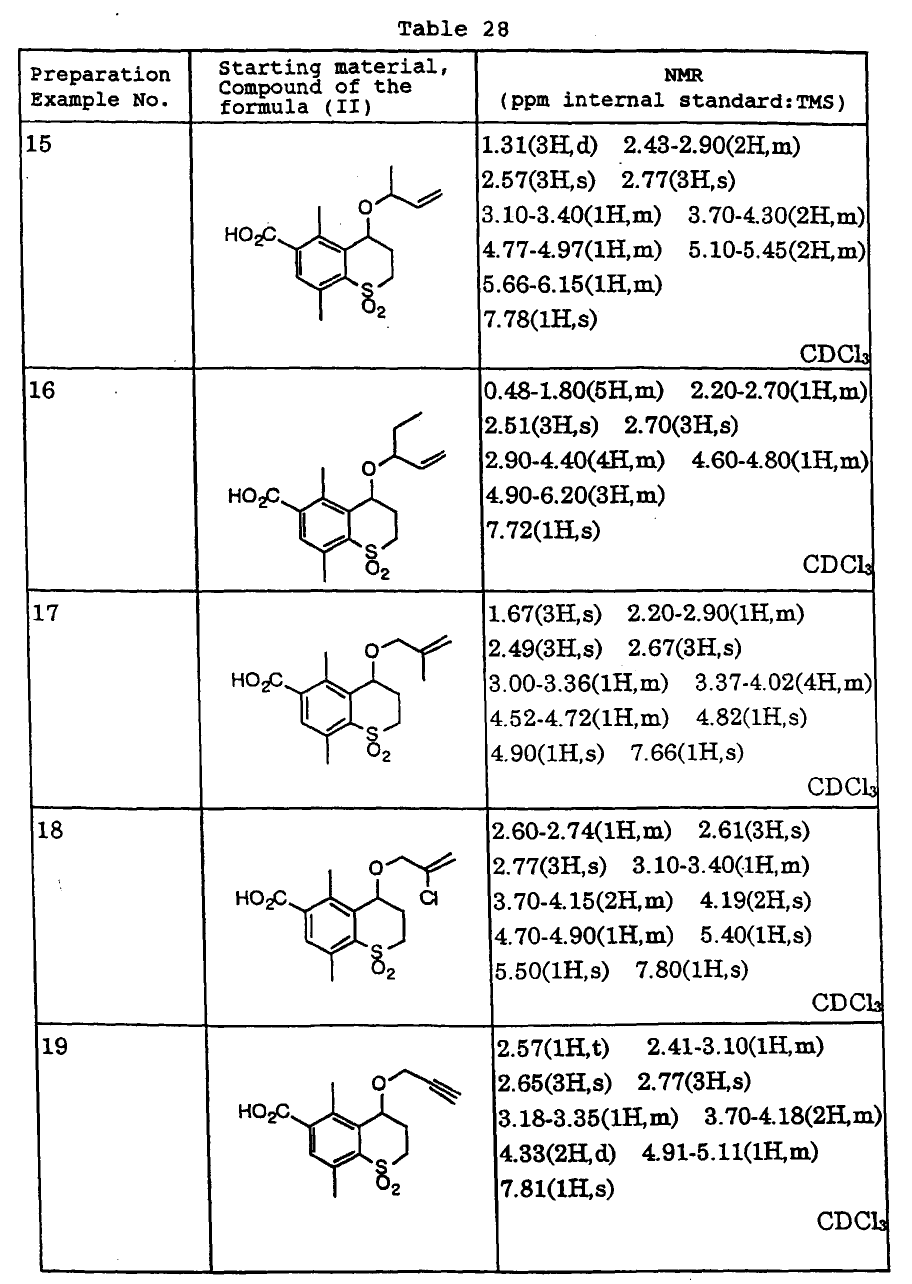

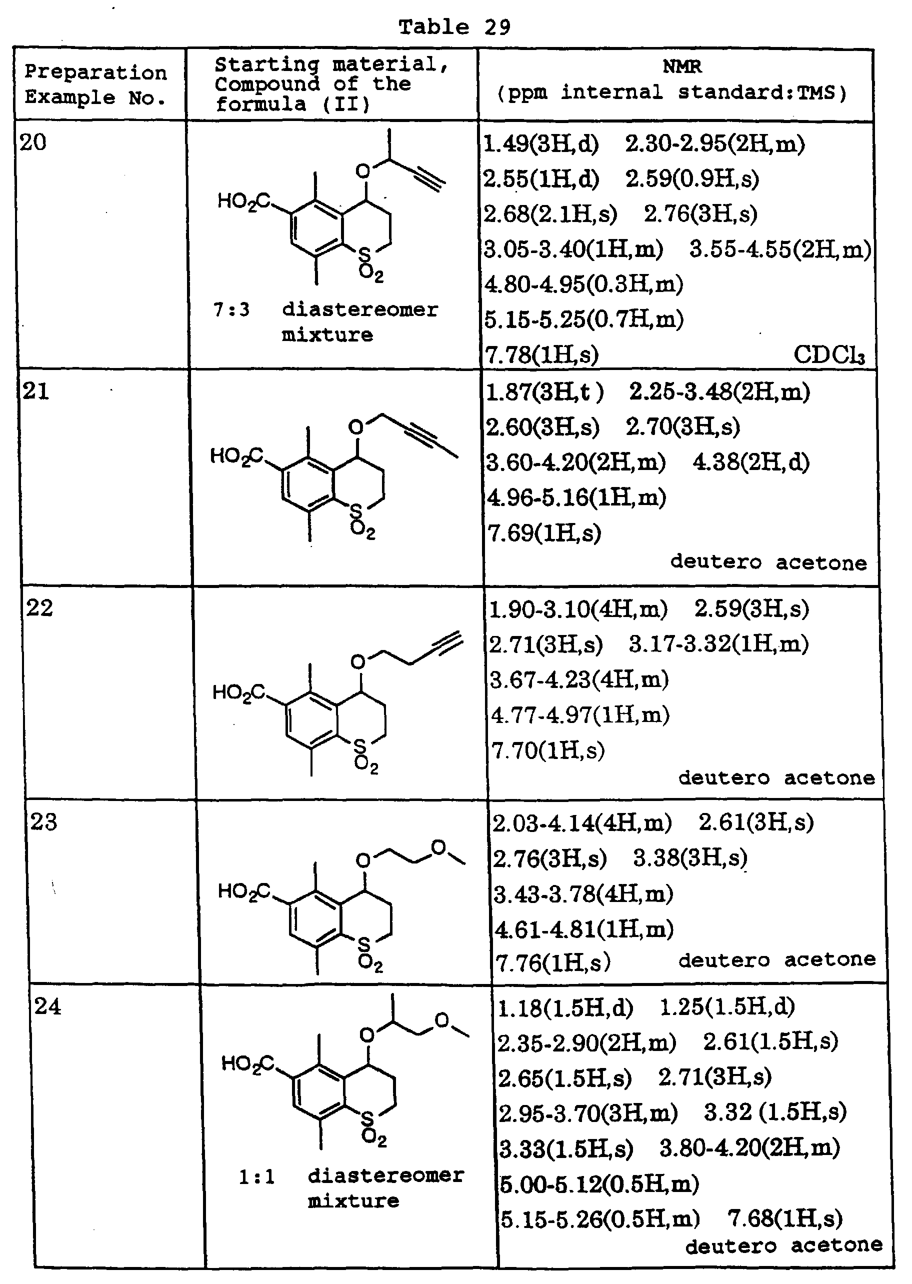

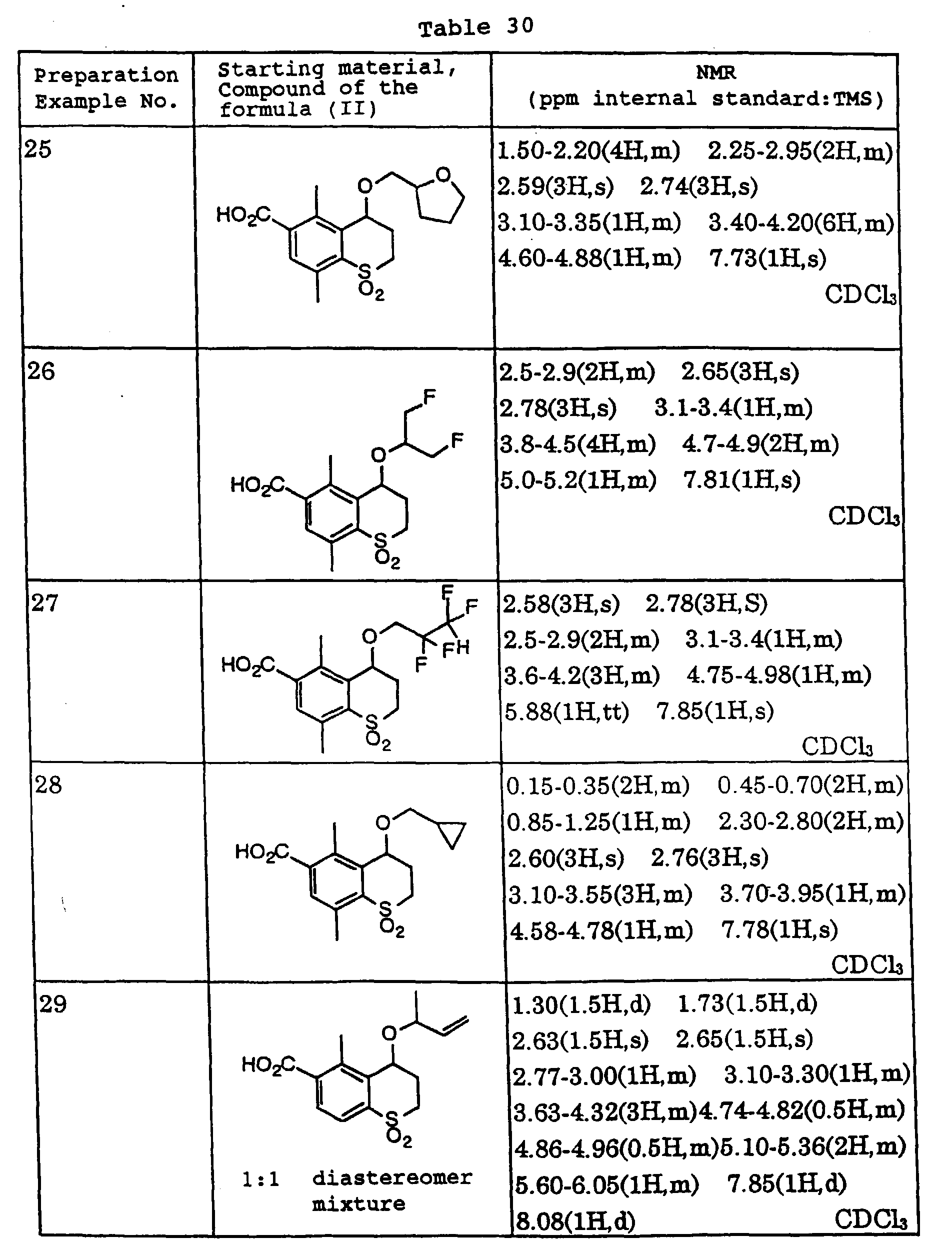

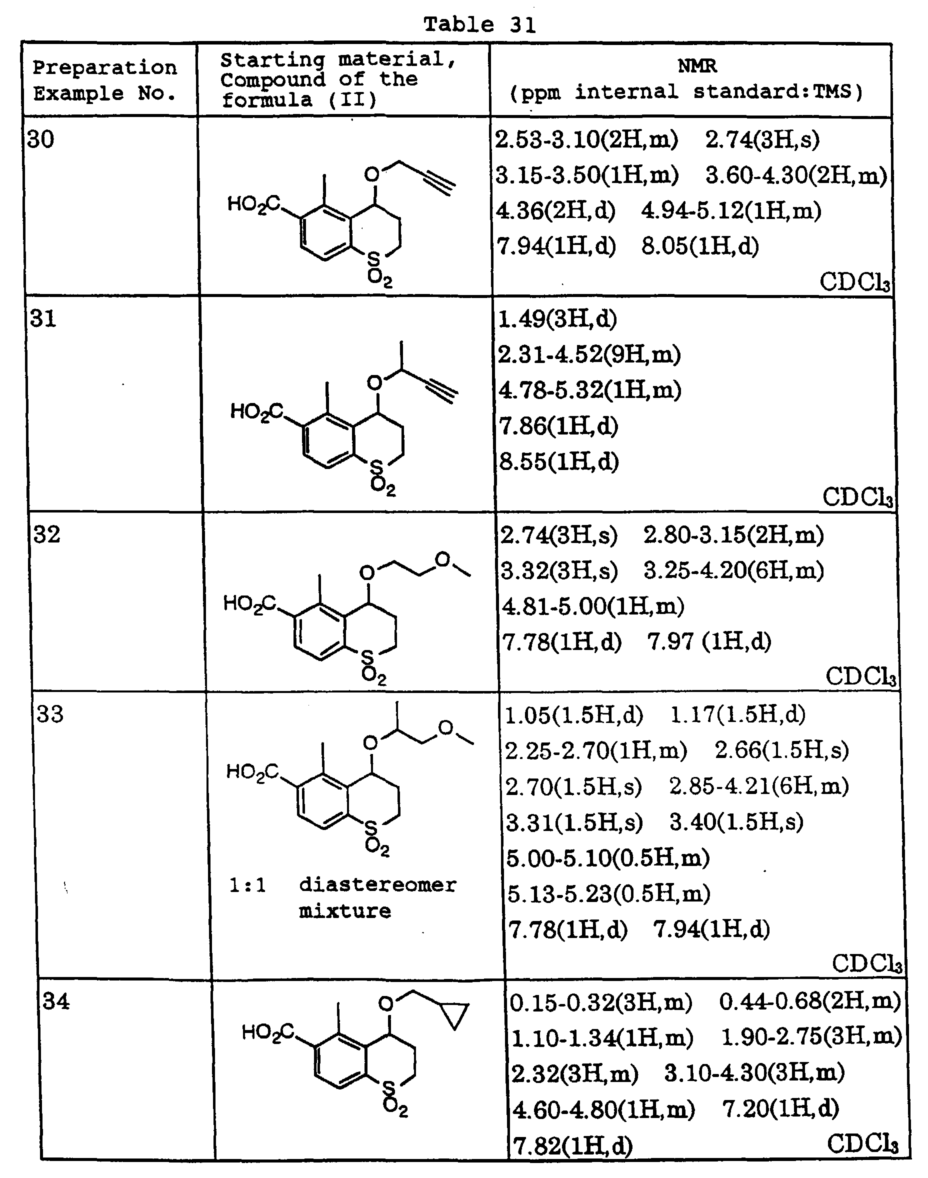

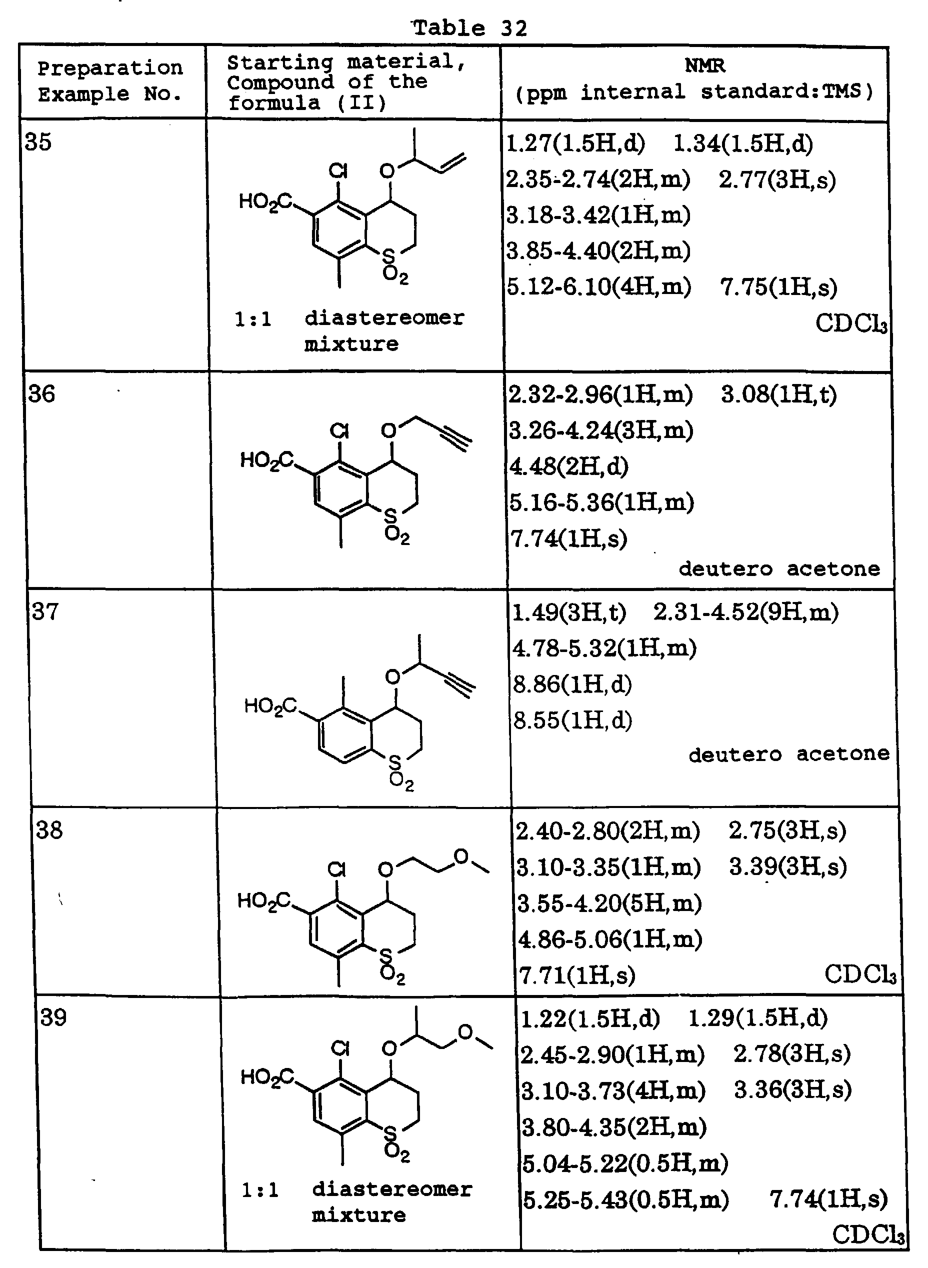

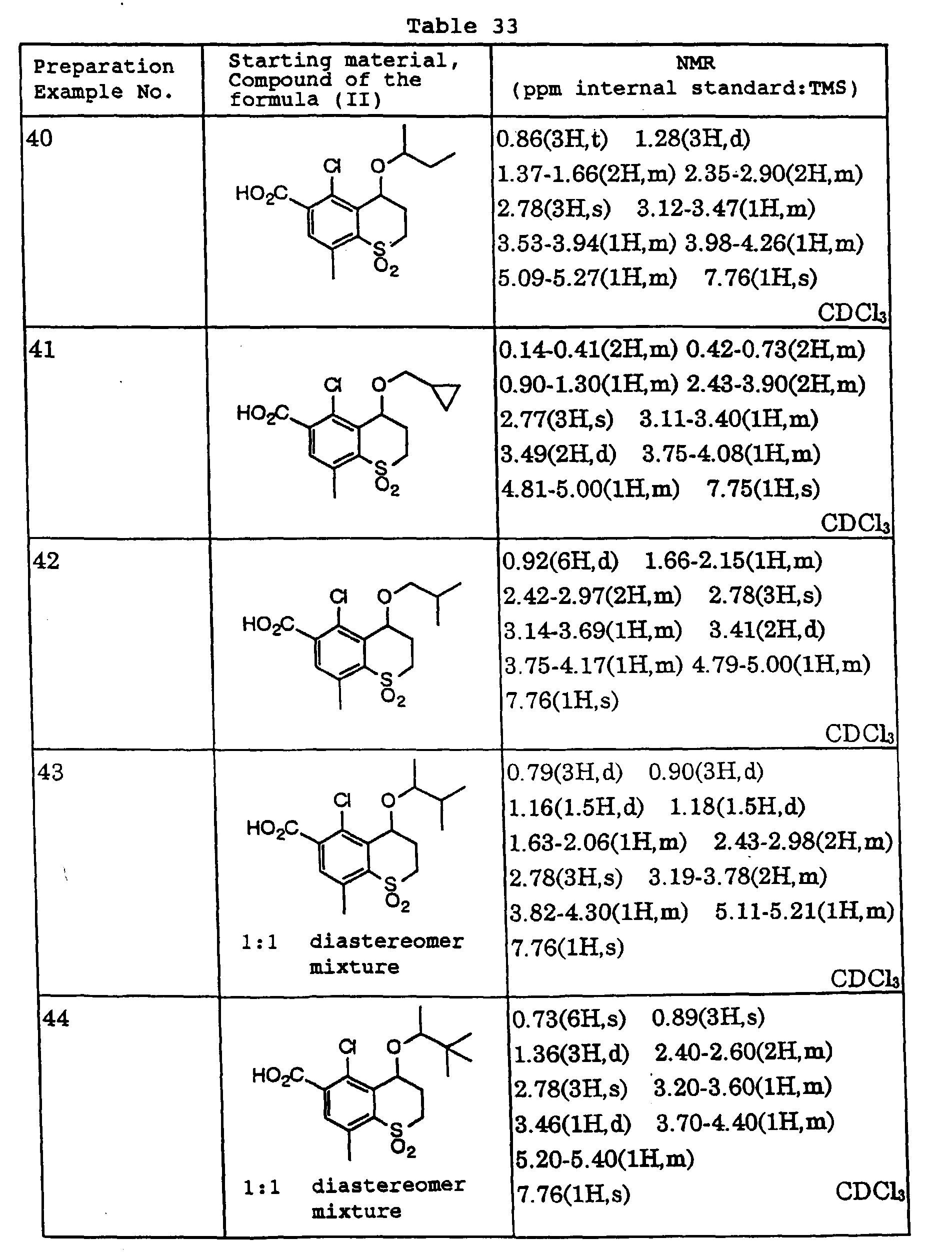

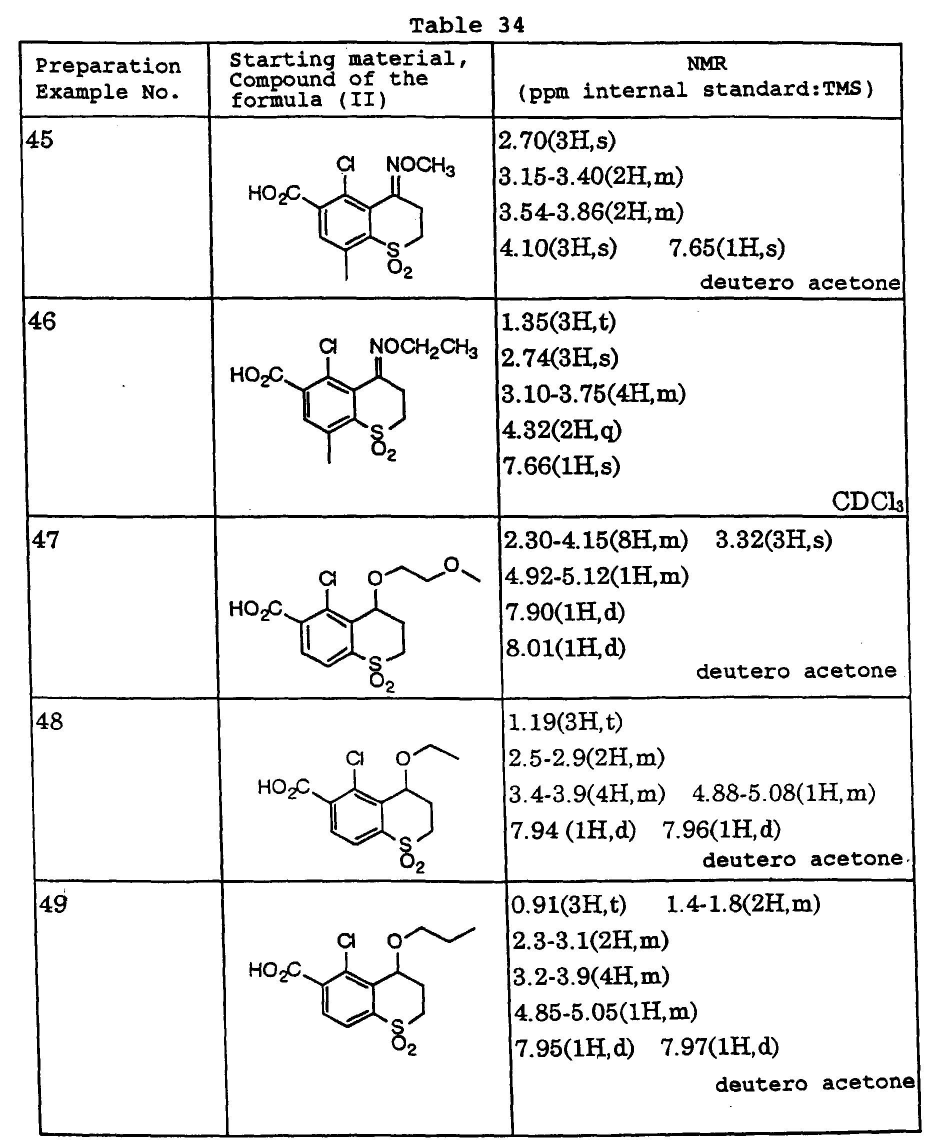

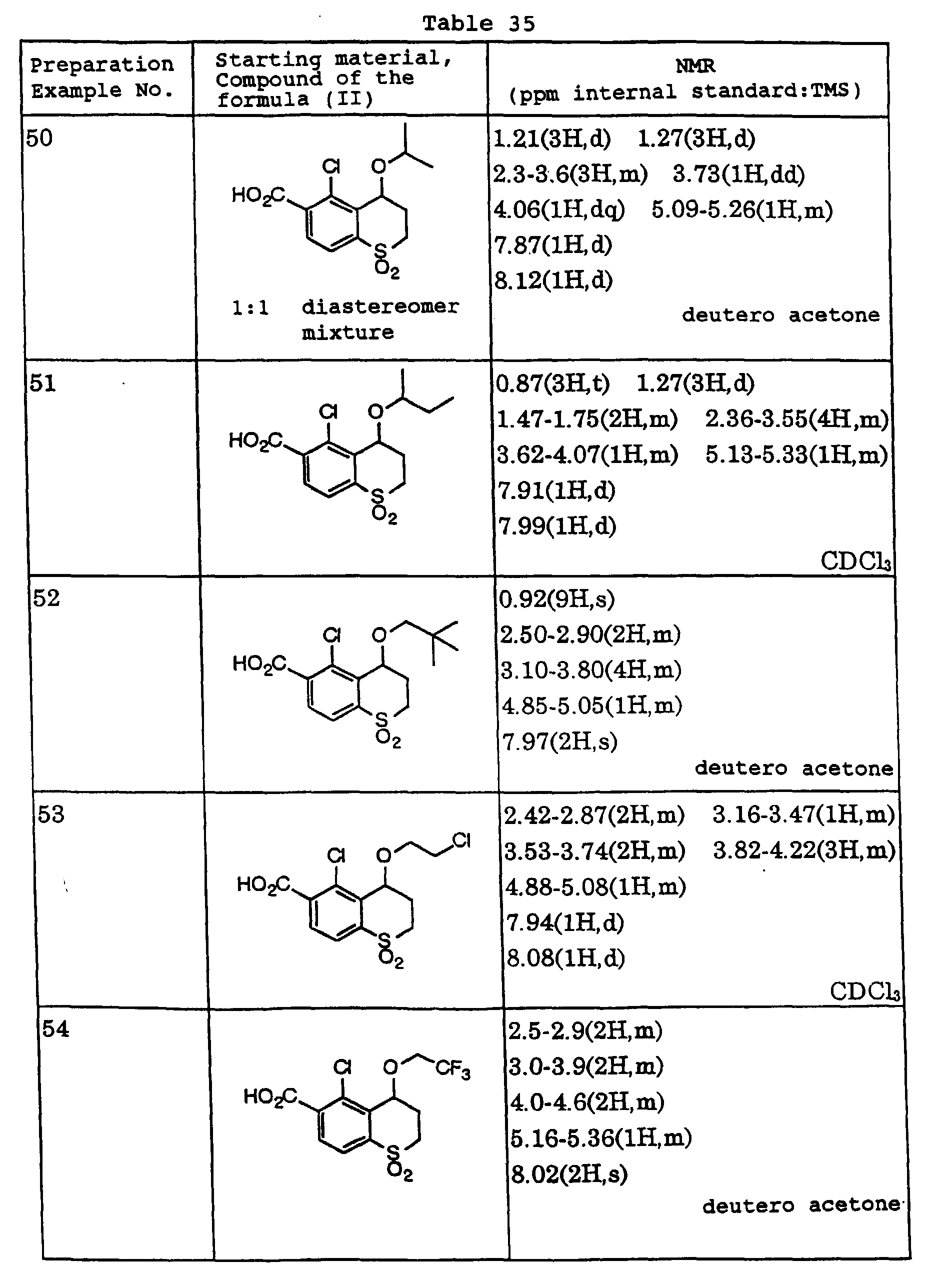

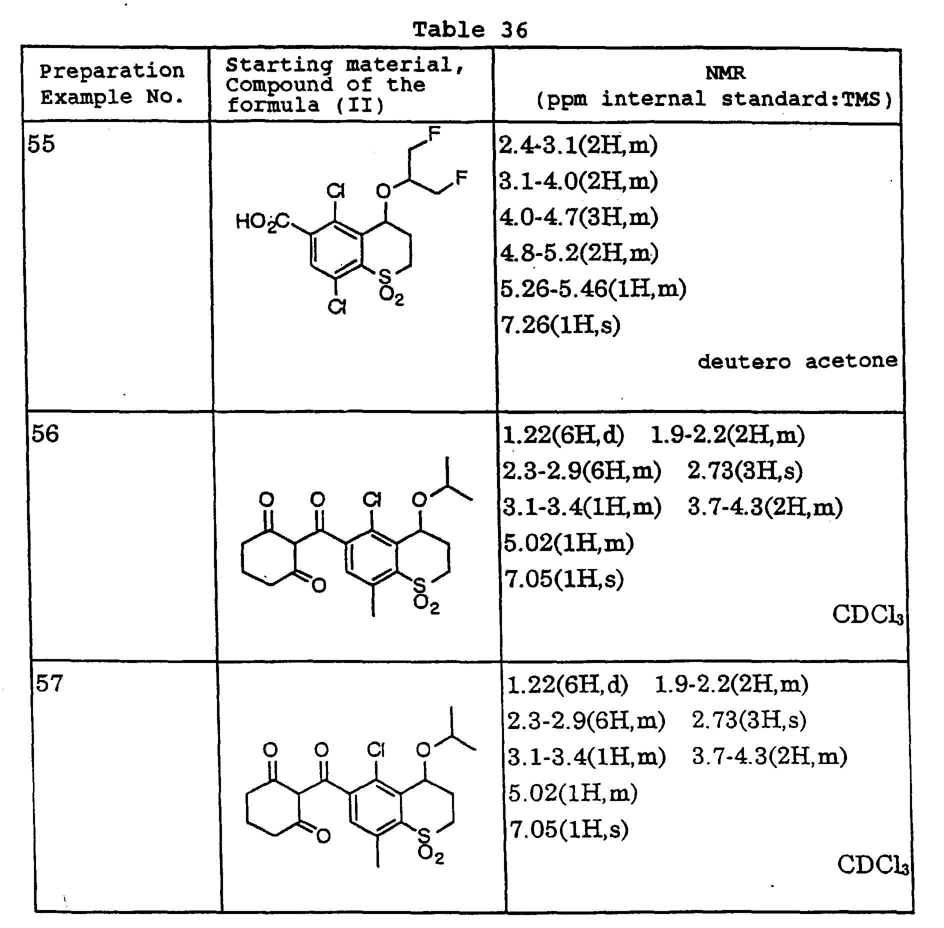

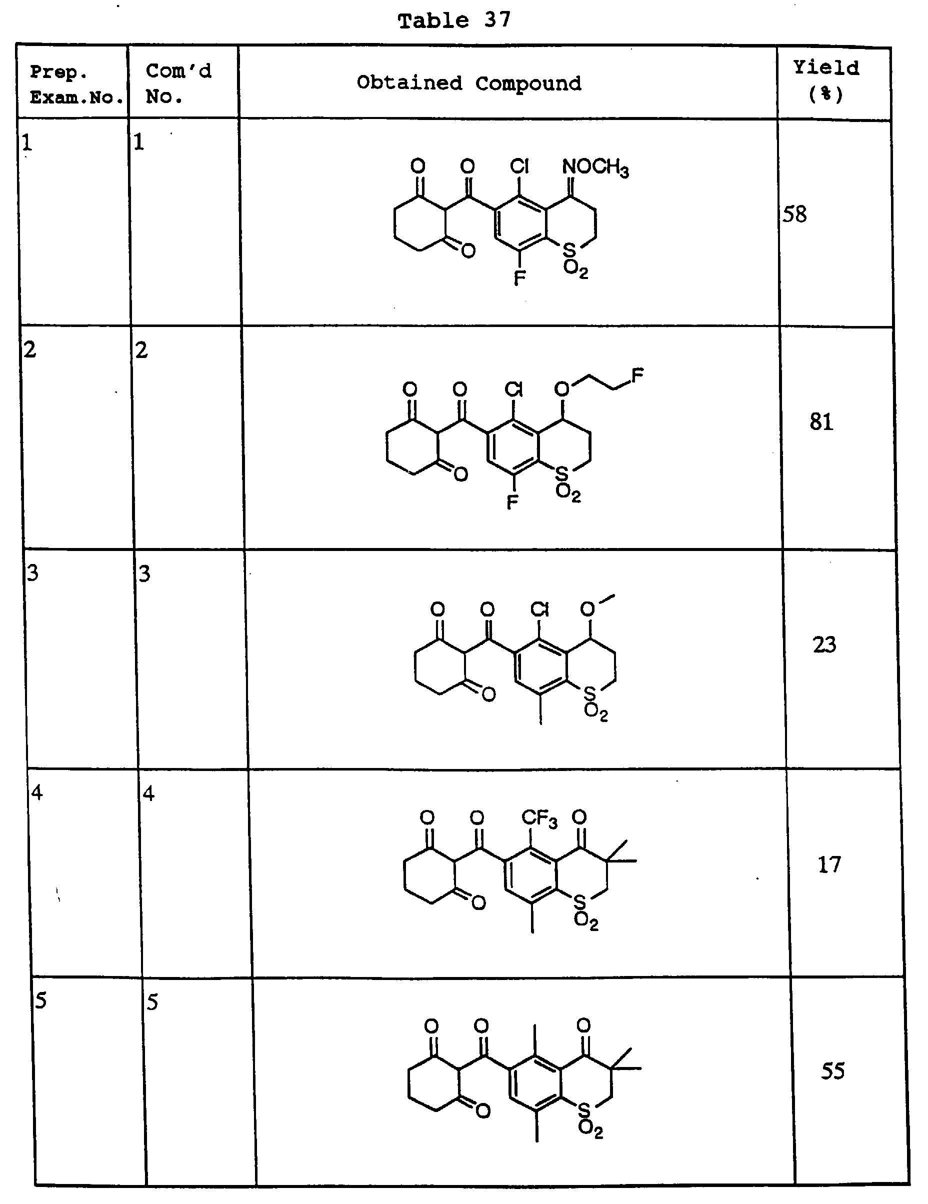

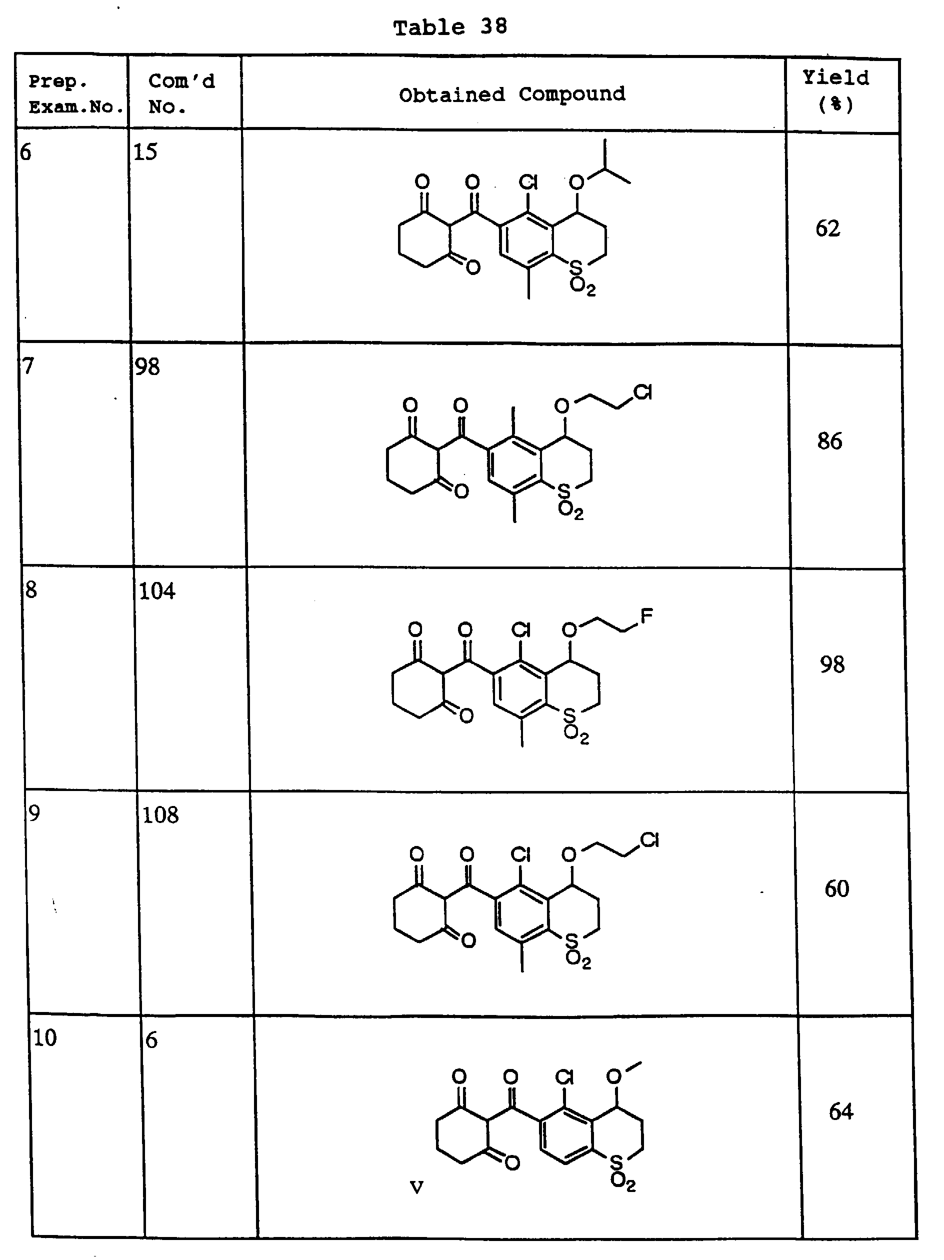

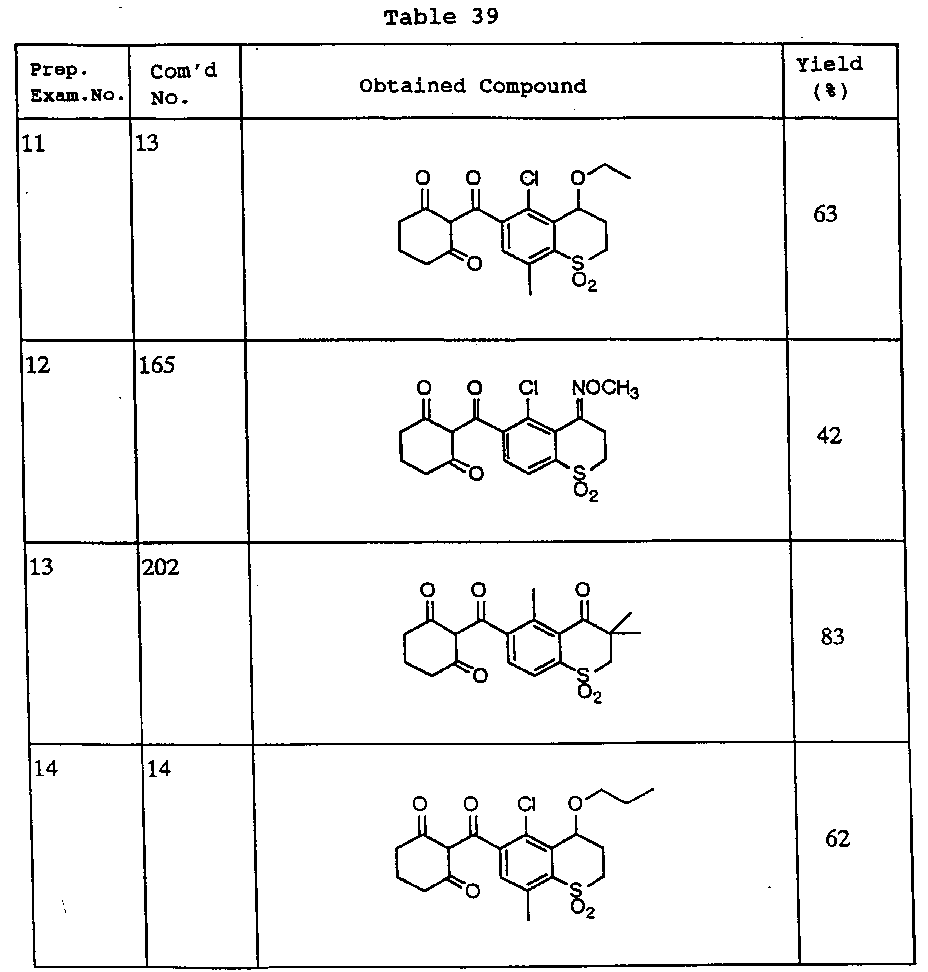

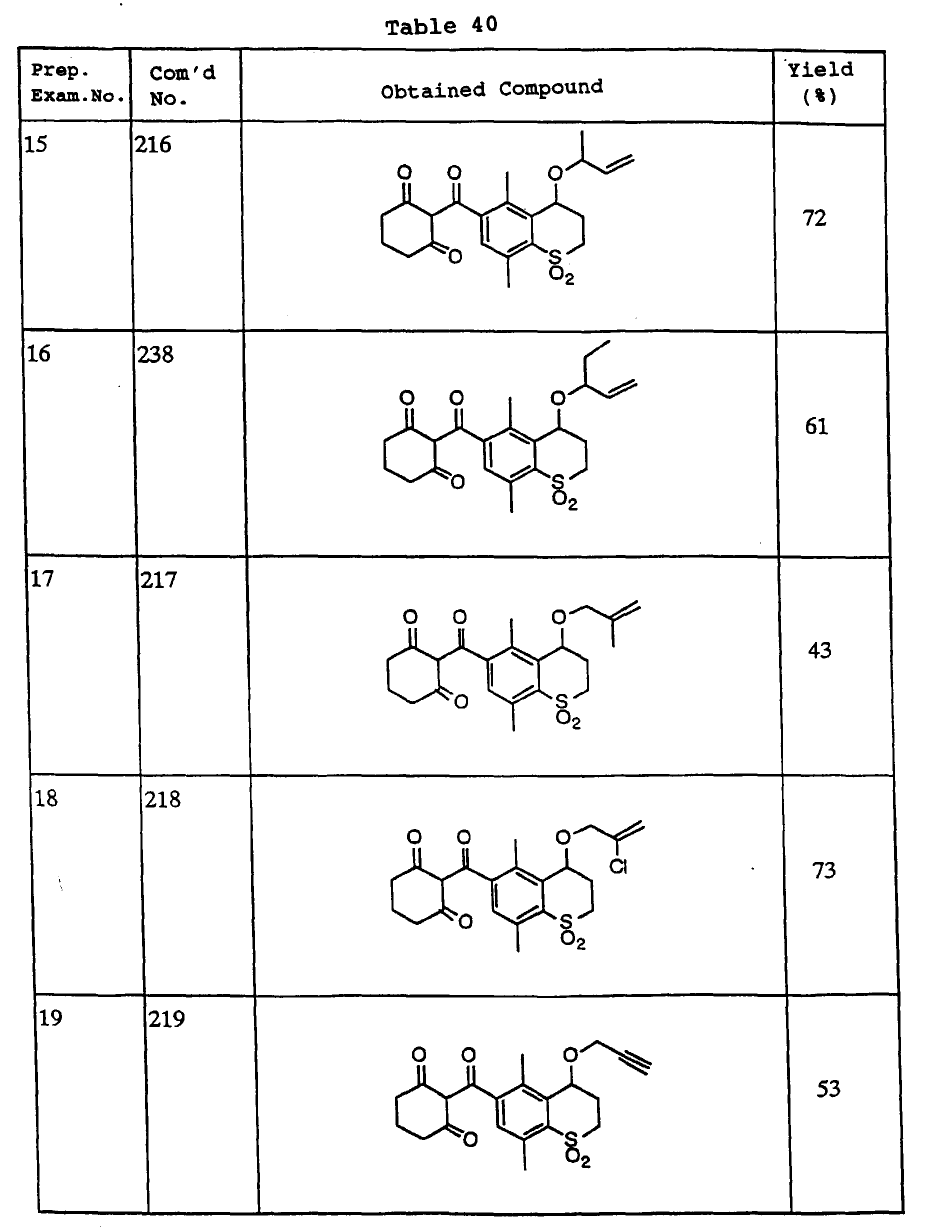

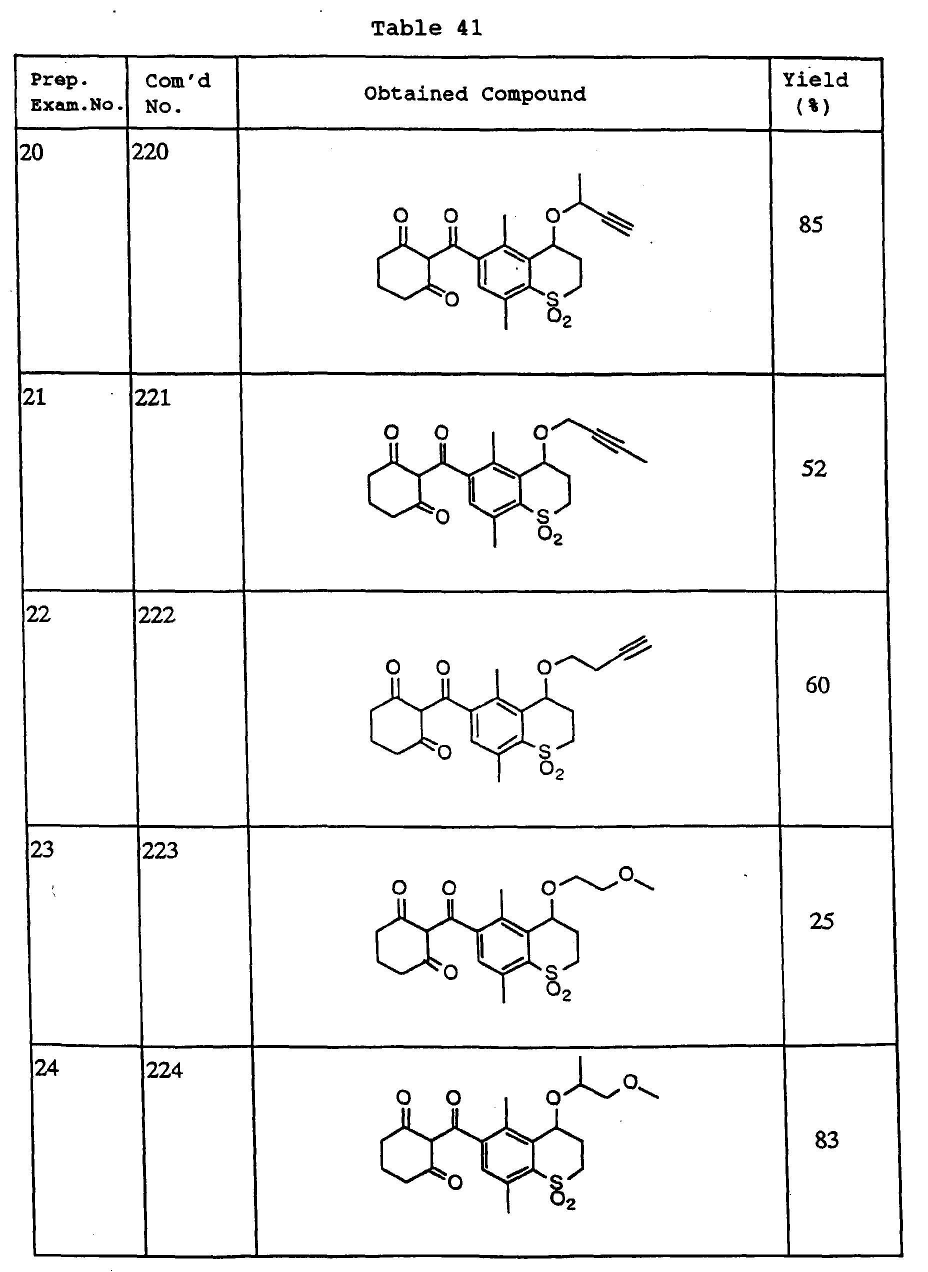

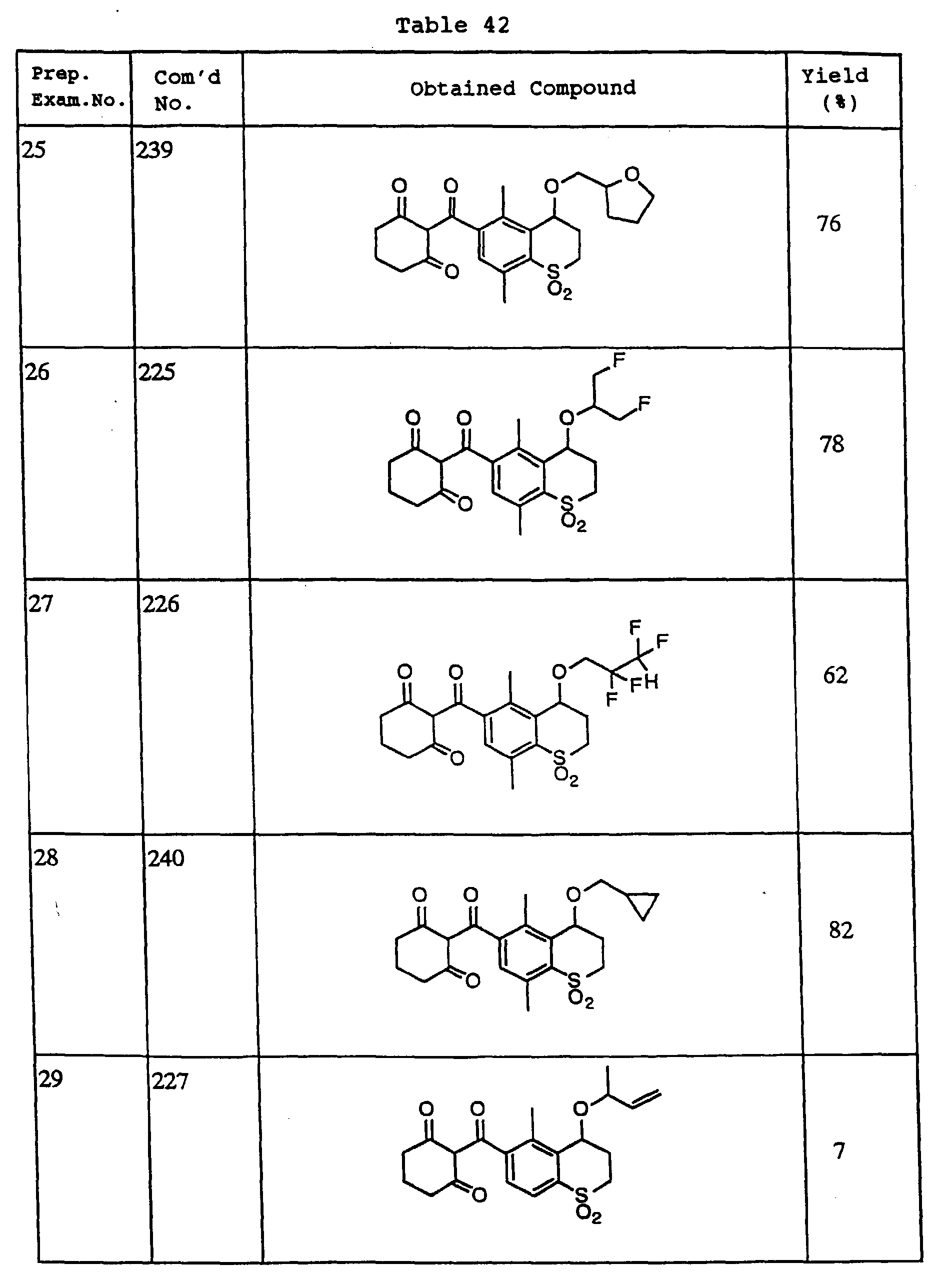

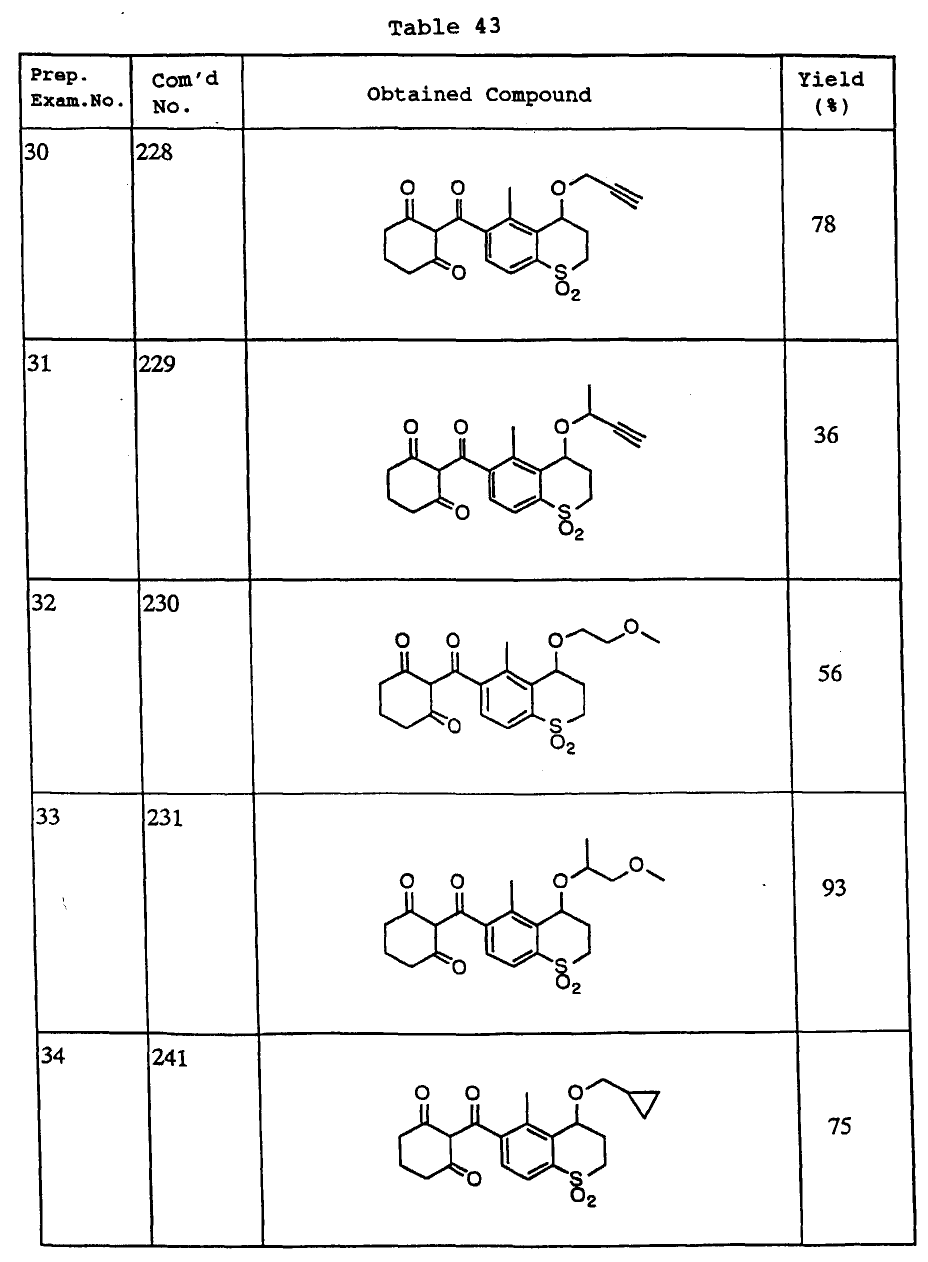

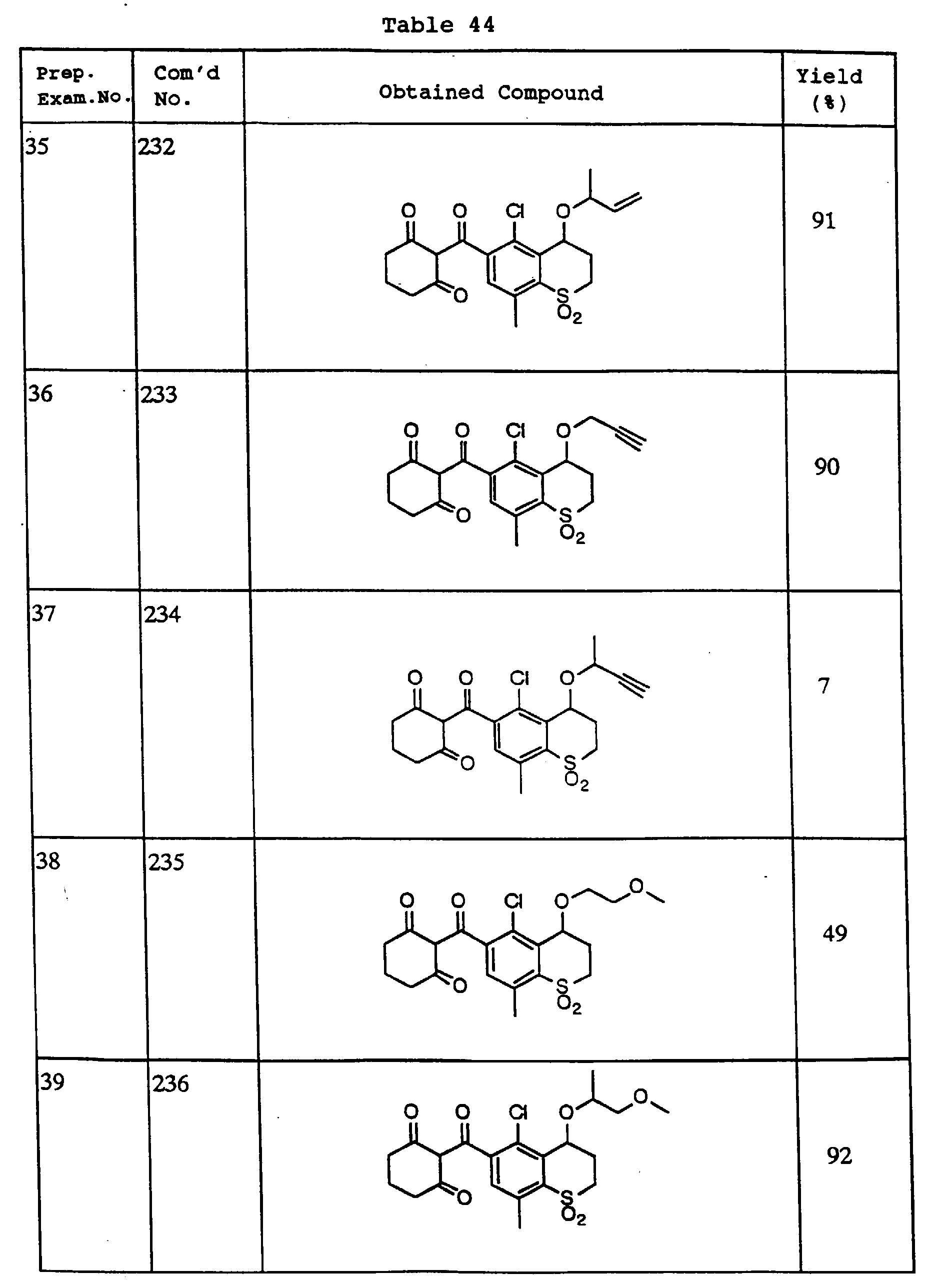

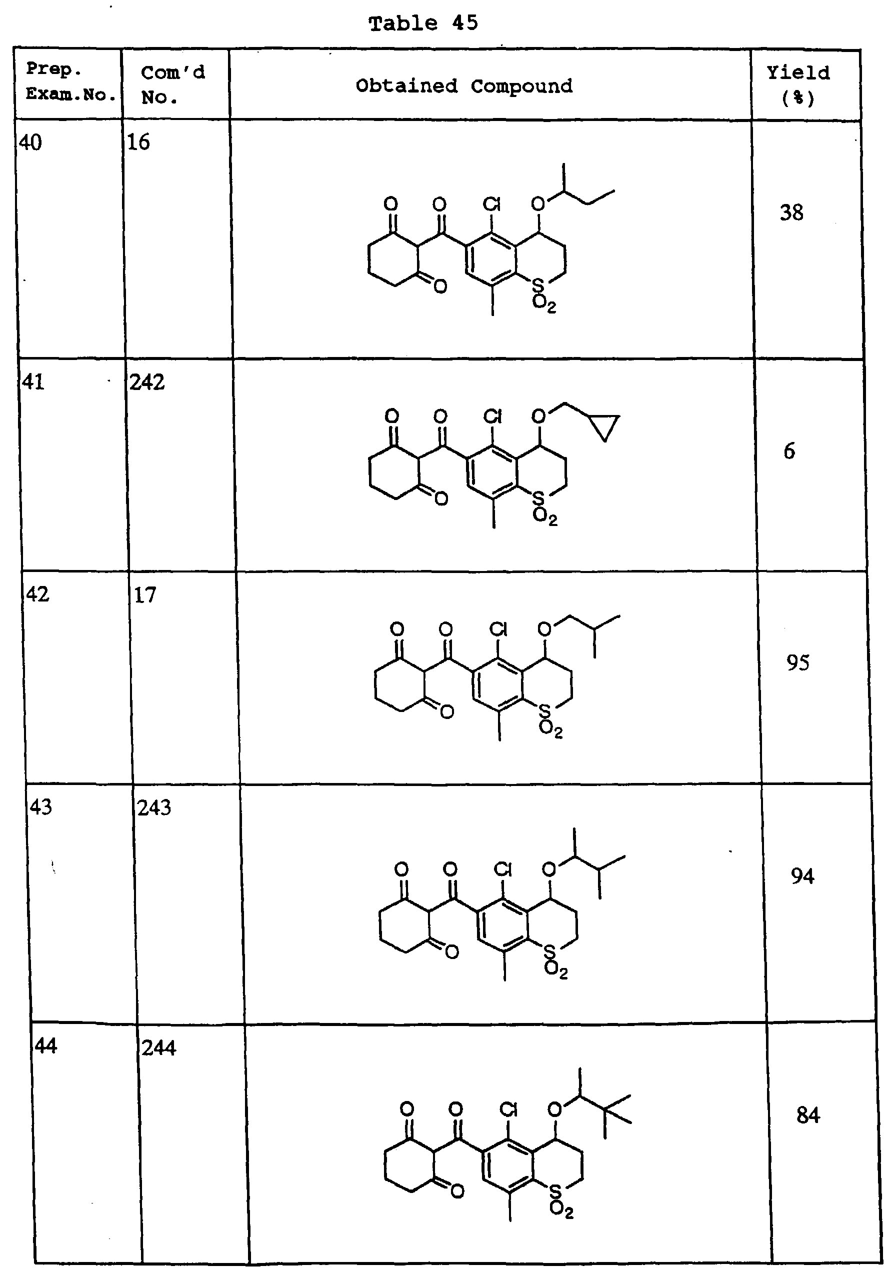

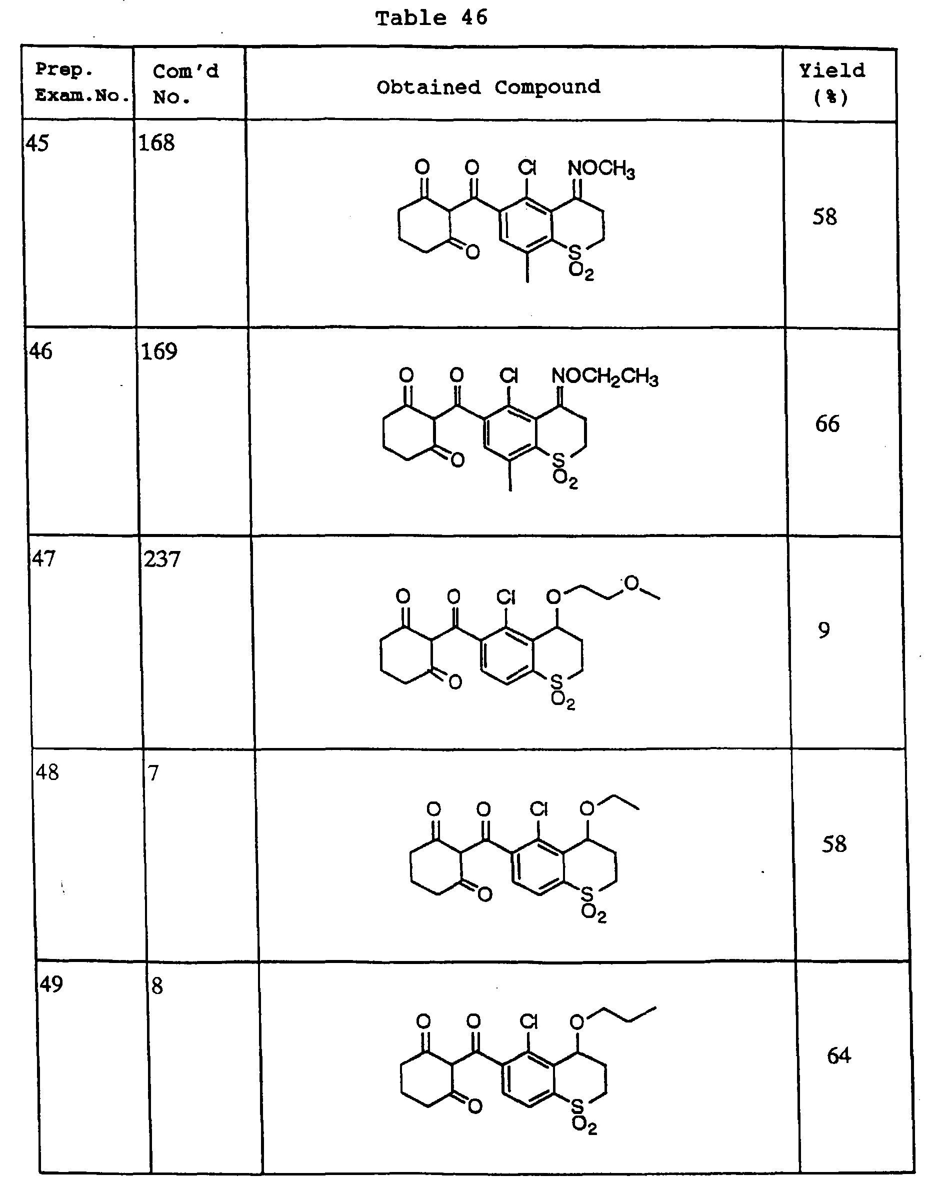

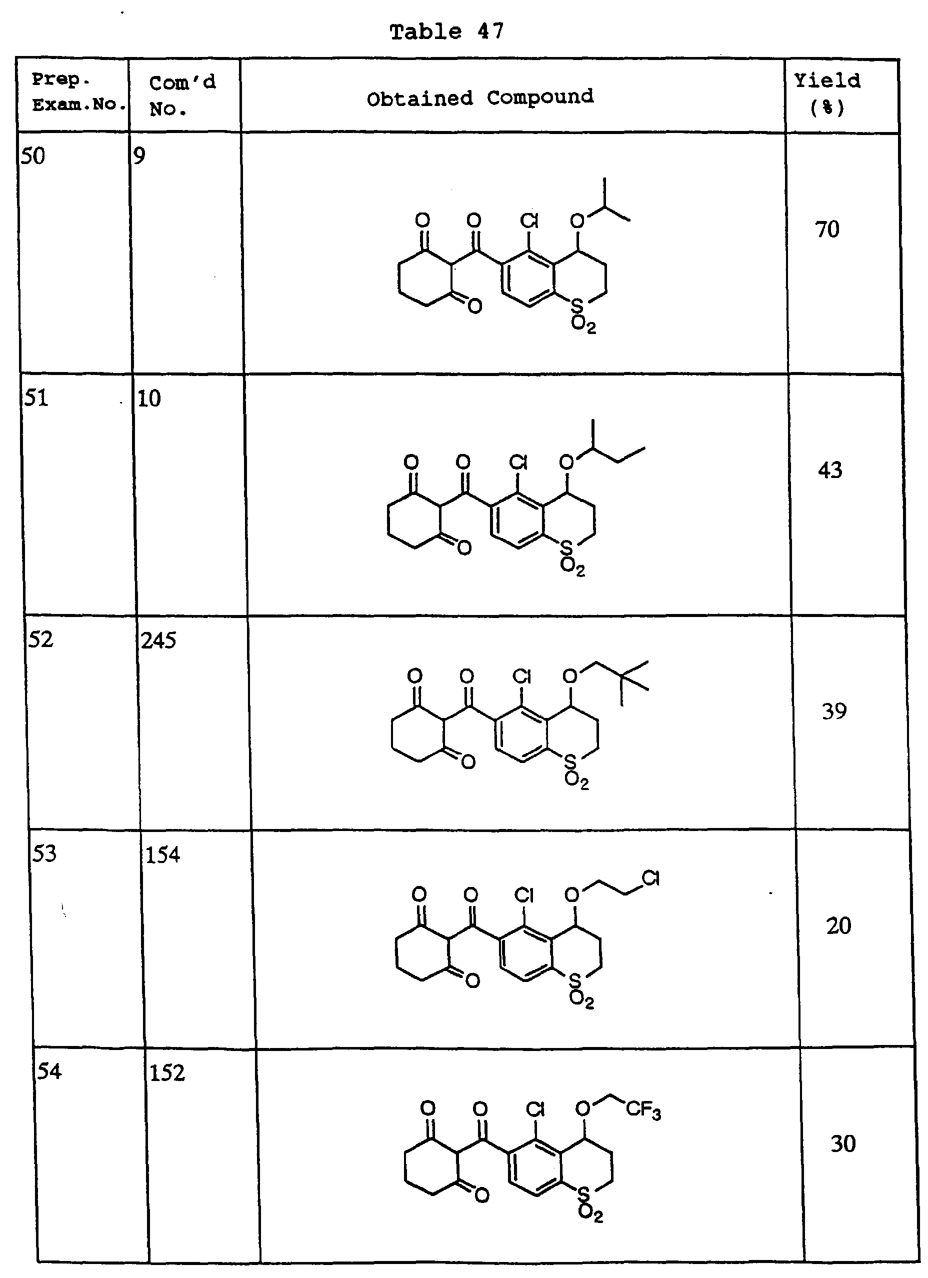

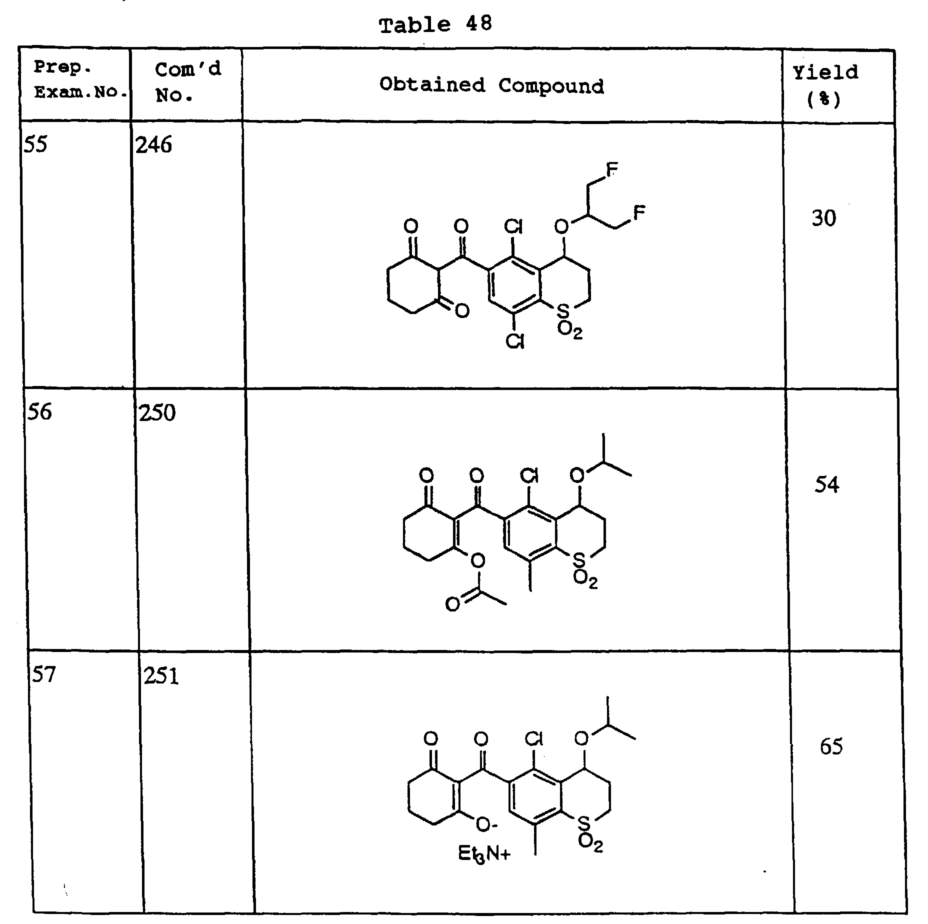

**Table 49**

| Prep. | Com'd No. | N.M.R. (ppm internal standard:TMS) | | IR (KBr) (cm⁻¹) |
|---|---|---|---|---|
| 1 | 1 | 2.0-2.7(6H,m) 3.4-3.5(4H,m) | | 2950,1710,1680 |
| | | 4.11(3H,s) | | 1250,1150 |
| | | 6.06(1H,broad) | | |
| | | 7.54(1H,d) | | |
| | | | CDCl₃ | |
| 2 | 2 | 1.8-3.4(8H,m) 3.6-4.4(5H,m) | | 2975,1690,1320 |
| | | 4.85(1H,t) | | 1170 |
| | | 4.98(1H,broad) | | |
| | | 7.36(1H,d) | | |
| | | | CDCl₃ | |
| 3 | 3 | 2.0-2.8(9H,m) 2.80(3H,s) | | 2950,1690,1300 |
| | | 3.0-3.4(1H,m) 3.46(3H,s) | | 1145 |
| | | 3.6-4.1(1H,m) 4.72(1H,t) | | |
| | | 7.06(1H,s) | | |
| | | | CDCl₃ | |
| 4 | 4 | 1.51(6H,s) 2.0-2.8(7H,m) | | 3000,1730,1690 |
| | | 2.82(3H,s) 2.70(3H,s) | | 1300,1195,1150 |
| | | 3.58(2H,s) | | |
| | | 7.16(1H,s) | | |
| | | | CDCl₃ | |
| 5 | 5 | 1.45(6H,s) 2.0-3.0(7H,m) | | 2975,1700,1680 |
| | | 2.05(3H,s) 2.70(3H,s) | | 1260,1195,1125 |
| | | 3.51(2H,s) | | |
| | | 7.07(1H,s) | | |
| | | | CDCl₃ | |

**Table 50**

| Prep. Exam.No. | Com'd No. | N.M.R. (ppm internal standard:TMS) | | IR (KBr) (cm⁻¹) |
|---|---|---|---|---|
| 6 | 15 | 1.22(6H,d) 1.9-2.2(2H,m) | | 1740,1690,1310, |
| | | 2.3-2.9(6H,m) 2.73(3H,s) | | 1130 |
| | | 3.1-3.4(1H,m) 3.7-4.3(2H,m) | | |
| | | 5.02(1H,m) 7.05(1H,s) | | |
| | | | CDCl₃ | |
| 7 | 98 | 1.9-2.2(2H,m) 2.23(3H,s) | | 1750,1690,1300 |
| | | 2.3-4.1(12H,m) 2.71(3H,s) | | 1140 |
| | | 4.67(1H,m) | | |
| | | 6.96(1H,s) | | |
| | | | CDCl₃ | |
| 8 | 104 | 1.9-2.2(2H,m) 2.3-4.2(10H,m) | | 1680,1330,1290 |
| | | 4.55(2H,m) | | 1140 |
| | | 4.91(1H,m) | | |
| | | 7.07(1H,s) | | |
| | | | CDCl₃ | |
| 9 | 108 | 1.9-2.2(2H,m) 2.3-4.3(12H,m) | | 1680,1290,1120 |
| | | 2.74(3H,s) | | |
| | | 4.90(1H,s) | | |
| | | 7.07(1H,s) | | |
| | | | CDCl₃ | |
| 10 | 6 | 1.9-2.2(2H,m) 2.3-3.9(8H,m) | | 1680,1300,1140 |
| | | 3.49(3H,s) 4.79(1H,m) | | |
| | | 7.48(1H,d) | | |
| | | 7.87(1H,d) | | |
| | | | deutero acetone | |

**Table 51**

| Prep. Exam.No. | Com'd No. | N.M.R. (ppm internal standard:TMS) | | IR (KBr) (cm⁻¹) |
|---|---|---|---|---|
| 11 | 13 | 1.22(3H,t) 1.9-2.3(2H,m) | | 1680,1310,1130 |
| | | 2.3-4.2(10H,m) 2.73(3H,s) | | |
| | | 4.82(1H,m) | | |
| | | 7.05(1H,s) | | |
| | | | CDCl₃ | |
| 12 | 165 | 1.9-2.2(2H,m) 2.3-2.6(2H,m) | | 1740,1690,1310 |
| | | 2.7-3.1(2H,m) 3.3-3.7(4H,m) | | 1160 |
| | | 4.05(3H,s) 7.46(1H,d) | | |
| | | 7.93(1H,d) | | |
| | | | deutero acetone | |
| 13 | 202 | 1.48(6H,s) 1.9-2.2(2H,m) | | 1750,1690,1310, |
| | | 2.35(3H,s) 2.3-2.5(2H,m) | | 1140 |
| | | 2.7-2.9(2H,m) 3.48(2H,s) | | |
| | | 7.32(1H,d) 7.85(1H,d) | | |
| | | | CDCl₃ | |
| 14 | 14 | 0.91(3H,t) 1.4-1.8(2H,m) | | 1690,1300,1140 |
| | | 1.9-4.2(12H,m) 2.72(3H,s) | | |
| | | 4.80(1H,s) | | |
| | | 7.05(1H,s) | | |
| | | | CDCl₃ | |

**Table 52**

| Prep. Exam.No. | Com'd No. | N.M.R. (ppm internal standard:TMS) | | IR (KBr) (cm⁻¹) |
|---|---|---|---|---|
| 15 | 216 | 1.27(3H,d) 1.90-2.90(8H,m) 2.20(3H,s) | | 3450,2980,1680 |
| | | 2.75(3H,s) 3.10-3.35(1H,m) | | 1550,1440,1300 |
| | | 3.70-4.20(2H,m) 4.68-4.88(1H,m) | | 1280,1120,1060 |
| | | 5.10-5.36(2H,m) 5.60-6.10(1H,m) | | 920,750,590 |
| | | 6.93(1H,s) | CDCl₃ | |
| 16 | 238 | 0.89(3H,t) 1.40-1.80(2H,m) | | 3550,3100,2950 |
| | | 1.90-3.08(8H,m) 2.21(3H,s) | | 1680,1600,1400 |
| | | 2.65(3H,s) 3.10-3.40(1H,m) | | 1300,1130 |
| | | 3.58-4.18(2H,m) 4.80-5.00(1H,m) | | |
| | | 5.10-6.10(3H,m) 7.05(1H,s) | CDCl₃ | |
| 17 | 217 | 1.75(3H,s) 2.20(3H,s) 2.61(3H,s) | | 3500,3190,2950 |
| | | 2.80-3.48(8H,m) 3.51-4.33(4H,m) | | 1580,1400,1280, |
| | | 4.70-4.95(2H,m) 4.89-5.15(1H,m) | | 1120 |
| | | 6.94(1H,s) | | |
| | | | deutero acetone | |
| 18 | 218 | 2.11-2.87(7H,m) 2.22(3H,s) 2.77(3H,s) | | 2950,1680,1550 |
| | | 3.10-3.40(1H,s) 3.70-4.10(2H,m) | | 1410,1310,1280 |
| | | 4.14(2H,s) 4.62-4.82(1H,m) | | 1190,1120,920 |
| | | 5.37(1H,s) 5.51(1H,s) 6.97(1H,s) | | 750,590,550 |
| | | | CDCl₃ | |
| 19 | 219 | 1.90-2.90(8H,m) 2.27(3H,s) 2.71(3H,s) | | 3450,3290,2950 |
| | | 3.05-3.45(1H,m) 3.65-4.34(2H,m) | | 1680,1550,1440 |
| | | 4.29(2H,d) 4.86-5.05(1H,m) | | 1310,1120,1060 |
| | | 6.96(1H,s) | | 920,790,750,590 |
| | | | CDCl₃ | |

**Table 53**

| Prep. Exam.No. | Com'd No. | N.M.R. (ppm internal standard:TMS) | | IR (KBr) (cm⁻¹) |
|---|---|---|---|---|
| 20 | 220 | 1.48(3H,d) 1.90-2.95(6H,m) 2.04(2H,t) | | 3300,3275,3000 |
| | | 2.22(0.9H,s) 2.31(2.1H,s) 2.53(1H,d) | | 2945,2900,1680 |
| | | 2.71(3H,s) 3.10-3.35(1H,m) | | 1590,1440,1410 |
| | | 3.55-4.50(2H,m) 4.75-4.95(0.3H,m) | | 1300,1280,1190 |
| | | 5.05-5.20(0.7H,m) 6.95(1H,s) | | 1120,1090,1050 |
| | | 7:3 diastereomer mixture | CDCl₃ | |
| 21 | 221 | 1.87(3H,t) 1.90-2.90(7H,m) 2.26(3H,s) | | 3460,2960,1740 |
| | | 2.70(3H,s) 3.05-3.35(1H,m) | | 1690,1595,1450 |
| | | 3.65-4.35(2H,m) 4.35(2H,d) | | 1390,1310,1290 |
| | | 4.82-5.00(1H,m) 6.93(1H,s) | | 1250,1195,1130 |
| | | | CDCl₃ | 1060,920,760 |
| 22 | 222 | 1.90-2.95(10H,m) 2.23(3H,s) 2.71(3H,s) | | 3390,2950,1730 |
| | | 3.05-3.35(1H,m) 3.40-4.25(4H,m) | | 1680,1565,1440 |
| | | 4.54-4.74(1H,m) | | 1310,1290,1120 |
| | | 6.95(1H,s) | | 1080,920,750 |
| | | | CDCl₃ | |
| 23 | 223 | 1.53(8H,m) 2.24(3H,s) | | 2940,1670,1540 |
| | | 2.71(3H,s) 3.10-3.30(1H,m) | | 1440,1410,1300 |
| | | 3.36(3H,s) 3.51-3.93(5H,m) | | 1280,1180,1120 |
| | | 4.54-4.74(1H,m) 6.94(1H,s) | | 1070,910,740 |
| | | | CDCl₃ | |
| 24 | 224 | 1.18(1.5H,d) 1.21(1.5H,d) | | 2950,1680,1570 |
| | | 1.70-2.20(3H,m) 2.25-2.55(2H,m) | | 1560,1300,1280 |
| | | 2.26(1.5H,s) 2.31(1.5H,s) 2.60-2.90(3H,m) | | 1120,1045,920 |
| | | 2.72(3H,s) 3.00-3.60(3H,m) 3.31(1.5H,s) | | 750 |
| | | 3.34(1.5H,s) 3.70-4.30(2H,m) | | |
| | | 4.70-4.90(0.5H,m) 5.00-5.15(0.5H,m) | | |
| | | 6.93(1H,s) | | |
| | | 1:1 diastereomer mixture | CDCl₃ | |

**Table 54**

| Prep. Exam.No. | Com'd No. | N.M.R. (ppm internal standard:TMS) | | IR (KBr) (cm⁻¹) |
|---|---|---|---|---|
| 25 | 239 | 1.50-2.90(12H,m) 2.23(3H,s) | | 2950,2880,1785 |
| | | 2.71(3H,s) 3.05-3.30(1H,m) | | 1560,1310,1290 |
| | | 3.40-4.20(6H,m) 4.50-4.70(1H,m) | | 1190,1125,1075 |
| | | 6.94(1H,s) | | 920,760,600,550 |
| | | | CDCl₃ | |
| 26 | 225 | 1.9-2.9(8H,m) 2.28(3H,s) | | 2980,1740,4680 |
| | | 3.1-3.4(1H,m) 3.8-4.4(4H,m) | | 1560,1420,1310 |
| | | 3.5-4.2(3H,m) 4.7-4.9(1H,m) | | 1290,1190,1130 |
| | | 5.93(1H,t) 6.99(1H,s) | | 1050,920,790 |
| | | | CDCl₃ | 760 |
| 27 | 226 | 1.9-2.9(8H,m) 2.19(3H,s) 2.72(3H,s) | | 2950,1730,1680 |
| | | 3.1-3.4(1H,m) 3.5-4.2(3H,m) | | 1560,1410,1310 |
| | | 4.7-4.9(1H,m) 5.93(1H,t) | | 1290,1240,1190 |
| | | 6.99(1H,s) | | 1120,1100,920 |
| | | | CDCl₃ | 840,790,760 |
| 28 | 240 | 0.10-0.30(2H,m) 0.45-0.65(2H,m) | | 2980,1690,1560 |
| | | 0.90-1.25(1H,m) 1.90-2.90(8H,m) | | 1420,1320,1300 |
| | | 2.23(3H,s) 2.70(3H,s) 3.05-3.60(3H,m) | | 1200,1140,1080 |
| | | 3.65-4.05(1H,m) 4.50-4.67(1H,m) | | 935,765,600 |
| | | 6.93(1H,s) | CDCl₃ | 560 |
| 29 | 227 | 1.2-1.4(3H,d) 1.8-2.9(10H,m) | | 2970,1590,1400, |
| | | 3.1-3.4(1H,m) 3.6-4.2(3H,m) | | 1315,1295,1200, |
| | | 4.6-4.9(1H,m) 5.0-5.9(3H,m) | | 1130 |
| | | 7.16(1H,d) 7.80(1H,d) | | |
| | | | CDCl₃ | |

**Table 55**

| Prep. Exam.No. | Com'd No. | N.M.R. (ppm internal standard:TMS) | | IR (KBr) (cm⁻¹) |
|---|---|---|---|---|
| 30 | 228 | 1.90-2.90(8H,m) 2.32(3H,s) | | 3370,2950,1680 |
| | | 3.10-3.40(1H,m) 3.60-4.20(2H,m) | | 1550,1410,1310 |
| | | 4.31(2H,d) 4.88-5.08(1H,m) | | 1280,1180,1120 |
| | | 7.21(1H,d) 7.81(1H,d) | CDCl₃ | 1060,750 |
| 31 | 229 | 1.47(3H,d) 1.85-2.90(8H,m) 2.25(3H,s) | | 3280,2950,1680 |
| | | 3.05-4.40(4H,m) 4.80-5.00(0.5H,m) | | 1550,1420,1300 |
| | | 5.10-5.30(0.5H,m) 7.21(1H,d) | | 1290,1190,1120 |
| | | 7.83(1H,d) | | 1090,1050,910 |
| | | | CDCl₃ | 750,590 |
| 32 | 230 | 2.00-3.10(8H,m) 2.30(3H,s) | | 3450,2900,1680 |
| | | 3.10-3.30(1H,m) 3.30(3H,s) | | 1550,1430,1280 |
| | | 3.42-3.95(5H,m) 4.73-4.93(1H,m) | | 1130,1080 |
| | | 7.31(1H,d) 7.70(1H,d) | | |
| | | | CDCl₃ | |
| 33 | 231 | 1.14-1.26(3H,m) 1.90-3.65(14H,m) | | 3480,2950,1730 |
| | | 3.32(3H,s) 3.72-4.10(2H,m) | | 1670,1410,1300 |
| | | 4.75-4.95(0.5H,m) 5.00-5.20(0.5H,m) | | 1190,1120,1040 |
| | | 7.19(1H,d) 7.80(1H,d) | | 970,910,750 |
| | | 1:1 diastereomer mixture | | 600,550 |
| | | | CDCl₃ | |
| 34 | 241 | 0.15-0.32(2H,m) 0.44-0.68(2H,m) | | 2960,1730,1610 |
| | | 1.10-1.34(1H,m) 1.90-2.80(9H,m) | | 1410,1290,1190 |
| | | 2.30(3H,s) 3.10-4.30(3H,m) | | 1120,1050,910 |
| | | 4.55-4.75(1H,m) 7.20(1H,d) 7.82(1H,d) | | 800,750 |
| | | | CDCl₃ | |

**Table 56**

| Prep. Exam.No. | Com'd No. | N.M.R. (ppm internal standard:TMS) | | IR (KBr) (cm⁻¹) |
|---|---|---|---|---|
| 35 | 232 | 1.24(1.5H,d) 1.28(1.5H,d) | | 3020,2980,1690 |
| | | 1.90-2.90(8H,m) 2.79(3H,s) | | 1580,1320,1300 |
| | | 3.10-3.40(1H,m) 3.70-4.35(2H,m) | | 1150,1080,930 |
| | | 4.95-5.40(3H,m) 5.60-6.15(1H,m) | | 760 |
| | | 7.05(1H,s) | | |
| | | 1:1 diastereomer mixture | CDCl₃ | |
| 36 | 233 | 2.00-2.95(8H,m) 2.73(3H,s) | | 3380,2960,1740 |
| | | 3.15-3.35(1H,m) 3.83-4.40(2H,m) | | 1680,1570,1420 |
| | | 4.34(2H,d) 5.00-5.20(1H,m) | | 1310,1290,1130 |
| | | 7.07(1H,s) | | 1060,910,740 |
| | | | CDCl₃ | 590 |
| 37 | 234 | 1.43(1.5H,d) 1.48(1.5H,d) | | 2980,1600,1400 |
| | | 1.8-2.2(4H,m) 2.3-2.8(6H,m) | | 1320,1300,1145 |
| | | 2.71(3H,s) 3.0-3.4(1H,m) | | 1110,1070,920 |
| | | 3.6-4.1(1H,m) 5.02-5.15(0.5H,m) | | 750 |
| | | 5.35-5.45(0.5H,m) 7.07(1H,s) | | |
| | | | CDCl₃ | |
| 38 | 235 | 1.95-2.70(8H,m) 2.73(3H,s) | | 3460,2940,1680 |
| | | 3.00-4.20(6H,m) 3.35(3H,s) | | 1410,1290,1130 |
| | | 4.77-4.97(1H,m) | | 1070,910,740 |
| | | 7.05(1H,s) | | 590,550 |
| | | | CDCl₃ | |
| 39 | 236 | 1.20(1.5H,d) 1.23(1.5H,d) | | 3540,2940,1680 |
| | | 1.80-2.86(7H,m) 2.73(3H,s) | | 1560,1410,1310 |
| | | 2.90-3.68(4H,m) 3.34(3H,s) | | 1290,1130,1050 |
| | | 3.76-4.42(2H,m) 5.00-5.15(0.5H,m) | | 980,910,740 |
| | | 5.15-5.35(0.5H,m) 7.05(1H,s) | | 590 |
| | | 1:1 diastereomer mixture | | |
| | | | CDCl₃ | |

**Table 57**

| Prep. Exam.No. | Com'd No. | N.M.R. (ppm internal standard:TMS) | | IR (KBr) (cm⁻¹) |
|---|---|---|---|---|
| 40 | 16 | 0.84(3H,t) 1.19(3H,d) 1.34-1.68(2H,m) | | 3000,2970,2900 |
| | | 1.96-2.10(2H,m) 2.27-2.93(6H,m) | | 1740,1690,1570 |
| | | 2.73(3H,s) 3.85-4.26(1H,m) | | 1420,1390,1310 |
| | | 3.06-3.40(1H,m) 3.45-3.78(1H,m) | | 1290,1160,1140 |
| | | 3.85-4.26(1H,m) 5.00-5.10(1H,m) | | 1060,1000,920 |
| | | 7.04(1H,s) | | |
| | | 1:1 diastereomer mixture | CDCl₃ | |
| 41 | 242 | 0.2-0.4(2H,m) 0.5-0.7(2H,m) | | 2980,1625,1400 |
| | | 1.2-1.6(1H,m) 1.8-2.2(2H,m) | | 1320,1300,1190 |
| | | 2.3-2.8(6H,m) 2.71(3H,s) 2.92(2H,d) | | 11401080,760 |
| | | 3.0-3.6(2H,m) 4.7-4.9(1H,m) 7.03(1H,s) | | |
| | | | CDCl₃ | |
| 42 | 17 | 0.90(6H,d) 1.70-1.93(1H,m) | | 2980,2900,1680 |
| | | 1.97-2.19(2H,m) 2.37-2.87(6H,m) | | 1580,1420,1310 |
| | | 2.73(3H,s) 3.06-3.26(1H,m) | | 1140,1080,920 |
| | | 3.35(2H,d) 3.75-4.08(1H,m) | | 750,590 |
| | | 4.68-4.88(1H,m) 7.05(1H,s) | CDCl₃ | |
| 43 | 243 | 0.79(3H,d) 0.89(3H,d) 1.13(3H,d) | | 2980,2900,1680 |
| | | 1.65-2.19(3H,m) 2.28-2.94(6H,m) | | 1580,1420,1310 |
| | | 2.74(3H,s) 3.16-3.65(2H,m) | | 1140,1080,920 |
| | | 3.82-4.29(1H,m) 5.00-5.15(1H,m) | | 750,590 |
| | | 7.05(1H,s) | CDCl₃ | |
| 44 | 244 | 0.73(6H,s) 0.88(3H,s) 1.15(3H,d) 1.90-2.90(8H,m) 2.78(3H,s) 3.10-3.55(2H,m) 3.75-4.45(1H,m) 5.13(1H,broad) 7.05(1H,s) | | 3000,2900,1690 |
| | | | | 1580,1430,1390 |
| | | | | 1320,1300,1140 |
| | | | | 1100,1060,920 |
| | | | CDCl₃ | 850 |

**Table 58**

| Prep. Exam.No. | Compound No. | N.M.R. (ppm internal standard:TMS) | | IR (KBr) (cm⁻¹) |
|---|---|---|---|---|
| 45 | 168 | 2.20-3.15(6H,m) 2.64(3H,s) | | 2950,1665,1550 |
| | | 3.20-3.39(2H,m) 3.51-3.70(2H,m) | | 1410,1315,1280 |
| | | 4.01(3H,s) | | 1160,1040,935 |
| | | 7.19(1H,s) | | 740,590,550 |
| | | | deutero acetone | |
| 46 | 169 | 1.32(3H,t) 1.90-2.95(6H,m) | | 3550,2980,2950 |
| | | 2.70(3H,s) 3.20-3.51(4H,m) | | 1730,1680,1560 |
| | | 4.29(2H,q) | | 1410,1320,1150 |
| | | 7.00(1H,s) | | 1040 |
| | | | CDCl₃ | |
| 47 | 237 | 1.90-4.15(14H,m) | | 3450,2950,1680 |
| | | 3.29(3H,s) | | 1560,1400,1310 |
| | | 4.82-5.00(1H,m) | | 1130 |
| | | 7.50(1H,d) 7.85(1H,d) | | |
| | | | CDCl₃ | |
| 48 | 7 | 1.8-2.3(2H,m) 2.4-3.0(4H,m) | | 2920,1660,1590 |
| | | 3.2-4.1(6H,m) 4.8-5.0(1H,m) | | 1470,1395,1140 |
| | | 7.31(1H,d) | | 880,750 |
| | | 7.90(1H,d) | | |
| | | | CDCl₃ | |
| 49 | 8 | 0.91(3H,t) 1.8-2.2(2H,m) | | 3000,1660,1600 |
| | | 2.3-2.9(6H,m) 3.1-4.0(6H,m) | | 1350,1330,1150 |
| | | 4.70-4.92(1H,m) 7.31(1H,d) | | 1100,920,880 |
| | | 7.89(1H,d) | | |
| | | | CDCl₃ | |

**Table 59**

| Prep. Exam.No. | Com'd No. | N.M.R. (ppm internal standard:TMS) | | IR (KBr) (cm⁻¹) |
|---|---|---|---|---|
| 50 | 9 | 1.22(6H,d) 1.86-2.25(2H,m) | | 3500,3000,1680 |
| | | 2.30-2.90(6H,m) 3.10-3.40(1H,m) | | 1600.1420,1320, |
| | | 3.70-4.20(2H,m) 4.99(1H,m) | | 1150,1060 |
| | | 7.30(1H,d) 7.89(1H,d) | | |
| | | | CDCl₃ | |
| 51 | 10 | 0.85(3H,t) 1.21(3H,d) 1.42-1.72(2H,m) | | 3470,3000,2960 |
| | | 1.94-2.22(2H,m) 2.37-2.98(6H,m) | | 2900,1690,1570 |
| | | 3.07-3.42(1H,m) 3.54-4.06(2H,m) | | 1420,1320,1300 |
| | | 4.92-5.12(1H,m) 7.30(1H,d) | | 1160,1140,1060 |
| | | 7.91(1H,d) | CDCl₃ | 1000,950,750 |
| 52 | 245 | 0.90(9H,s) 2.2-2.9(8H,m) | | 3400,2995,2990 |
| | | 3.1-3.9(4H,m) 4.70-4.90(1H,m) | | 1695,1580,1330 |
| | | 7.33(1H,d) | | 1150,1095,1020 |
| | | 7.89(1H,d) | | |
| | | | CDCl₃ | |
| 53 | 154 | 1.92-2.22(2H,m) 2.30-3.04(6H,m) | | 3460,2960,1740 |
| | | 3.13-3.42(1H,m) 3.56-3.73(2H,m) | | 1680,1590,1570 |
| | | 3.77-4.18(3H,m) 4.78-4.95(1H,m) | | 1420,1340,1320 |
| | | 7.32(1H,d) 7.92(1H,d) | | 1300,1160,1140, |
| | | | CDCl₃ | 1110,760 |
| 54 | 152 | 2.2-3.0(8H,m) 3.2-3.4(1H,m) | | 3360,2975,1690 |
| | | 3.7-4.1(3H,m) 4.93-5.08(1H,m) | | 1420,1300,1150 |
| | | 7.93(1H,d) | | 1020,980,920 |
| | | 7.36(1H,d) | | 840,750 |
| | | | CDCl₃ | |

**Table 60**

| Prep. Exam.No. | Com'd No. | N.M.R. (ppm internal standard:TMS) | | IR (KBr) (cm⁻¹) |
|---|---|---|---|---|
| 55 | 246 | 2.2-3.0(8H,m) 3.2-3.5(1H,m) | | 3400,2975,2900 |
| | | 3.8-4.5(4H,m) 4.6-5.0(2H,m) | | 1690,1580,1345 |
| | | 5.1-5.3(1H,m) | | 1320,1300,1200 |
| | | 7.31(1H,s) | | 1140,1100,1080 |
| | | | CDCl₃ | 920,750 |
| 56 | 250 | 1.98-2.20(2H,m) 2.29-2.95(6H,m) | | 2950,1740,1680 |
| | | 2.74(3H,s) 3.06-3.42(1H,m) | | 1570,1310,1290 |
| | | 3.51-3.77(2H,m) 3.80-4.26(3H,m) | | 1150,1130,1070 |
| | | 4.80-5.00(1H,m) 7.07(1H,s) | | 910,750,590,550 |
| | | | CDCl₃ | |
| 57 | 251 | 0.91(3H,t) 1.48-1.81(2H,m) | | 2970,2950,2880 |
| | | 1.97-2.18(8H,m) 2.26-2.87(6H,m) | | 1680,1580,1560 |
| | | 2.72(3H,s) 3.09-3.35(1H,m) | | 1410,1310,1290 |
| | | 3.46-3.61(2H,m) 3.77-4.17(1H,m) | | 1150,1130,1070 |
| | | 4.70-4.90(1H,m) 7.05(1H,s) | | 910,740,590,540 |
| | | | CDCl₃ | |

### Herbicide Examples

### (1) Preparation of herbicides

97 Parts by weight of talc (trade name: Zeaklite, supplied by Zeaklite Industry) as a carrier, 1.5 parts by weight of alkylarylsulfonic acid salt(trade name: Neoplex, supplied by Kao-Atlas K.K.) as a surfactant and 1.5 parts by weight of a nonionic and anionic surfactant (trade name: Sorpol 800A, supplied by Toho Chemical Co., Ltd.) were uniformly pulverized and mixed to prepare a carrier for a wettable powder.

90 Parts by weight of the above carrier for a wettable powder and 10 parts by weight of one of the compounds of the of the present invention were uniformly pulverized and mixed to obtain herbicides. Further, in Comparative Herbicide Examples, comparative herbicides were also prepared from the following compounds (A) to (G) in the same manner.

The compounds (A) and (B)are disclosed in WO94/08988, the compounds (C), (D) and (E) are disclosed in European Patent 94/283261, and the compounds (F) and (G) are disclosed in WO 94/04524. (2) Ratings of evaluation of herbicidal efficacy and phytotoxicity to crops

The ratio of remaining plant weight to plant weight in non-treated plot was determined on the basis of the ratio of remaining plant weight to plant weight in non-treated plot = (remaining plant weight in treated plot/plant weight in non-treated plot) x 100. The ratings were applied to the following biological tests.

### Ratings

| Herbicidal efficacy | Ratio of remaining plant weight to plant weight in non-treated plot (%) |
|---|---|
| 0 | 81 - 100 |
| 1 | 61 - 80 |
| 2 | 41 - 60 |
| 3 | 21 - 40 |
| 4 | 1 - 20 |
| 5 | 0 |

| Phytotoxicity to crops | Ratio of remaining plant weight to plant weight in non-treated plot (%) |
|---|---|
| - | 100 |
| ± | 95 - 99 |
| + | 90 - 94 |
| ++ | 80 - 89 |
| +++ | 0 - 79 |

### (3) Biological tests

### (a) Upland pre-emergence treatment test 1

An upland pre-emergence treatment test was carried out in the following manner with regard to Compounds Nos. 1 to 5 (Examples) and Compounds (A) to (E) (Comparative Examples).

Seeds of weeds such as velvetleaf, Jimsonweed, black nightshade, barnyardgrass and large crabgrass and seeds of corn, sorgo and cotton were sown in 1/5,000-are Wagner pots filled with upland soil, and covered with upland soil. Then, a predetermined amount of the herbicide prepared in the above (1) was suspended in water, and the suspension was uniformly sprayed onto the soil surface. Then, the seeds were grown in a greenhouse, and on the 20th day after the treatment, the herbicide was evaluated for herbicidal efficacy and phytotoxicity to the crops on the basis of the ratings shown in (2). Table 61 shows the results.

**Table 61**

| Com'd No. | Dosage (g/ha) | Herbicidal efficacy | | | | | Phytotoxicity | | |
|---|---|---|---|---|---|---|---|---|---|
| | | AA | BB | CC | DD | EE | FF | GG | HH |
| 1 | 300 | 5 | 5 | 5 | 3 | 0 | - | - | - |
| 2 | 300 | 5 | 5 | 5 | 4 | 3 | - | - | - |
| 3 | 300 | 5 | 5 | 5 | 5 | 5 | - | - | - |
| 4 | 100 | 5 | 5 | 5 | 0 | 0 | - | - | - |
| 5 | 300 | 5 | 5 | 5 | 0 | 3 | - | - | - |
| A | 300 | 5 | 5 | 5 | 3 | 1 | - | ++ | ++ |
| B | 300 | 1 | 0 | 0 | 0 | 0 | - | - | - |
| C | 300 | 0 | 1 | 1 | 0 | 0 | - | - | - |
| D | 300 | 1 | 0 | 1 | 0 | 0 | - | - | - |
| E | 300 | 0 | 0 | 0 | 0 | 0 | - | - | - |

| | | | | | | | | | |
|---|---|---|---|---|---|---|---|---|---|
| AA = Velvetleaf, | | | | | | | | | |
| BB = Jimsonweed, | | | | | | | | | |
| CC = Black nightshade, | | | | | | | | | |
| DD = Barnyardgrass, | | | | | | | | | |
| EE = Large crabgrass, | | | | | | | | | |
| FF = Corn, | | | | | | | | | |
| GG = Sorgo, | | | | | | | | | |
| HH = Cotton | | | | | | | | | |

Table 61 shows that the herbicides of the present invention can selectively control a broad range of upland soil weeds at a low dosage without causing phytotoxicity on corn, sorgo and cotton. In contrast, it is also shown that Compound A is poor in safety to sorgo and cotton, and that Compounds B to E are all poor in the efficacy on all the test weeds.

### (b) Upland post-emergence treatment test 1

An upland post-emergence treatment test was carried out in the following manner with regard to Compounds Nos. 1 to 5 (Examples) and Compounds (A), (C), (D) and (E) (Comparative Examples).

Seeds of weeds such as cocklebur, velvetleaf, Jimsonweed, barnyardgrass and large crabgrass and seeds of corn, sorgo and beet were sown in 1/5,000-are Wagner pots filled with upland soil, and covered with upland soil. The seeds were grown in a greenhouse, and at the stage of 3 to 4 leaves of these plants, a predetermined amount of the herbicide prepared in the above (1) was suspended in water, and the suspension was uniformly sprayed onto leaf and stalk portions at a rate of 2,000 liters/ha. Then, the plants were grown in the greenhouse, and on the 30th day after the treatment, the herbicide was evaluated for herbicidal efficacy and phytotoxicity to the crops on the basis of the ratings shown in (2). Table 62 shows the results.

**Table 62**

| Com'd No. | Dosage (g/ha) | Herbicidal efficacy | | | | | Phytotoxicity | | |
|---|---|---|---|---|---|---|---|---|---|
| | | AA | BB | CC | DD | EE | FF | GG | HH |
| 1 | 300 | 5 | 5 | 4 | 0 | 0 | - | - | - |
| 2 | 300 | 5 | 5 | 5 | 4 | 4 | - | - | ± |
| 3 | 300 | 5 | 5 | 3 | 3 | 3 | - | - | ± |
| 4 | 100 | 4 | 5 | 5 | 4 | 3 | - | - | ± |
| 5 | 300 | 5 | 4 | 2 | 4 | 3 | - | - | - |
| A | 300 | 5 | 5 | 5 | 4 | 0 | - | ++ | +++ |
| C | 300 | 5 | 0 | 5 | 0 | 1 | - | ++ | +++ |
| D | 300 | 5 | 4 | 5 | 0 | 1 | - | ++ | +++ |
| E | 300 | 0 | 0 | 0 | 0 | 0 | - | - | - |

| | | | | | | | | | |
|---|---|---|---|---|---|---|---|---|---|
| AA = Cocklebur, | | | | | | | | | |
| BB = Velvetleaf, | | | | | | | | | |
| CC = Jimsonweed, | | | | | | | | | |
| DD = Barnyardgrass, | | | | | | | | | |
| EE = Large crabgrass, | | | | | | | | | |
| FF = Corn, | | | | | | | | | |
| GG = Sorgo, | | | | | | | | | |
| HH = Beet | | | | | | | | | |

Table 62 shows that the herbicides of the present invention show no phytotoxicity on corn and sorgo, has selectivity for beet and further can selectively control a broad range of upland soil weeds at a low dosage. In contrast, it is also shown that Compounds A, C and D are poor in safety to sorgo and beet and that Compound E is poor in efficacy on all the test weeds.

### (c) Upland pre-emergence treatment test 2

An upland pre-emergence treatment test was carried out in the following manner with regard to Compounds Nos. 6, 15, 104 and 165 (Examples) and Compound (F) (Comparative Example).

Seeds of weeds such as velvetleaf, black nightshade, barnyardgrass, large crabgrass and giant foxtail and seeds of corn and cotton were sown in 1/5,000-are Wagner pots filled with upland soil, and covered with upland soil. Then, a predetermined amount of the herbicide prepared in the above (1) was suspended in water and uniformly sprayed onto the soil surface. Then, the seeds were grown in a greenhouse, and on the 20th day after the treatment, the herbicide was evaluated for herbicidal efficacy and phytotoxicity to the crops on the basis of the ratings shown in (2). Table 63 shows the results.

**Table 63**

| Com'd No. | Dosage (g/ha) | Herbicidal efficacy | | | | | Phytotoxicity | |
|---|---|---|---|---|---|---|---|---|
| | | AA | BB | CC | DD | EE | FF | GG |
| 6 | 100 | 5 | 5 | 3 | 5 | 3 | - | - |
| 15 | 100 | 5 | 5 | 5 | 4 | 4 | - | - |
| 104 | 100 | 5 | 5 | 4 | 5 | 4 | - | - |
| 165 | 100 | 5 | 5 | 5 | 5 | 3 | - | - |
| F | 100 | 5 | 5 | 0 | 1 | 0 | - | - |

| | | | | | | | | |
|---|---|---|---|---|---|---|---|---|
| AA = Velvetleaf, | | | | | | | | |
| BB = Black nightshade, | | | | | | | | |
| CC = Barnyardgrass, | | | | | | | | |
| DD = Large crabgrass, | | | | | | | | |
| EE = Giant foxtail, | | | | | | | | |
| FF = Corn, | | | | | | | | |
| GG = Cotton | | | | | | | | |

Table 62 shows that the herbicides of the present invention cause no phytotoxicity on corn and cotton and can selectively control a broad range of upland soil weeds at a low dosage. In contrast, it is shown that Compound F is poor in efficacy on grass weeds.

### (d) Upland post-emergence treatment test 2

An upland post-emergence treatment test was carried out in the following manner with regard to Compounds Nos. 15, 104, 165 and 202 (Examples) and Compound (F) (Comparative Example).

Seeds of weeds such as cocklebur, velvetleaf, black nightshade, barnyardgrass, large crabgrass and giant foxtail and seeds of corn and sorgo were sown in 1/5,000-are Wagner pots filled with upland soil, and covered with upland soil. The seeds were grown in a greenhouse, and at the stage of 3 - 4 leaves of these plants, a predetermined amount of the herbicide prepared in the above (1) was suspended in water and the suspension was uniformly sprayed onto leaf and stalk portions at a rate of 2,000 ℓ/ha. Then, the plants were grown in the greenhouse, and on the 30th day after the treatment, the herbicide was evaluated for herbicidal efficacy and phytotoxicity to the crops on the basis of the ratings shown in (2). Table 64 shows the results.

**Table 64**

| Com'd No. | Dosage (g/ha) | Herbicidal efficacy | | | | | | Phytotoxicity | |
|---|---|---|---|---|---|---|---|---|---|
| | | AA | BB | CC | DD | EE | FF | GG | HH |
| 15 | 100 | 5 | 5 | 5 | 5 | 4 | 5 | - | - |
| 104 | 100 | 5 | 5 | 5 | 4 | 4 | 3 | - | - |
| 165 | 100 | 5 | 5 | 5 | 5 | 4 | 4 | - | - |
| 202 | 100 | 5 | 5 | 5 | 4 | 4 | 3 | - | - |
| F | 100 | 5 | 5 | 5 | 0 | 0 | 0 | - | - |

| | | | | | | | | | |
|---|---|---|---|---|---|---|---|---|---|
| AA = Cocklebur, | | | | | | | | | |
| BB = Velvetleaf, | | | | | | | | | |
| CC = Black nightshade, | | | | | | | | | |
| DD = Barnyardgrass | | | | | | | | | |
| EE = Large crabgrass, | | | | | | | | | |
| FF = Giant foxtail, | | | | | | | | | |
| GG = Corn, | | | | | | | | | |
| HH = Cotton | | | | | | | | | |

Table 64 shows that the herbicides of the present invention do not cause phytotoxicity on corn and sorgo and can selectively control a broad range of upland soil weeds at a low dosage. In contrast, it is shown that Compound F is poor in efficacy on grass weeds.

### (e) Upland post-emergence treatment test 3

An upland post-emergence treatment test was carried out in the following manner with regard to Compound No. 15 (Example) and Compound (G) (Comparative

Example). Seeds of weeds such as velvetleaf, common ragweed, barnyardgrass and giant foxtail and seeds of corn and sorgo were sown in 1/5,000-are Wagner pots filled with upland soil, and covered with upland soil. The seeds were grown in a greenhouse, and at the stage of 3 - 4 leaves of these plants, a predetermined amount of the herbicide prepared in the above (1) was suspended in water, and the suspension was uniformly sprayed onto leaf and stalk portions at a rate of 2,000 liters/ha. Then, the plants were grown in the greenhouse, and on the 30th day after the treatment, the herbicide was evaluated for herbicidal efficacy and phytotoxicity to the crops on the basis of the ratings shown in (2). Table 65 shows the results.

**Table 65**

| Com'd No. | Dosage (g/ha) | Herbicidal efficacy | | | | Phytotoxicity | |
|---|---|---|---|---|---|---|---|
| | | AA | BB | CC | DD | EE | FF |
| 15 | 50 | 5 | 5 | 5 | 5 | - | - |
| G | 50 | 5 | 2 | 1 | 0 | - | - |

| | | | | | | | |
|---|---|---|---|---|---|---|---|
| AA = Velvetleaf, | | | | | | | |
| BB = Common ragweed, | | | | | | | |
| CC = Barnyardgrass, | | | | | | | |
| DD = Giant foxtail, | | | | | | | |
| EE = Corn, | | | | | | | |
| GG = Sorgo | | | | | | | |

Table 65 shows that the herbicide of the present invention causes no phytotoxicity on corn and sorgo and further that it can selectively control main upland soil weeds at a low dosage. In contrast, it is shown that Compound G is poor in efficacy on common ragweed, barnyardgrass and giant foxtail which are mainly to be controlled in upland fields.

### (f) Upland post-emergence treatment test 4

An upland post-emergence treatment test was carried out in the following manner with regard to Compounds Nos. 7 to 9, 16, 17, 154, 168, 169, 216 to 244, 250 and 251 (Examples) and Compound (F) (Comparative Example).

Seeds of weeds such as cocklebur, velvetleaf, common lambsguaters, common ragweed, large crabgrass and green foxtail and seeds of corn were sown in 1/5,000-are Wagner pots filled with upland soil, and covered with upland soil. The seeds were grown in a greenhouse, and at the stage of 3 - 4 leaves of these plants, a predetermined amount of the herbicide prepared in the above (1) was suspended in water and the suspension was uniformly sprayed onto leaf and stalk portions at a rate of 2,000 liters/ha. Then, the plants were grown in the greenhouse, and on the 30th day after the treatment, the herbicide was evaluated for herbicidal efficacy and phytotoxicity to the crop on the basis of the ratings shown in (2). Table 66 shows the results.

**Table 66**

| Com'd No. | Dosage (g/ha) | Herbicidal efficacy | | | | | | Phytotoxicity Corn |
|---|---|---|---|---|---|---|---|---|
| | | AA | BB | CC | DD | EE | FF | |
| 7 | 300 | 5 | 5 | 5 | 5 | 5 | 4 | - |
| 8 | 300 | 5 | 5 | 5 | 5 | 4 | 4 | - |
| 9 | 300 | 5 | 5 | 5 | 5 | 4 | 4 | - |
| 16 | 300 | 5 | 5 | 5 | 5 | 5 | 5 | - |
| 17 | 300 | 5 | 5 | 5 | 5 | 5 | 5 | - |
| 154 | 300 | 4 | 5 | 5 | 5 | 5 | 4 | - |
| 168 | 300 | 5 | 5 | 5 | 5 | 5 | 5 | ± |
| 169 | 300 | 5 | 5 | 5 | 5 | 5 | 5 | ± |
| 216 | 300 | 5 | 5 | 5 | 5 | 5 | 5 | - |
| 217 | 300 | 5 | 5 | 5 | 5 | 4 | 4 | - |
| 218 | 300 | 5 | 5 | 5 | 5 | 5 | 5 | - |
| 219 | 300 | 5 | 5 | 5 | 5 | 5 | 5 | - |
| 220 | 300 | 5 | 5 | 5 | 5 | 5 | 5 | - |
| 221 | 300 | 5 | 5 | 4 | 5 | 5 | 4 | - |
| 222 | 300 | 5 | 5 | 5 | 4 | 4 | 5 | - |
| 223 | 300 | 5 | 5 | 5 | 5 | 5 | 5 | - |
| 224 | 300 | 5 | 5 | 5 | 5 | 5 | 5 | - |
| 225 | 300 | 4 | 5 | 5 | 5 | 5 | 5 | - |
| 226 | 300 | 4 | 5 | 5 | 5 | 5 | 5 | - |
| 227 | 300 | 5 | 5 | 4 | 4 | 5 | 5 | - |
| 228 | 300 | 5 | 5 | 5 | 5 | 5 | 5 | - |
| 229 | 300 | 5 | 5 | 5 | 5 | 5 | 5 | - |
| 230 | 300 | 5 | 5 | 5 | 5 | 5 | 5 | - |
| 231 | 300 | 5 | 5 | 5 | 5 | 4 | 4 | - |
| 232 | 300 | 5 | 5 | 5 | 5 | 5 | 5 | - |
| 233 | 300 | 5 | 5 | 5 | 5 | 5 | 5 | - |
| 234 | 300 | 5 | 5 | 5 | 5 | 5 | 5 | - |
| 235 | 300 | 5 | 5 | 5 | 5 | 5 | 5 | - |
| 236 | 300 | 5 | 5 | 5 | 5 | 5 | 5 | - |
| 237 | 300 | 5 | 5 | 5 | 5 | 5 | 5 | - |
| 238 | 300 | 5 | 5 | 5 | 5 | 5 | 5 | - |
| 239 | 300 | 5 | 5 | 5 | 5 | 5 | 5 | - |
| 240 | 300 | 5 | 5 | 5 | 5 | 5 | 5 | - |
| 241 | 300 | 5 | 5 | 5 | 5 | 5 | 5 | - |
| 242 | 300 | 5 | 5 | 5 | 5 | 5 | 4 | - |
| 243 | 300 | 5 | 5 | 5 | 5 | 4 | 4 | - |
| 244 | 300 | 5 | 5 | 5 | 5 | 4 | 4 | - |
| 250 | 300 | 5 | 5 | 5 | 5 | 4 | 4 | - |
| 251 | 300 | 5 | 5 | 5 | 5 | 5 | 5 | ± |
| F | 300 | 5 | 5 | 5 | 5 | 2 | 1 | ++ |

| | | | | | | | | |
|---|---|---|---|---|---|---|---|---|
| AA = Cocklebur, | | | | | | | | |
| BB = Velvetleaf, | | | | | | | | |
| CC = Common lambsquaters, | | | | | | | | |
| DD = Common ragweed, | | | | | | | | |
| EE = Large crabgrass, | | | | | | | | |
| FF = Green foxtail | | | | | | | | |

Table 66 shows that the herbicides of the present invention can selectively control upland soil weeds at a low dosage almost without causing phytotoxicity on corn. In contrast, it is shown that Compound F is poor in safety for corn and efficacy on upland soil weeds.

### Industrial Utility

The cyclohexanedione derivative of the present invention can selectively control a broad range of upland soil weeds at a low dosage both in pre-emergence treatment and in post-emergence treatment without causing phytotoxicity on crops cultivated in upland fields such as corn.

## Claims

1. A cyclohexanedione derivative of the general formula (I), 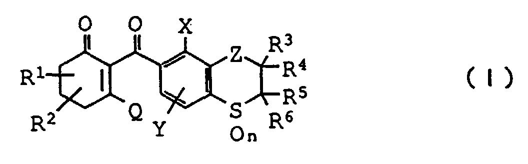 wherein:
each of R¹ and R² is independently a hydrogen atom or a C₁-C₆ alkyl group,
each of R³ to R⁶ is independently a hydrogen atom, a C₁-C₆ alkyl group, a C₁-C₆ haloalkyl group or a halogen atom,
n is 0, 1 or 2,
X is a C₁-C₆ alkyl group, a C₁-C₆ haloakyl group, a halogen atom, a C₁-C₆ alkoxy group, a C₁-C₆ haloalkoxy group, a C₂-C₆ alkoxyalkyl group, a C₁-C₆ alkylthio group, a C₁-C₆ haloalkylthio group, a C₁-C₆ alkylsulfinyl group or a C₁-C₆ alkylsulfonyl group,
Y is a hydrogen atom, a C₁-C₆ alkyl group, C₁-C₆ haloalkyl group, a halogen atom, a C₁-C₆ alkoxy group, a C₁-C₆ haloalkoxy group or a C₂-C₆ alkoxyalkyl group,
Z is a group of 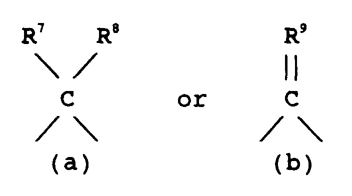
in which each of R⁷ and R⁸ is independently a hydrogen atom, a halogen atom, a C₁-C₆ alkyl group, a C₁-C₆ alkoxy group, a C₁-C₆ alkylthio group or a group of -NR¹⁰R¹¹, provided that when R⁷, R⁸ or both is/are C₁-C₆ alkyl, C₁-C₆ alkoxy or C₁-C₆ alkylthio group(s), 1 to 13 hydrogen atoms thereof may be substituted with 1 to 13 halogen atoms or C₁-C₆ alkoxy groups and that when the carbon number thereof is C₂-C₆, the group(s) may contain an unsaturated bond and that when it is C₃-C₆, the group(s) may have a cyclized structure, each of R¹⁰ and R¹¹ is a hydrogen atom, a C₁-C₆ alkyl group or a C₁-C₆ alkylcarbonyl group, further provided that when both R⁷ and R⁸ are C₁-C₆ alkyl groups, C₁-C₆ alkoxy groups or C₁-C₆ alkylthio groups, carbon atoms of R⁷ and R⁸ may bond to each other to form a 3- to 7-membered ring, provided that when both R⁷ and R⁸ are alkyl groups, compounds of the general formula (I) in which X is a C₁-C₆ alkyl group, a halogen atom or a haloalkyl group and all of R³, R⁴, R⁵ and R⁶ are hydrogen atoms are excluded,
provided that when one of R⁷ and R⁸ is an alkoxy group and when the other is a hydrogen atom, compounds of the general formula (I) in which X is a C₁-C₆ alkyl group and all of R³, R⁴, R⁵ and R⁶ are hydrogen atoms are excluded when hydrogen atom of the alkoxy group is not replaced with halogen or when the alkoxy group does not contain an unsaturated bond or a cyclized structure, and
R⁹ is an oxygen atom, a sulfur atom or a C₁-C₆ alkoxyimino group, provided that when R⁹ is a C₁-C₆ alkoxyimino group, 1 to 13 hydrogen atoms thereof may be replaced with 1 to 13 halogen atoms and that when the carbon number of R⁹ is C₂-C₆, the C₂-C₆ alkoxyimino group may contain an unsaturated bond, and provided that compounds of the general formula (I) in which X is a C₁-C₆ alkyl group and all of R³, R⁴, R⁵ and R⁶ are hydrogen atoms are excluded when R⁹ is an alkoxyimino group and when hydrogen atom thereof is not replaced with a halogen or the alkoxyimino group does not contain an unsaturated bond, and
Q is a hydroxyl group or a group of 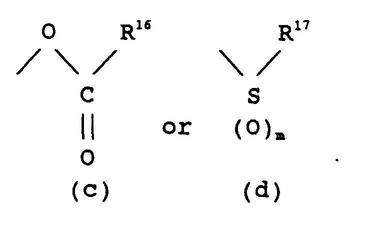
in which each of R¹⁶ and R¹⁷ is a C₁-C₆ alkyl group, a C₁-C₆ haloalkyl group or a phenyl group which may be substituted with a C₁-C₆ alkyl group, a C₁-C₆ haloalkyl group, a halogen atom, a cyano group or a nitro group, and m is 0, 1 or 2, or a salt thereof.

2. A cyclohexanedione derivative of the general formula (I-a1), 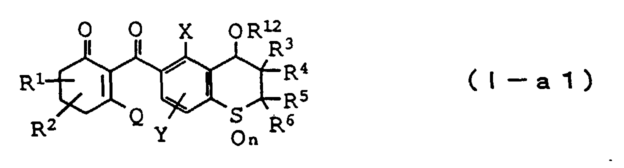 wherein:
each of R¹ and R² is independently a hydrogen atom or a C₁-C₆ alkyl group,
each of R³ to R⁶ is independently a hydrogen atom, a C₁-C₆ alkyl group, a C₁-C₆ haloalkyl group or a halogen atom,
n is 0, 1 or 2,
X is a C₁-C₆ alkyl group, a C₁-C₆ haloakyl group, a halogen atom, a C₁-C₆ alkoxy group, a C₁-C₆ haloalkoxy group, a C₂-C₆ alkoxyalkyl group, a C₁-C₆ alkylthio group, a C₁-C₆ haloalkylthio group, a C₁-C₆ alkylsulfinyl group or a C₁-C₆ alkylsulfonyl group,
Y is a hydrogen atom, a C₁-C₆ alkyl group, C₁-C₆ haloalkyl group, a halogen atom, a C₁-C₆ alkoxy group, a C₁-C₆ haloalkoxy group or a C₂-C₆ alkoxyalkyl group,
R¹² is a C₁-C₆ alkyl group or a C₁-C₆ haloalkyl group, provided that R¹² may be substituted with a C₁-C₆ alkoxy group, that when the number of carbon atoms of R¹² is C₂-C₆, R¹² may contain an unsaturated bond, that wnen the number of carbon atoms of R¹² is C₃-C₆, R¹² may contain a cyclic structure, and further that when R¹² is a C₁-C₆ alkyl group, compounds of the general formula (I-a1) in which X is a C₁-C₆ alkyl group and all of R³, R⁴, R⁵ and R⁶ are hydrogen atoms are excluded, and
Q is a hydroxyl group or a group of 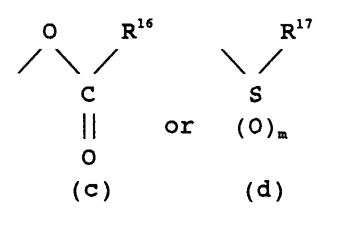
in which each of R¹⁶ and R¹⁷ is a C₁-C₆ alkyl group, a C₁-C₆ haloalkyl group or a phenyl group which may be substituted with a C₁-C₆ alkyl group, a C₁-C₆ haloalkyl group, a halogen atom, a cyano group or a nitro group, and m is 0, 1 or 2, or a salt thereof.

3. A cyclohexanedione derivative of the general formula (I-a2), 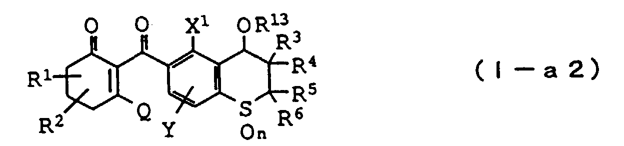 wherein:
each of R¹ and R² is independently a hydrogen atom or a C₁-C₆ alkyl group,
each of R³ to R⁶ is independently a hydrogen atom, a C₁-C₆ alkyl group, a C₁-C₆ haloalkyl group or a halogen atom,
n is 0, 1 or 2,
X¹ is a C₁-C₆ haloalkyl group, a halogen atom, a C₁-C₆ alkoxy group, a C₁-C₆ haloalkoxy group, a C₂-C₆ alkoxyalkyl group, a C₁-C₆ alkylthio group, a C₁-C₆ haloalkylthio group, a C₁-C₆ alkylsulfinyl group or a C₁-C₆ alkylsulfonyl group,
Y is a hydrogen atom, a C₁-C₆ alkyl group, C₁-C₆ haloalkyl group, a halogen atom, a C₁-C₆ alkoxy group, a C₁-C₆ haloalkoxy group or a C₂-C₆ alkoxyalkyl group, and
Q is a hydroxyl group or a group of 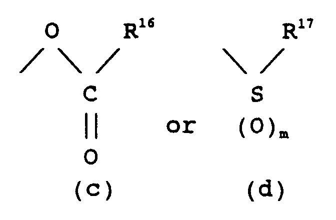
in which each of R¹⁶ and R¹⁷ is a C₁-C₆ alkyl group, a C₁-C₆ haloalkyl group or a phenyl group which may be substituted with a C₁-C₆ alkyl group, a C₁-C₆ haloalkyl group, a halogen atom, a cyano group or a nitro group, and m is 0, 1 or 2, or a salt thereof.

4. A cyclohexanedione derivative of the general formula (I-a3), 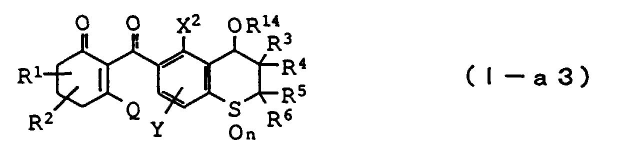 wherein:
each of R¹ and R² is independently a hydrogen atom or a C₁-C₆ alkyl group,
each of R³ to R⁶ is independently a hydrogen atom, a C₁-C₆ alkyl group, a C₁-C₆ haloalkyl group or a halogen atom, R¹⁴ is a C₁-C₆ haloalkyl group, an alkoxyalkyl group, an alkenyl group or an alkynylalkyl group,
n is 0, 1 or 2,
X² is a C₁-C₆ alkyl group, a C₁-C₆ haloalkyl group, a halogen atom, a C₁-C₆ alkoxy group, a C₁-C₆ haloalkoxy group, a C₂-C₆ alkoxyalkyl group, a C₁-C₆ alkylthio group, a C₁-C₆ haloalkylthio group, a C₁-C₆ alkylsulfinyl group or a C₁-C₆ alkylsulfonyl group, or salts thereof,
Y is a hydrogen atom, a C₁-C₆ alkyl group, C₁-C₆ haloalkyl group, a halogen atom, a C₁-C₆ alkoxy group, a C₁-C₆ haloalkoxy group or a C₂-C₆ alkoxyalkyl group, and
Q is a hydroxyl group or a group of 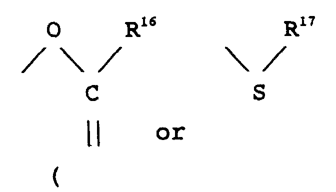
in which each of R¹⁶ and R¹⁷ is a C₁-C₆ alkyl group, a C₁-C₆ haloalkyl group or a phenyl group which may be substituted with a C₁-C₆ alkyl group, a C₁-C₆ haloalkyl group, a halogen atom, a cyano group or a nitro group, and m is 0, 1 or 2, or a salt thereof.

5. A cyclohexanedione derivative of the general formula (I-b1), 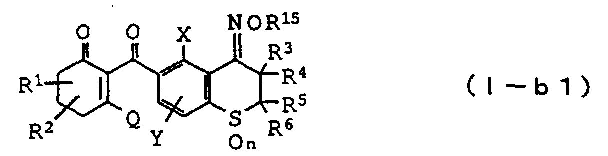 wherein:
each of R¹ and R² is independently a hydrogen atom or a C₁-C₆ alkyl group,
each of R³ to R⁶ is independently a hydrogen atom, a C₁-C₆ alkyl group, a C₁-C₆ haloalkyl group or a halogen atom,
n is 0, 1 or 2,
X is a C₁-C₆ alkyl group, a C₁-C₆ haloakyl group, a halogen atom, a C₁-C₆ alkoxy group, a C₁-C₆ haloalkoxy group, a C₂-C₆ alkoxyalkyl group, a C₁-C₆ alkylthio group, a C₁-C₆ haloalkylthio group, a C₁-C₆ alkylsulfinyl group or a C₁-C₆ alkylsulfonyl group,
Y is a hydrogen atom, a C₁-C₆ alkyl group, C₁-C₆ haloalkyl group, a halogen atom, a C₁-C₆ alkoxy group, a C₁-C₆ haloalkoxy group or a C₂-C₆ alkoxyalkyl group,
R¹⁵ is a C₁-C₆ alkyl group or a C₁-C₆ alkenyl group, provided that when R¹⁵ is a C₁-C₆ alkyl group, compounds of the general formula (I-b1) in which X is a C₁-C₆ alkyl group and all of R³, R⁴, R⁵ and R⁶ are hydrogen atoms are excluded, and
Q is a hydroxyl group or a group of 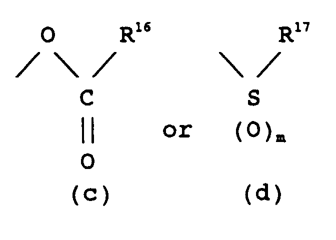
in which each of R¹⁶ and R¹⁷ is a C₁-C₆ alkyl group, a C₁-C₆ haloalkyl group or a phenyl group which may be substituted with a C₁-C₆ alkyl group, a C₁-C₆ haloalkyl group, a halogen atom, a cyano group or a nitro group, and m is 0, 1 or 2, or a salt thereof.

6. A cyclohexanedione derivative of the general formula (I-b2), 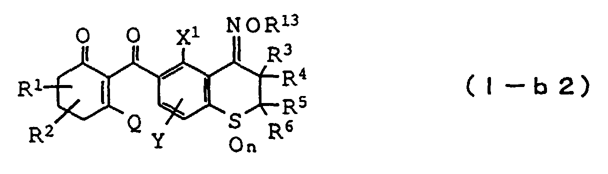 wherein:
each of R¹ and R² is independently a hydrogen atom or a C₁-C₆ alkyl group,
each of R³ to R⁶ is independently a hydrogen atom, a C₁-C₆ alkyl group, a C₁-C₆ haloalkyl group or a halogen atom, R¹³ is a C₁-C₆ alkyl group,
n is 0, 1 or 2,
X¹ is a C₁-C₆ haloalkyl group, a halogen atom, a C₁-C₆ alkoxy group, a C₁-C₆ haloalkoxy group, a C₂-C₆ alkoxyalkyl group, a C₁-C₆ alkylthio group, a C₁-C₆ haloalkylthio group, a C₁-C₆ alkylsulfinyl group or a C₁-C₆ alkylsulfonyl group,
Y is a hydrogen atom, a C₁-C₆ alkyl group, C₁-C₆ haloalkyl group, a halogen atom, a C₁-C₆ alkoxy group, a C₁-C₆ haloalkoxy group or a C₂-C₆ alkoxyalkyl group, and
Q is a hydroxyl group or a group of 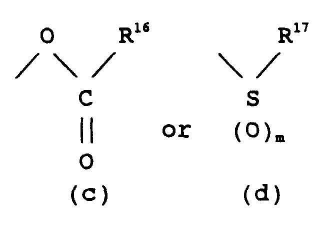
in which each of R¹⁶ and R¹⁷ is a C₁-C₆ alkyl group, a C₁-C₆ haloalkyl group or a phenyl group which may be substituted with a C₁-C₆ alkyl group, a C₁-C₆ haloalkyl group, a halogen atom, a cyano group or a nitro group, and m is 0, 1 or 2, or a salt thereof.

7. A cyclohexanedione derivative of the general formula (I-c), 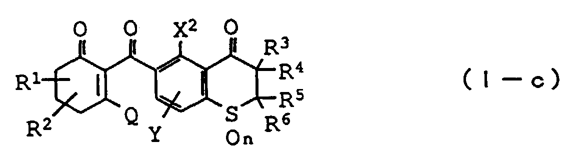 wherein:
each of R¹ and R² is independently a hydrogen atom or a C₁-C₆ alkyl group,
each of R³ to R⁶ is independently a hydrogen atom, a C₁-C₆ alkyl group, a C₁-C₆ haloalkyl group or a halogen atom,
n is 0, 1 or 2,
X² is a C₁-C₆ alkyl group, a C₁-C₆ haloalkyl group, a halogen atom, a C₁-C₆ alkoxy group, a C₁-C₆ haloalkoxy group, a C₂-C₆ alkoxyalkyl group, a C₁-C₆ alkylthio group, a C₁-C₆ haloalkylthio group, a C₁-C₆ alkylsulfinyl group or a C₁-C₆ alkylsulfonyl group, or salts thereof,
Y is a hydrogen atom, a C₁-C₆ alkyl group, C₁-C₆ haloalkyl group, a halogen atom, a C₁-C₆ alkoxy group, a C₁-C₆ haloalkoxy group or a C₂-C₆ alkoxyalkyl group, and
Q is a hydroxyl group or a group of 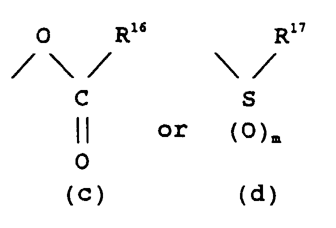
in which each of R¹⁶ and R¹⁷ is a C₁-C₆ alkyl group, a C₁-C₆ haloalkyl group or a phenyl group which may be substituted with a C₁-C₆ alkyl group, a C₁-C₆ haloalkyl group, a halogen atom, a cyano group or a nitro group, and m is 0, 1 or 2, or a salt thereof.

8. The cyclohexanedione derivative of any one of claims 1 to 7, wherein each of R³, R⁴, R⁵ and R⁶ is independently a hydrogen atom or methyl.

9. The cyclohexanedione derivative of any one of claims 1 to 7, wherein Y is substituted on the 8-position of a thiochroman ring.

10. The cyclohexanedione derivative of any one of claims 1 to 7, wherein n is 0 or 2.

11. A Herbicide containing, as an active ingredient, the cyclohexanedione derivative or the salts thereto recited in any one of claims 1 to 10.
